(19) Europäisches Patentamt
European Patent Office
Office européen des brevets

(11) EP 3 548 467 B1

(12) # EUROPÄISCHE PATENTSCHRIFT

(45) Veröffentlichungstag und Bekanntmachung des Hinweises auf die Patenterteilung:
**21.06.2023 Patentblatt 2023/25**

(21) Anmeldenummer: **17811242.1**

(22) Anmeldetag: **27.11.2017**

(51) Internationale Patentklassifikation (IPC):
***C07D 221/12*** *(2006.01)* ***C07D 471/04*** *(2006.01)*
***C07D 471/14*** *(2006.01)* ***C09K 11/06*** *(2006.01)*
***H05B 33/14*** *(2006.01)*

(52) Gemeinsame Patentklassifikation (CPC):
**C07D 221/12; C07D 471/04; C07D 471/14;**
Y02E 10/549

(86) Internationale Anmeldenummer:
**PCT/EP2017/080485**

(87) Internationale Veröffentlichungsnummer:
**WO 2018/099846 (07.06.2018 Gazette 2018/23)**

(54) **VERBINDUNGEN MIT VALEROLAKTAM-STRUKTUREN**

COMPOUNDS HAVING VALEROLACTAM STRUCTURES

COMPOSÉS À STRUCTURES VALÉROLACTAME

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.11.2016 EP 16201334**

(43) Veröffentlichungstag der Anmeldung:
**09.10.2019 Patentblatt 2019/41**

(73) Patentinhaber: **Merck Patent GmbH**
**64293 Darmstadt (DE)**

(72) Erfinder:
- **JATSCH, Anja**
  **60489 Frankfurt Am Main (DE)**
- **PARHAM, Amir**
  **60486 Frankfurt Am Main (DE)**
- **LINGE, Rouven**
  **64291 Darmstadt (DE)**
- **GROSSMANN, Tobias**
  **64297 Darmstadt (DE)**

(56) Entgegenhaltungen:
**WO-A1-2009/140467 WO-A1-2013/064206**
**US-A1- 2010 249 168 US-A1- 2013 053 555**
**US-A1- 2013 072 485 US-A1- 2015 270 495**

- **KAZUKO SHICHIRI ET AL.: "Studies on tertiary amine oxides. LXVI. Reactions of quinoline 1-oxide derivatives with tosyl chloride in the presence of triethylamine.", CHEM. PHARM. BULL., Bd. 28, Nr. 2, 1. Januar 1980 (1980-01-01) , Seiten 493-499, XP055439555, JP ISSN: 0009-2363, DOI: 10.1248/cpb.28.493**
- **CAMBARAU ET AL.: "Increased short circuit current in an azafullerene-based organic solar cell", CHEM. COMMUN., Bd. 51, Nr. 6, 1. Januar 2015 (2015-01-01) , Seiten 1128-1130, XP55439557, ISSN: 1359-7345, DOI: 10.1039/C4CC08094G**
- **YOSHIFUMI HASHIKAWA ET AL.: "Synthesis of Open-Cage Ketolactam Derivatives of Fullerene C 60 Encapsulating a Hydrogen Molecule", ORG. LETT., Bd. 16, Nr. 11, 6. Juni 2014 (2014-06-06), Seiten 2970-2973, XP55439558, ISSN: 1523-7060, DOI: 10.1021/ol501113y**
- **ALMOND ET AL.: "421. The structure and properties of certain polycyclic indolo- and quinolino-derivatives. Part III. Derivatives of 1 : 2 : 2a : 3 : 4 : 5 : 8 : 9 : 10 : 10a-decahydro-5 : 8-diketo-2a : 10a-diazapyrene", J. CHEM. SOC., 1. Januar 1951 (1951-01-01), Seiten 1906-1909, XP55439639, LETCHWORTH; GB ISSN: 0368-1769, DOI: 10.1039/jr9510001906**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- YOSHIKI HAMADA, ISAO TAKEUCHI: "Syntheses of Nitrogen-containing Heterocyclic Compounds. XXV. Chemical Reactivity of 4,6-Phenanthroline", CHEM. PHARM. BULL., Bd. 24, Nr. 11, 1. Januar 1976 (1976-01-01), Seiten 2769-2774, XP008081599, ISSN: 0009-2363
- MINTAS ET AL.: "STERICALLY HINDERED N-ARYL-2(1H)-QUINOLONES AND N-ARYL-6(5H)-PHENANTHRIDINONES: SEPARATION OF ENANTIOMERS AND BARRIERS TO RACEMIZATION", J. CHEM. SOC. PERKIN TRANS. 2, 1. Januar 1990 (1990-01-01), Seiten 619-624, XP008076006, ISSN: 1472-779X
- NAKAMURA ET AL.: "Enantioselective Synthesis and Enhanced Circularly Polarized Luminescence of S-Shaped Double Azahelicenes", J. AM. CHEM. SOC., Bd. 136, Nr. 15, 16. April 2014 (2014-04-16), Seiten 5555-5558, XP55439669, US ISSN: 0002-7863, DOI: 10.1021/ja500841f
- PERRIN, HUDHOMME: "Synthesis, Electrochemical and Optical Absorption Properties of New Perylene-3,4:9,10-bis(dicarboximide) and Perylene-3,4:9,10-bis(benzimidazole) Derivatives", EUR. J. ORG. CHEM., Bd. 2011, Nr. 28, 16. August 2011 (2011-08-16), Seiten 5427-5440, XP55439550, DE ISSN: 1434-193X, DOI: 10.1002/ejoc.201100513
- TANG: "Two-layer organic photovoltaic cell", APPL. PHYS. LETT., Bd. 48, Nr. 2, 13. Januar 1986 (1986-01-13), Seiten 183-185, XP002165026, ISSN: 0003-6951, DOI: 10.1063/1.96937
- NAKAMURA ET AL.: "The Photovoltaic Mechanism of a Polythiophene/Perylene Pigment Two-Layer Solar Cell", BULL. CHEM. SOC. JPN., Bd. 77, 2004, Seiten 2185-2188, XP9502680,

## Beschreibung

Die vorliegende Erfindung beschreibt aromatische oder heteroaromatische Valerolaktamderivate, welche mit Strukturgruppen substituiert sind, die drei anellierte aromatische Ringe umfassen, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen. Auch für fluoreszierende OLEDs gibt es bei diesen Materialien noch Verbesserungsbedarf.

Gemäß dem Stand der Technik werden unter anderem Lactame, z. B. gemäß WO 2011/137951 oder WO 2013/064206, als Matrixmaterialien für phosphoreszierende Emitter verwendet.

WO 2009/140467 A1 beschreibt Oxobenzindolizinochinoline und deren Verwendungen.

K. Shichiri et al. berichtet in Chem. Pharm. Bull. 1980, 28, 493-499 über Reaktionen von Chinolin-1-oxid-Derivaten mit Tosylchlorid in Gegenwart von Triethylamin.

US 2013/072485 A1 beschreibt niedermolekulare Modulatoren von mTORC1 und mTORC2, deren Synthesen und Zwischenprodukte dazu.

W. Cambarau et al. berichtet in Chem. Commun. 2015, 51, 1128-1130 über die Synthese eines lösungsverarbeitbaren Dodecyloxyphenylsubstituierten Azafulleren-Monoaddukts und seine Anwendung als Elektronenakzeptor in organischen Solarzellen mit Heteroübergang.

Y. Hashikawa et al. berichtet in Org. Lett. 2014, 16, 2970-2973 über die Synthese von Open-Cage-Ketolactam-Derivaten von Fulleren $C_{60}$, die ein Wasserstoffmolekül einkapseln.

C. Y. Almond et al. berichtet in J. Chem. Soc. 1951, 1906-1909 über die Struktur und Eigenschaften bestimmter polycyclischer Indolo- und Chinolino-Derivate.

Y. Hamada et al. berichtet in Chem. Pharm. Bull. 1976, 24, 2769-2774 über die Synthese stickstoffhaltiger heterocyclischer Verbindungen.

M. Mintas et al. berichtet in J. Chem. Soc. Perkin Trans. 2 1990, 619-624 über sterisch gehinderte N-Aryl-2(1*H*)-Chinolone und N-Aryl-6(5*H*)-Phenanthridinone, die Trennung von Enantionmeren und Barrieren für die Racemisierung.

US 2010/249168 A1 beschreibt Verbindungen und Formulierungen, die als Radikalfänger geeignet sind.

K. Nakamura et al. berichtet in J. Am. Chem. Soc. 2014, 136, 555-558 über die enantioselektive Synthese und verstärkte zirkular polarisierte Lumineszenz von S-förmigen Doppelazahelicenen.

L. Perrin et al. berichtet in Eur. J. Org. Chem. 2011, 5427-5440 über die Synthese und elektrochemischen und optischen Absorptionseigenschaften von neuen Perylen-3,4: 9,10-bis(dicarboximid)- und Perylen-3,4: 9,10-bis(benzimidazol)-Derivaten.

C. W. Tang beschreibt in Appl. Phys. Lett. 1986, 48, 183-185 eine zweischichtige organische Photovoltaikzelle.

J. Nakamura et al. beschreibt in Bull. Chem. Soc. Jpn. 2004, 77, 2185-2188 den photovoltaische Mechanismus einer zweischichtigen Solarzelle aus Polythiophen/Perylenpigment.

US 2015/0270495 A1 beschreibt Materialien für organische Elektrolumineszenzvorrichtungen, ein Verfahren zur Herstellung dieser Verbindungen und elektronischer Vorrichtungen, insbesondere organische Elektrolumineszenzvorrichtungen, die diese Verbindungen enthalten.

Generell besteht bei diesen Materialien, beispielsweise für die Verwendung als Matrixmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu

hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden OLED eignen, insbesondere als Matrixmaterial oder als Elektronentransportmaterial. Insbesondere ist es eine Aufgabe der vorliegenden Erfindung, Matrixmaterialien oder Elektronentransportmaterialien bereitzustellen, welche sich für rot, gelb und grün phosphoreszierende OLEDs und gegebenenfalls auch für blau phosphoreszierende OLEDs eignen.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen.

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Ausführungsformen sind daher Gegenstand der vorliegenden Erfindung.

Gegenstand der vorliegenden Erfindung ist daher eine Verbindung gemäß der Formel (II), (IV), oder (VI):

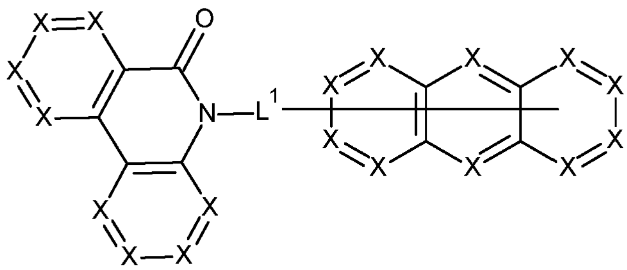

Formel (II)

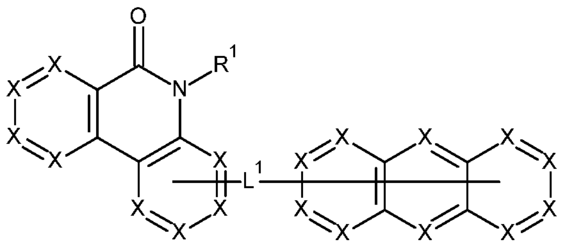

Formel (IV)

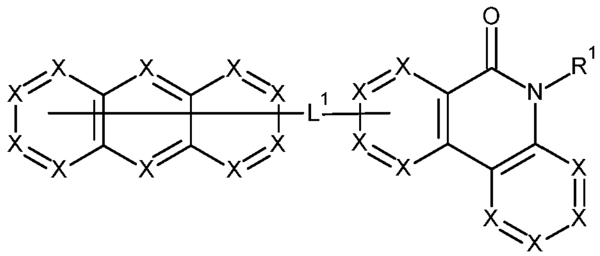

Formel (VI)

wobei für die verwendeten Symbole gilt:

X ist bei jedem Auftreten gleich oder verschieden $CR^1$ oder C für die Anbindungsstelle des Strukturelements mit drei anellierten Ringen (AR);

$R^1$ ist bei jedem Auftreten gleich oder verschieden H, D, CN, eine geradkettige Alkylgruppe mit 1 bis 40 C-Atomen oder eine verzweigte oder cyclische Alkylgruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C=C-, C=O, C=S, C=Se, $C=NR^2$, -C(=O)O-, $-C(=O)NR^2-$, $NR^2$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann;

$R^2$ ist bei jedem Auftreten gleich oder verschieden H, D, F, CN, eine geradkettige Alkylgruppe mit 1 bis 40 C-Atomen oder eine verzweigte oder cyclische Alkylgruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C=C-, C=O, C=S, C=Se, $C=NR^3$, -C(=O)O-, $-C(=O)NR^3-$, $NR^3$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können;

$L^1$ ist eine Bindung oder eine Arylgruppe mit 6 bis 40 C-Atomen oder eine Heteroarylgruppe mit 3 bis 40 C-Atomen, welche jeweils durch einen oder mehrere Reste $R^1$ substituiert sein kann.

Das Strukturelement mit drei anellierten aromatischen Ringen (AR) kann eine Struktur gemäß Formel (AR-2) umfassen

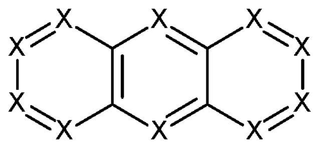

Formel (AR-2)

wobei für die verwendeten Symbole gilt:

X ist bei jedem Auftreten gleich oder verschieden $CR^1$ oder C für die Anbindungsstelle des Strukturelements mit einem aromatischen oder heteroaromatischen Valerolaktam (AV);

und das Symbol $R^1$ die zuvor genannte Bedeutung aufweist.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

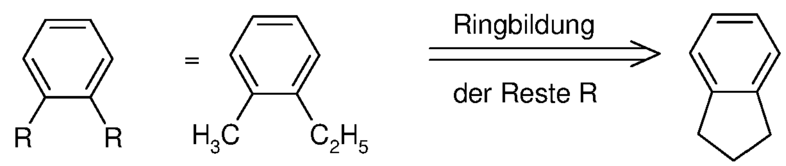


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

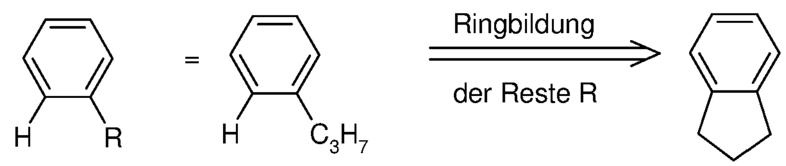


Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindestens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Im Rahmen der vorliegenden Erfindung ist der Begriff anelliert synonym zu kondensiert, so dass ein Strukturelement mit mindestens zwei, vorzugsweise drei anellierten aromatischen oder heteroaromatischen Ringen (AR) zwei beziehungsweise drei Ringe umfasst, wobei jeweils zwei Ringe eine gemeinsame Kante aufweisen. Hierbei sind aromatische oder heteroaromatische Ringe mit jeweils 6 Ringatomen bevorzugt, die jeweils kondensiert sind, so dass Strukturelemente mit mindestens zwei, vorzugsweise drei anellierten aromatischen oder heteroaromatischen Ringen (AR) bevorzugt sind, die 10 beziehungsweise 14 Ringatome und ein bzw. zwei gemeinsame Kanten aufweisen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl,

Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Das Strukturelement mit einem aromatischen Valerolaktam (AV) kann eine Struktur gemäß Formel (AV-1a), (AV-1b), (AV-1c), (AV-1d), (AV-1e), (AV-1f), (AV-1g) und/oder (AV-1h) umfassen

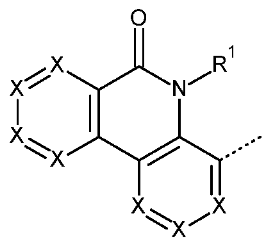

Formel (AV-1a)

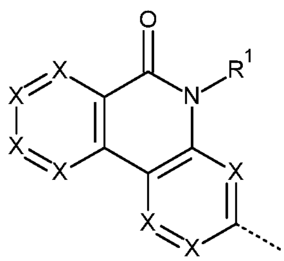

Formel (AV-1b)

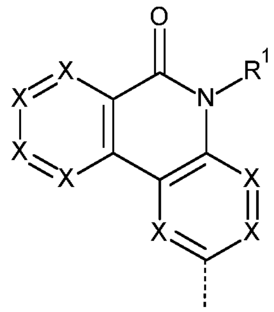

Formel (AV-1c)

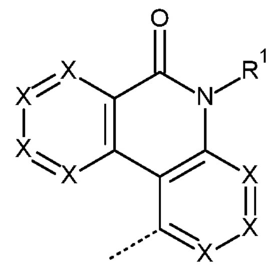

Formel (AV-1d)

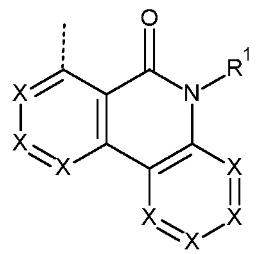

Formel (AV-1e)

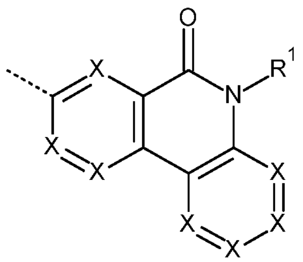

Formel (AV-1f)

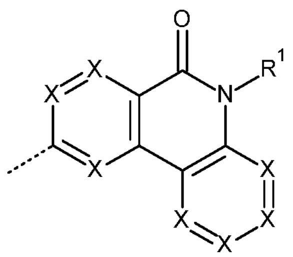

Formel (AV-1g)

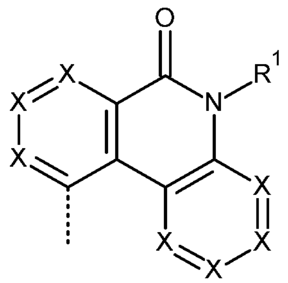

Formel (AV-1h)

wobei die Symbole X und $R^1$ die zuvor genannte Bedeutung aufweisen und die gestrichelte Bindung die Anbindungsstelle des Strukturelements mit drei anellierten Ringen (AR) darstellt.

Die erfindungsgemäßen Verbindungen umfassen mindestens ein Strukturelement mit mindestens drei annelierten aromatischen Ringen (AR), wobei dieses Strukturelement (AR) mindestens eine Struktur gemäß Formel (AR-2a), (AR-2b) und/oder (AR-2c) umfasst

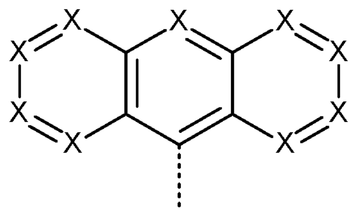

Formel (AR-2a)

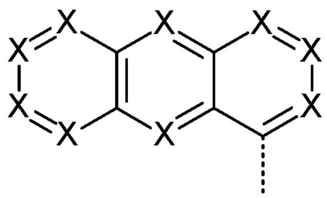

Formel (AR-2b)

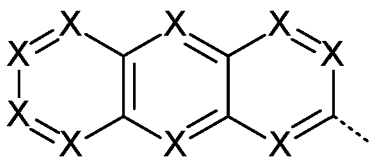

Formel (AR-2c)

wobei das Symbol X die zuvor genannte Bedeutung aufweist und die gestrichelte Bindung die Anbindungsstelle des Strukturelements mit einem aromatischen Valerolaktam (AV) darstellt.

Bevorzugt können die erfindungsgemäßen Verbindungen eine der Strukturen der Formeln (II-1), (II-2), (II-3), (II-4) oder (II-5) aufweisen

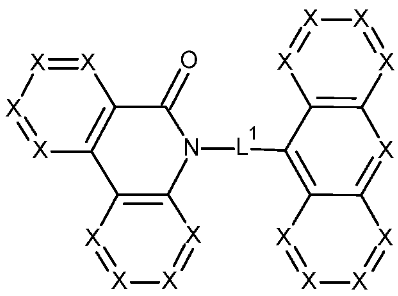

Formel (II-1)

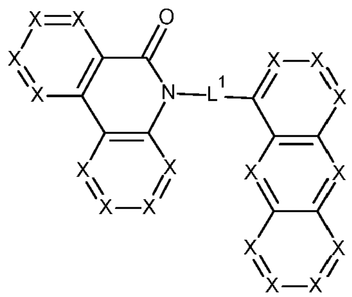

Formel (II-2)

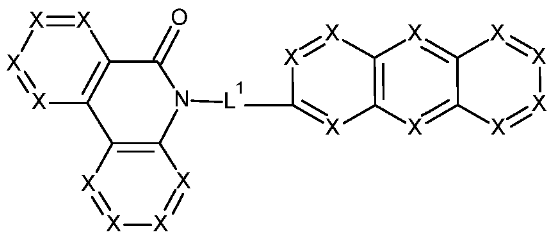

Formel (II-3)

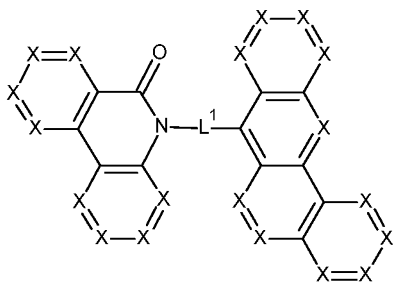

Formel (II-4)

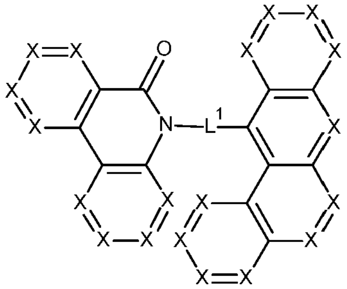

Formel (II-5)

wobei die Symbole X, $L^1$ und $R^1$ die zuvor, insbesondere für Formel (II) genannte Bedeutung aufweisen.

In einer weiterhin bevorzugten Ausführungsform können die erfindungsgemäßen Verbindungen eine der Strukturen der Formeln (IV-1), (IV-2) oder (IV-3)aufweisen

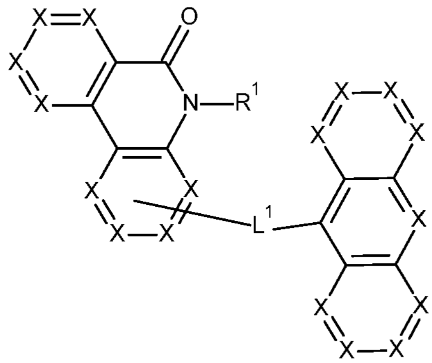

Formel (IV-1)

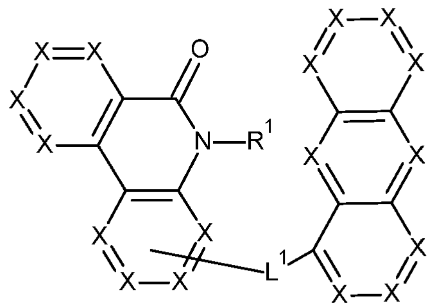

Formel (IV-2)

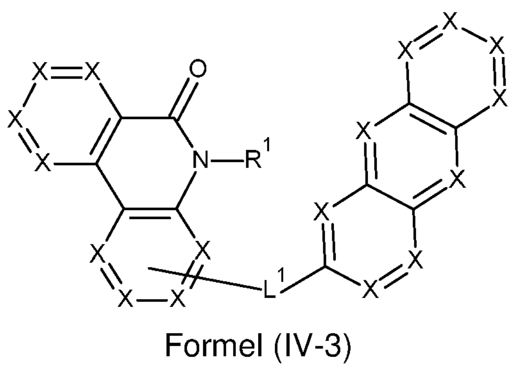

Formel (IV-3)

wobei die Symbole X, $L^1$ und $R^1$ die zuvor, insbesondere für Formel (IV) genannte Bedeutung aufweisen.

Darüber hinaus können die erfindungsgemäßen Verbindungen eine der Strukturen der Formeln (IV-6), (IV-7), (IV-8) oder (IV-9)aufweisen

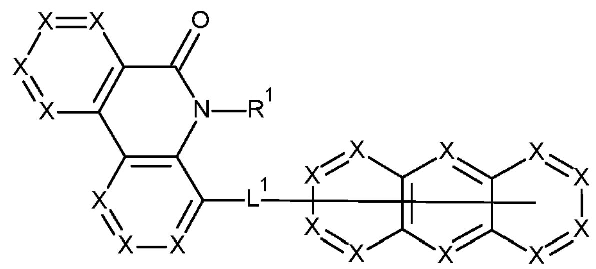

Formel (IV-6)

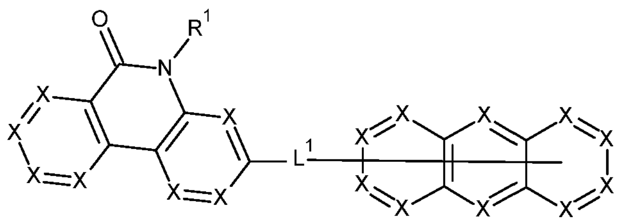

Formel (IV-7)

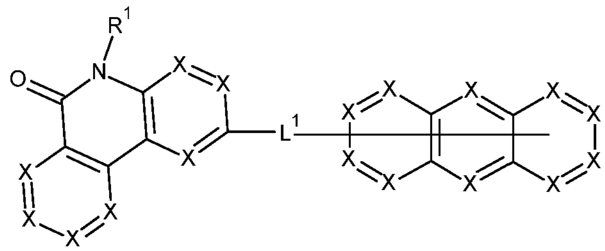

Formel (IV-8)

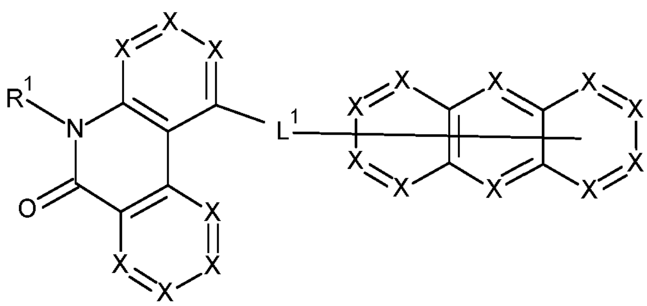

Formel (IV-9)

wobei die Symbole X, $L^1$ und $R^1$ die zuvor, insbesondere für Formel (IV) genannte Bedeutung aufweisen.

In einer weiterhin bevorzugten Ausführungsform können die erfindungsgemäßen Verbindungen eine der Strukturen der Formeln (VI-1), (VI-2) oder (VI-3) aufweisen

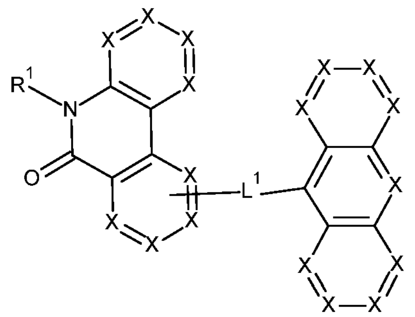

Formel (VI-1)

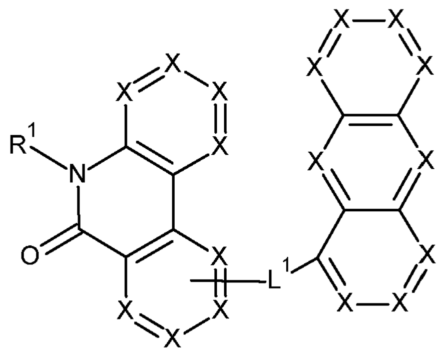

Formel (VI-2)

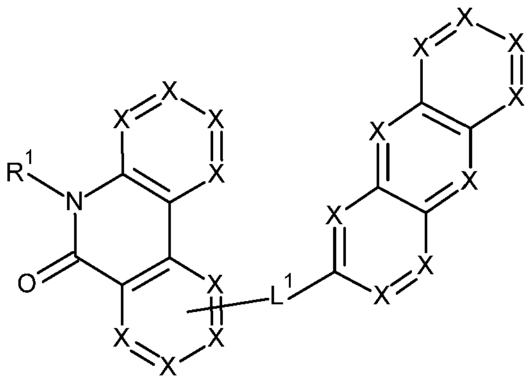

Formel (VI-3)

wobei die Symbole X, $L^1$ und $R^1$ die zuvor, insbesondere für Formel (VI) genannte Bedeutung aufweisen.

Ferner kann vorgesehen sein, dass erfindungsgemäße Verbindungen eine der Strukturen der Formeln (VI-6), (VI-7), (VI-8) oder (VI-9) aufweisen

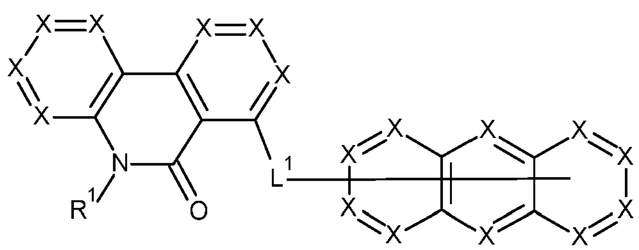

Formel (VI-6)

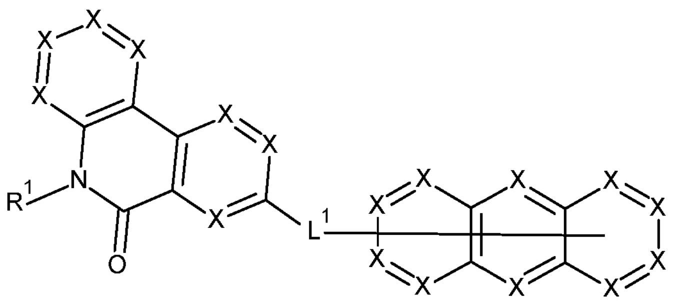

Formel (VI-7)

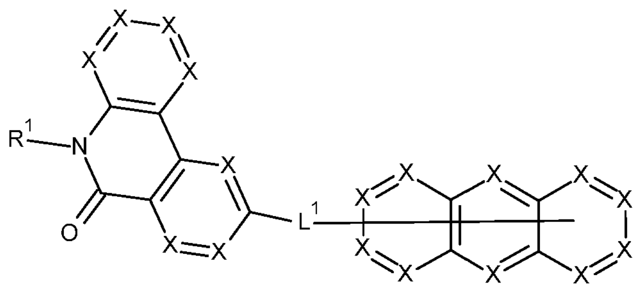

Formel (VI-8)

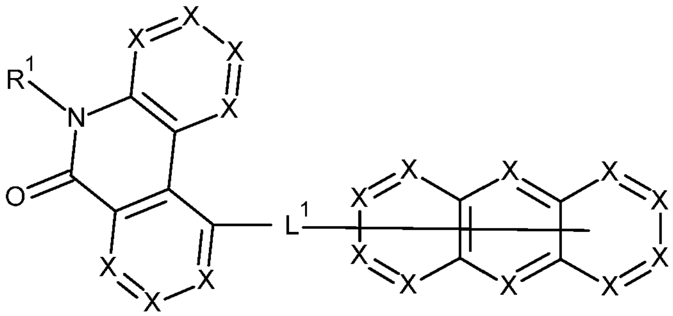

Formel (VI-9)

, wobei die Symbole X, $L^1$ und $R^1$ die zuvor, insbesondere für Formel (VI) genannte Bedeutung aufweisen.

Ferner kann vorgesehen sein, dass in Formeln (II), (II-1), (II-2), (II-3), (II-4), (II-5), (IV), (IV-1), (IV-2), (IV-3), (IV-6), (IV-7), (IV-8), (IV-9), (VI), (VI-1), (VI-2), (VI-3), (VI-6), (VI-7), (VI-8), (VI-9) alle X für $CR^1$ stehen, wobei vorzugsweise höchstens 4, besonders bevorzugt höchstens 3 und speziell bevorzugt höchstens 2 der Gruppen $CR^1$ für die X steht, ungleich der Gruppe CH ist.

Weiterhin sind Verbindungenmit einer der Strukturen der Formeln (IIa), (IVa), oder (VIa) bevorzugt

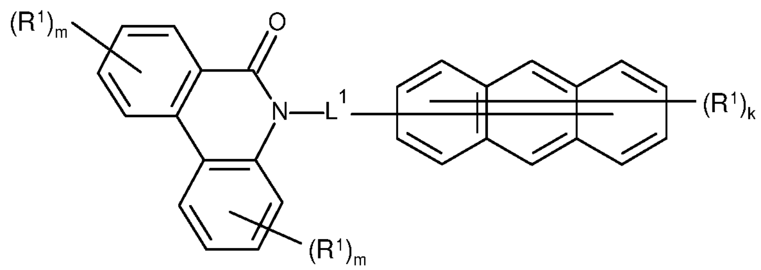

Formel (IIa)

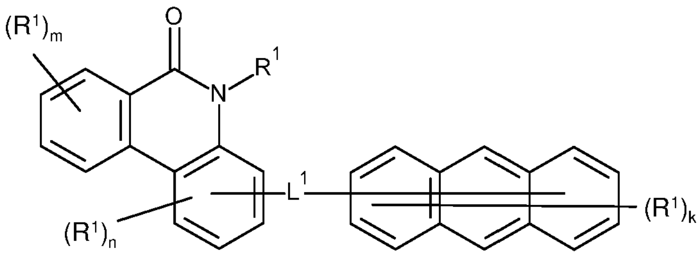

Formel (IVa)

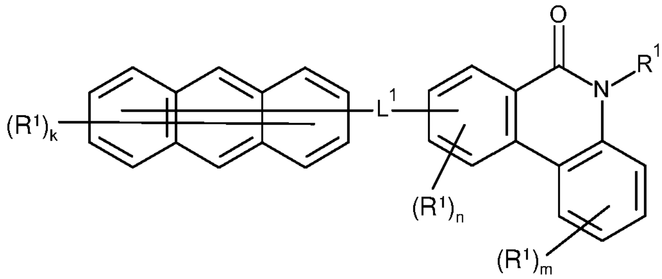

Formel (VIa)

, worin die Symbole $R^1$ und $L^1$ die zuvor, insbesondere für Formel (II), (IV) oder (VI) genannte Bedeutung haben und der Index k 0, 1, 2, 3, 4, 5, 6, 7, 8 oder 9, vorzugsweise 0, 1, 2 oder 3; der Index m 0, 1, 2, 3 oder 4, vorzugsweise 0, 1 oder 2; und der Index n 0, 1, 2 oder 3, vorzugsweise 0 oder 1 ist.

In einer weiterhin bevorzugten Ausführungsform können die erfindungsgemäßen Verbindungen eine der Strukturen der Formeln (IIa-1), (IIa-2), (IIa-3), (IIa-4) und/oder (IIa-5) aufweisen

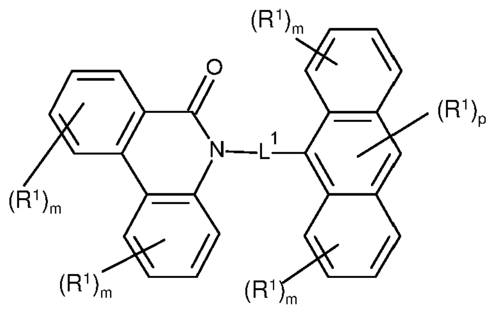

Formel (IIa-1)

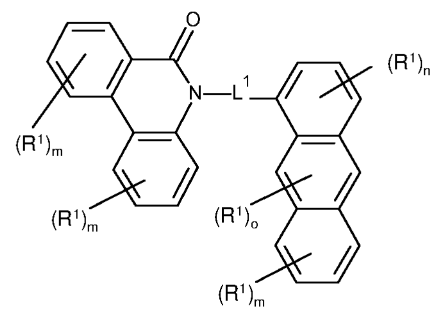

Formel (IIa-2)

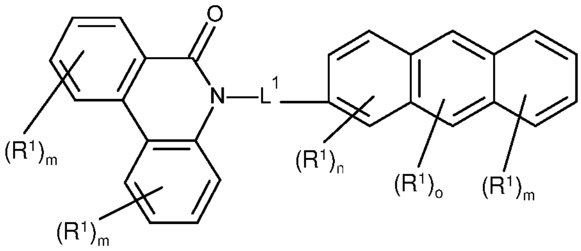

Formel (IIa-3)

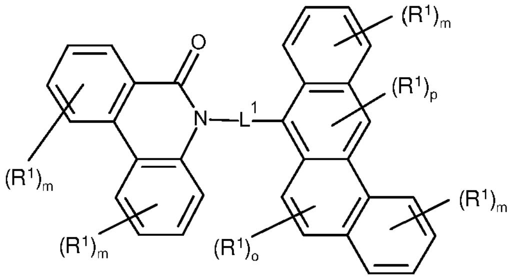

Formel (IIa-4)

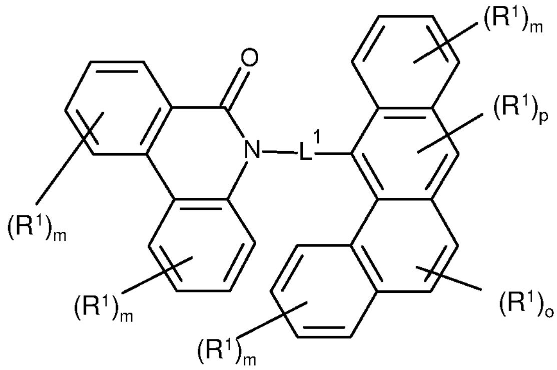

Formel (IIa-5)

wobei die Symbole $R^1$ und $L^1$ die zuvor, insbesondere für Formel (II) genannte Bedeutung haben, und der Index m 0, 1, 2, 3 oder 4, vorzugsweise 0, 1 oder 2; und der Index n 0, 1, 2 oder 3, vorzugsweise 0 oder 1; der Index o 0, 1 oder 2, vorzugsweise 0 oder 1, und der Index p 0 oder 1, vorzugsweise 1 ist.

Weiterhin können die erfindungsgemäßen Verbindungen eine der Strukturen der Formeln (IVa-1), (IVa-2) oder (IVa-3) aufweisen

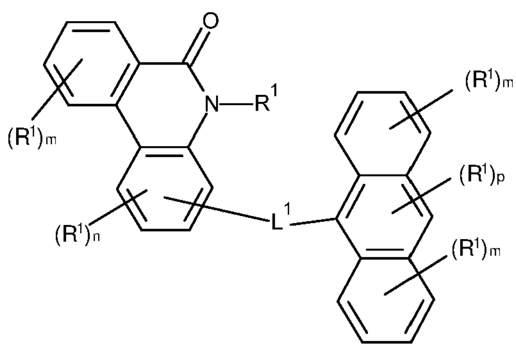

Formel (IVa-1)

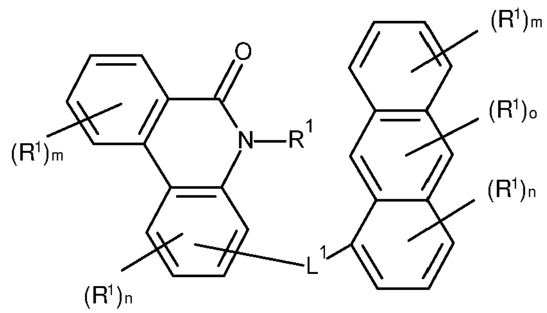

Formel (IVa-2)

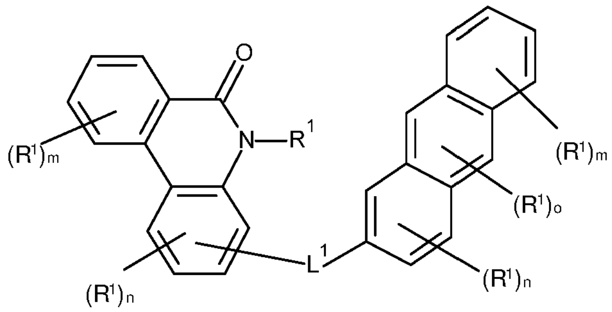

Formel (IVa-3)

wobei die Symbole $R^1$ und $L^1$ die zuvor, insbesondere für Formel (IV) genannte Bedeutung haben, und der Index m 0, 1, 2, 3 oder 4, vorzugsweise 0, 1 oder 2; und der Index n 0, 1, 2 oder 3, vorzugsweise 0 oder 1; der Index o 0, 1 oder 2, vorzugsweise 0 oder 1, und der Index p 0 oder 1, vorzugsweise 1 ist.

Weiterhin sind Verbindungen mit einer der Strukturen der Formeln (VIa-1), (VIa-2) oder (VIa-3) bevorzugt

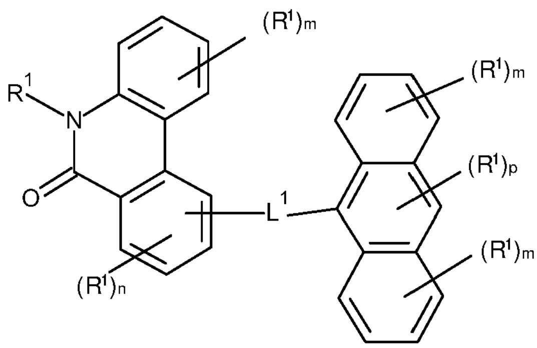

Formel (VIa-1)

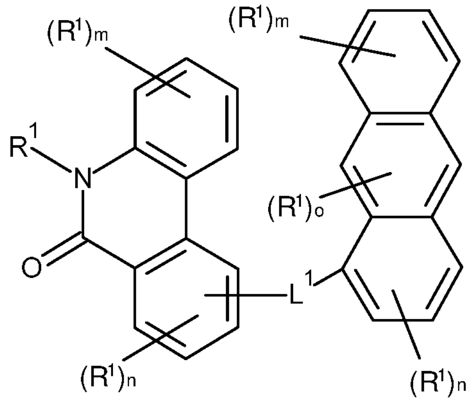

Formel (VIa-2)

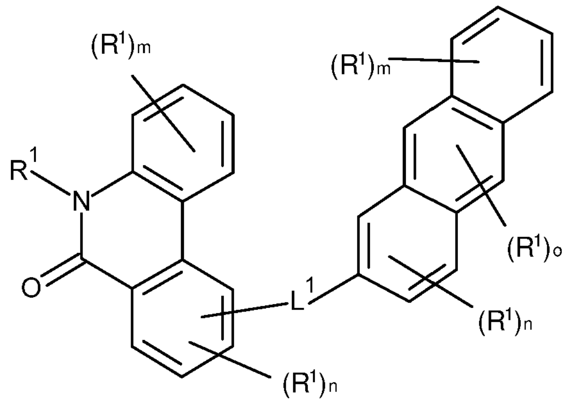

Formel (VIa-3)

, wobei die Symbole $R^1$ und $L^1$ die zuvor, insbesondere für Formel (VI) genannte Bedeutung haben, und der Index m 0, 1, 2, 3 oder 4, vorzugsweise 0, 1 oder 2; und der Index n 0, 1, 2 oder 3, vorzugsweise 0 oder 1; der Index o 0, 1 oder 2, vorzugsweise 0 oder 1, und der Index p 0 oder 1, vorzugsweise 1 ist.

Weiterhin können die erfindungsgemäßen Verbindungen eine der Strukturen der Formeln (IVa-6), (IVa-7), (IVa-8) oder (IVa-9) aufweisen

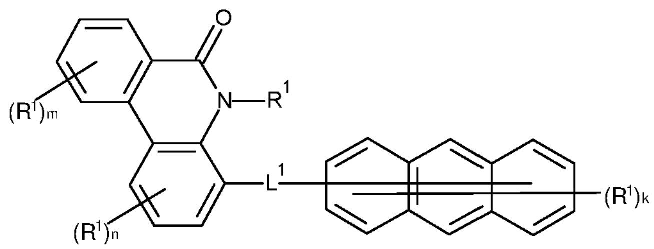

Formel (IVa-6)

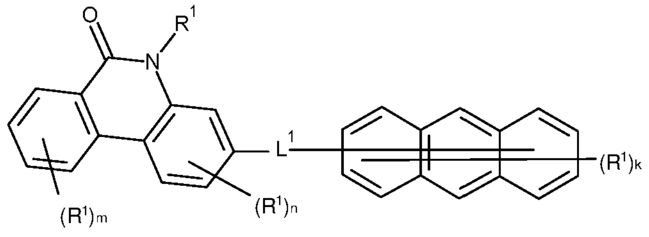

Formel (IVa-7)

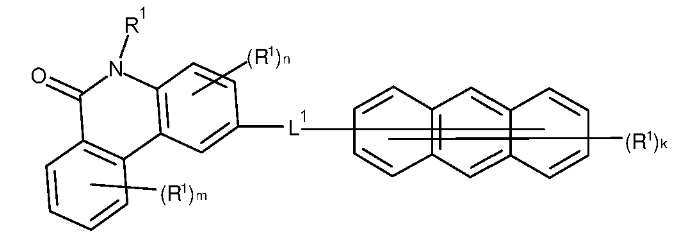

Formel (IVa-8)

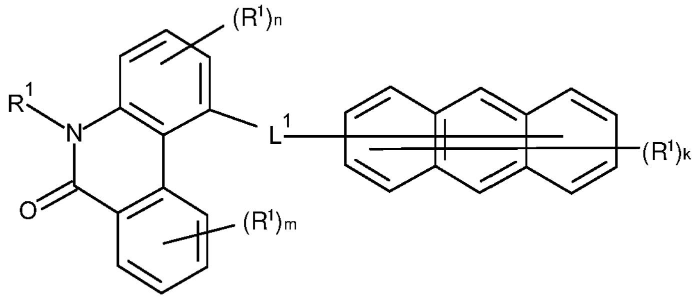

Formel (IVa-9)

wobei die Symbole $R^1$ und $L^1$ die zuvor, insbesondere für Formel (IV) genannte Bedeutung haben, und der der Index k 0, 1, 2, 3, 4, 5, 6, 7, 8 oder 9, vorzugsweise 0, 1, 2 oder 3; der Index m 0, 1, 2, 3 oder 4, vorzugsweise 0, 1 oder 2; und der Index n 0, 1, 2 oder 3, vorzugsweise 0 oder 1 ist.

Weiterhin sind Verbindungen mit einer der Strukturen der Formeln (VIa-6), (VIa-7), (VIa-8) oder (VIa-9) bevorzugt

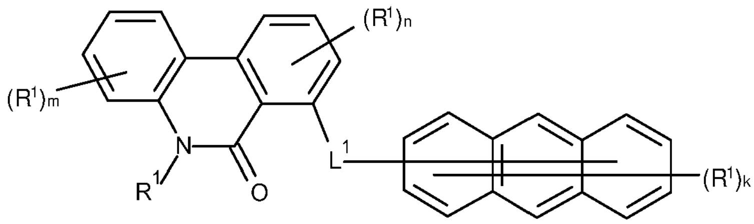

Formel (VIa-6)

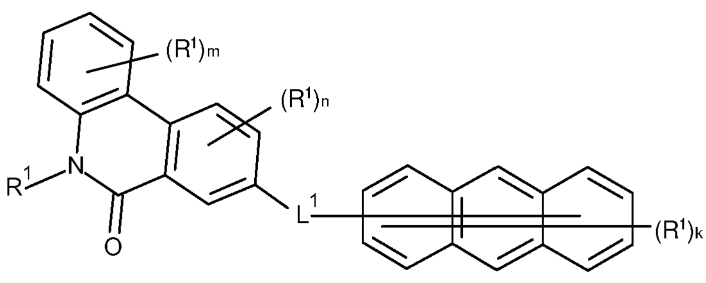

Formel (VIa-7)

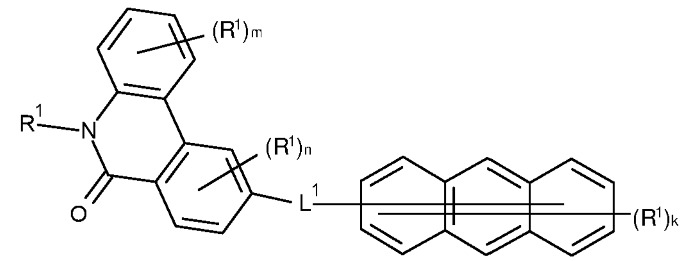

Formel (VIa-8)

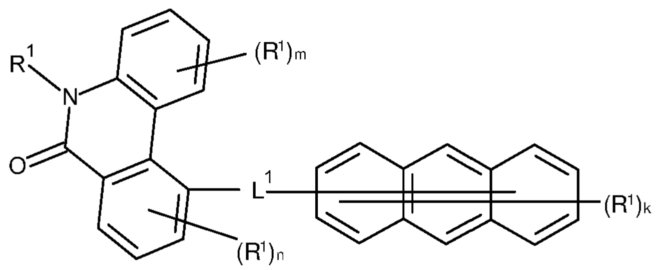

Formel (VIa-9)

, wobei die Symbole $R^1$ und $L^1$ die zuvor, insbesondere für Formel (II), (IV) oder (VI) genannte Bedeutung haben, und der Index k 0, 1, 2, 3, 4, 5, 6, 7, 8 oder 9, vorzugsweise 0, 1, 2 oder 3; der Index m 0, 1, 2, 3 oder 4, vorzugsweise 0, 1 oder 2; und der Index n 0, 1, 2 oder 3, vorzugsweise 0 oder 1 ist.

Ferner kann vorgesehen sein, dass in den Strukturen gemäß Formeln (IIa), (IIa-1), (IIa-2), (IIa-3), (IIa-4), (IIa-5), (IVa), (IVa-1), (IVa-2), (IVa-3), (IVa-6), (IVa-7), (IVa-8), (IVa-9), (VIa), (VIa-1), (VIa-2), (VIa-3), (VIa-6), (VIa-7), (VIa-8) und/oder (VIa-9) die Summe der Indices k, m, n, o und p höchstens 7, vorzugsweise höchstens 6 und besonders bevorzugt höchstens 5 beträgt.

Weiterhin kann vorgesehen sein, dass die Substituenten $R^1$ des aromatischen Ringsystems gemäß den Formeln (II), (II-1), (II-2), (II-3), (II-4), (II-5), (IIa), (IIa-1), (IIa-2), (IIa-3), (IIa-4), (IIa-5), (IV), (IV-1), (IV-2), (IV-3), (IV-6), (IV-7), (IV-8), (IV-9), (IVa), (IVa-1), (IVa-2), (IVa-3), (IVa-6), (IVa-7), (IVa-8), (IVa-9), (VI), (VI-1), (VI-2), (VI-3), (VI-6), (VI-7), (VI-8), (VI-9), (VIa), (VIa-1), (VIa-2), (VIa-3), (VIa-6), (VIa-7), (VIa-8) und/oder (VIa-9) mit den Ringatomen des aromatischen Ringsystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Substituenten $R^1$ des aromatischen Ringsystems gemäß den Formeln (II), (II-1), (II-2), (II-3), (II-4), (II-5), (IIa), (IIa-1), (IIa-2), (IIa-3), (IIa-4), (IIa-5), (IV), (IV-1), (IV-2), (IV-3), (IV-6), (IV-7), (IV-8), (IV-9), (IVa), (IVa-1), (IVa-2), (IVa-3), (IVa-6), (IVa-7), (IVa-8), (IVa-9), (VI), (VI-1), (VI-2), (VI-3), (VI-6), (VI-7), (VI-8), (VI-9), (VIa), (VIa-1), (VIa-2), (VIa-3), (VIa-6), (VIa-7), (VIa-8) und/oder (VIa-9) mit den Ringatomen des aromatischen Ringsystems kein Ringsystem bilden. Dies schließt die Bildung eines Ringsystems mit möglichen Substituenten $R^2$, $R^3$ ein, die an die Reste $R^1$ gebunden sein können.

Gemäß einer bevorzugten Ausgestaltung sind erfindungsgemäße Verbindungen durch Strukturen der Formel (II), (II-1), (II-2), (II-3), (II-4), (II-5), (IIa), (IIa-1), (IIa-2), (IIa-3), (IIa-4), (IIa-5), (IV), (IV-1), (IV-2), (IV-3), (IV-6), (IV-7), (IV-8), (IV-9), (IVa), (IVa-1), (IVa-2), (IVa-3), (IVa-6), (IVa-7), (IVa-8), (IVa-9), (VI), (VI-1), (VI-2), (VI-3), (VI-6), (VI-7), (VI-8), (VI-9), (VIa), (VIa-1), (VIa-2), (VIa-3), (VIa-6), (VIa-7), (VIa-8) und/oder (VIa-9) darstellbar. Vorzugsweise weisen erfindungsgemäße Verbindungen, besonders bevorzugt Verbindungen, mit Strukturen gemäß Formel (II), (II-1), (II-2), (II-3), (II-4), (II-5), (IIa), (IIa-1), (IIa-2), (IIa-3), (IIa-4), (IIa-5), (IV), (IV-1), (IV-2), (IV-3), (IV-6), (IV-7), (IV-8), (IV-9), (IVa), (IVa-1), (IVa-2), (IVa-3), (IVa-6), (IVa-7), (IVa-8), (IVa-9), (VI), (VI-1), (VI-2), (VI-3), (VI-6), (VI-7), (VI-8), (VI-9), (VIa), (VIa-1), (VIa-2), (VIa-3), (VIa-6), (VIa-7), (VIa-8) und/oder (VIa-9) ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Wenn X für $CR^1$ steht bzw. wenn die aromatische und/oder heteroaromatische Gruppen durch Substituenten $R^1$ substituiert sind, dann sind diese Substituenten $R^1$ bevorzugt gewählt aus der Gruppe bestehend aus H, D, CN, einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten $R^2$ substituiert sein kann, wobei eine oder mehrere nichtbenachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten $R^2$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^2$ substituiert sein kann; dabei können optional zwei Substituenten $R^1$, die vorzugsweise an benachbarte Kohlenstoffatome gebunden sind, ein monocyclisches oder polycyclisches, aliphatisches, heteroaliphatisches, aromatisches oder heteroaromatisches Ringsystem bilden, das mit einem oder mehreren Resten $R^2$ substituiert sein kann Besonders bevorzugt sind diese Substituenten $R^1$ ausgewählt aus der Gruppe bestehend aus H, D, CN, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten $R^2$ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist; dabei können optional zwei Substituenten $R^1$, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten $R^2$ substituiert sein kann, bevorzugt aber unsubstituiert ist.

Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Weiterhin kann vorgesehen sein, dass in einer Struktur gemäß Formel (II), (II-1), (II-2), (II-3), (II-4), (II-5), (IIa), (IIa-1), (IIa-2), (IIa-3), (IIa-4), (IIa-5), (IV), (IV-1), (IV-2), (IV-3), (IV-6), (IV-7), (IV-8), (IV-9), (IVa), (IVa-1), (IVa-2), (IVa-3), (IVa-6), (IVa-7), (IVa-8), (IVa-9), (VI), (VI-1), (VI-2), (VI-3), (VI-6), (VI-7), (VI-8), (VI-9), (VIa), (VIa-1), (VIa-2), (VIa-3), (VIa-6), (VIa-7), (VIa-8) und/oder (VIa-9) mindestens ein Rest $R^1$ für eine Gruppe steht, die ausgewählt ist aus den Formeln ($R^1$-1) bis ($R^1$- 86)

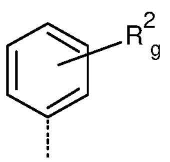

Formel ($R^1$-1)

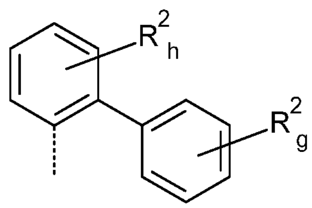

Formel ($R^1$-2)

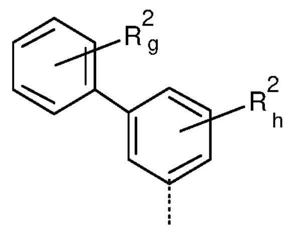

Formel ($R^1$-3)

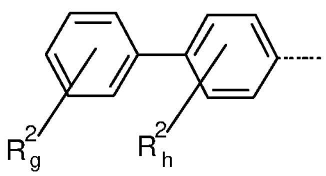

Formel ($R^1$-4)

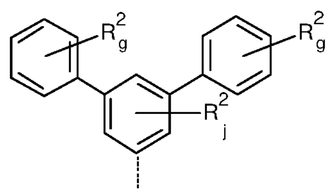

Formel ($R^1$-5)

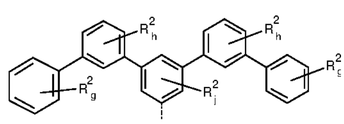

Formel ($R^1$-6)

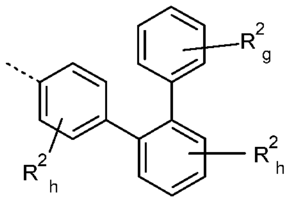

Formel ($R^1$-7)

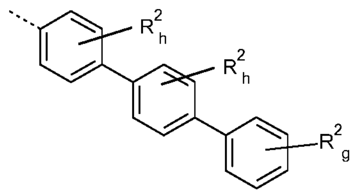

Formel ($R^1$-8)

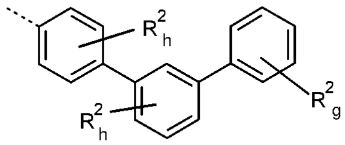

Formel ($R^1$-9)

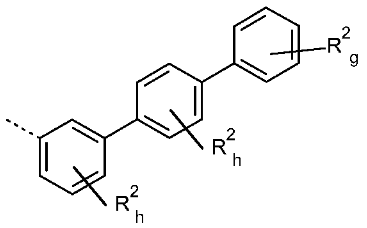

Formel ($R^1$-10)

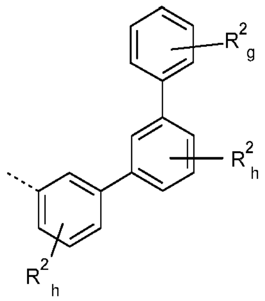

Formel ($R^1$-11)

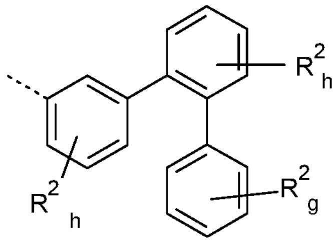

Formel ($R^1$-12)

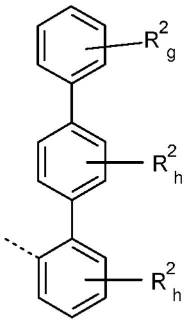

Formel ($R^1$-13)

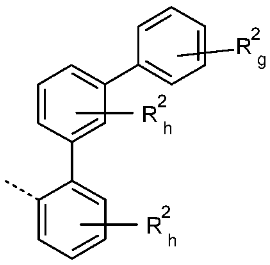

Formel ($R^1$-14)

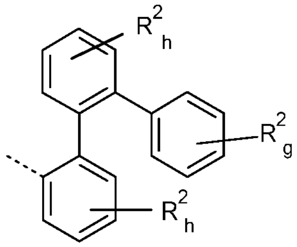

Formel ($R^1$-15)

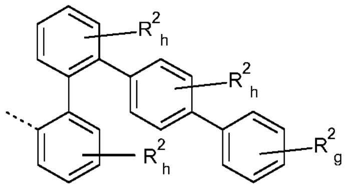

Formel ($R^1$-16)

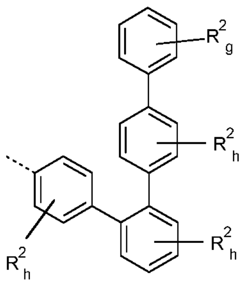

Formel ($R^1$-17)

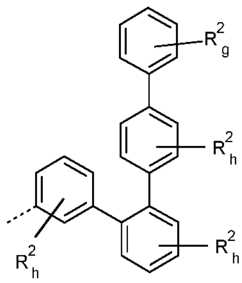

Formel ($R^1$-18)

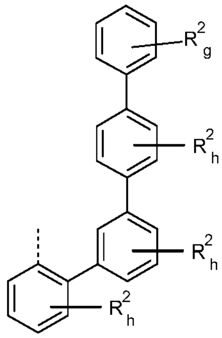

Formel ($R^1$-19)

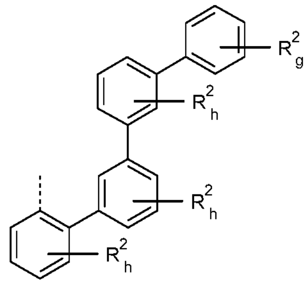

Formel ($R^1$-20)

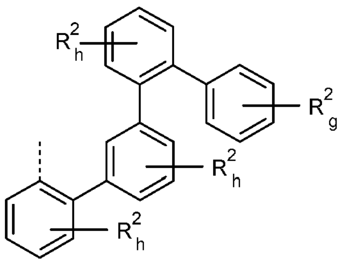

Formel ($R^1$-21)

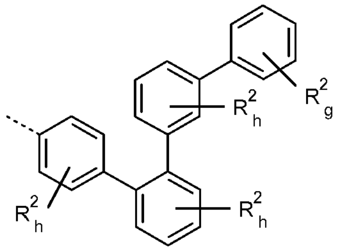

Formel ($R^1$-22)

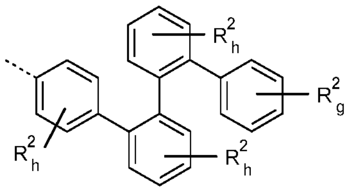

Formel ($R^1$-23)

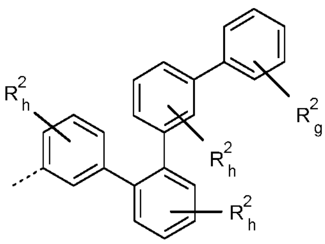

Formel ($R^1$-24)

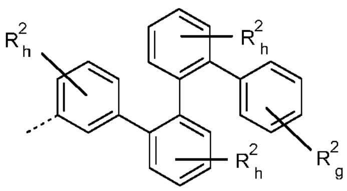

Formel ($R^1$-25)

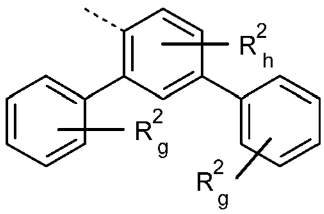

Formel ($R^1$-26)

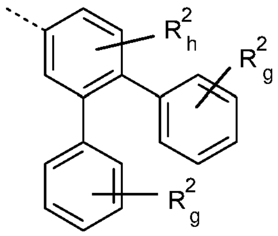

Formel ($R^1$-27)

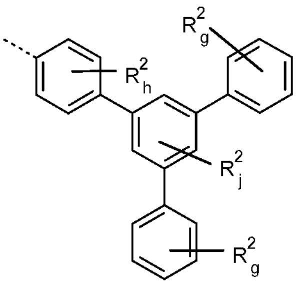

Formel ($R^1$-28)

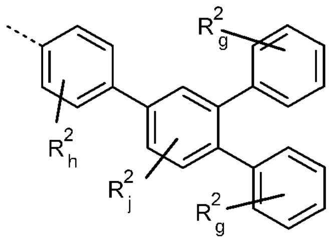

Formel ($R^1$-29)

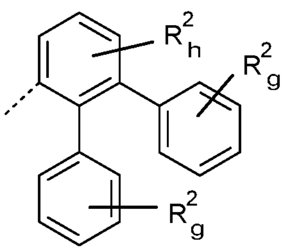

Formel ($R^1$-30)

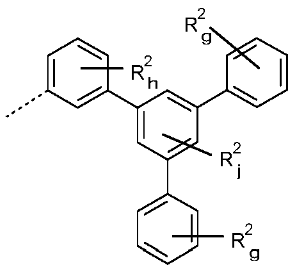

Formel ($R^1$-31)

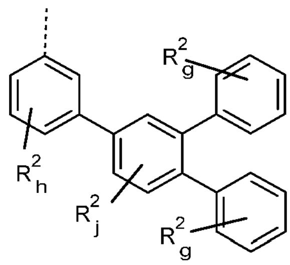

Formel ($R^1$-32)

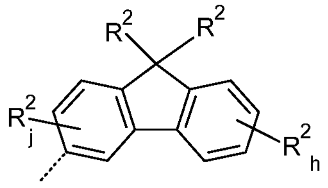

Formel ($R^1$-33)

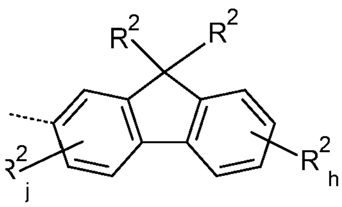

Formel ($R^1$-34)

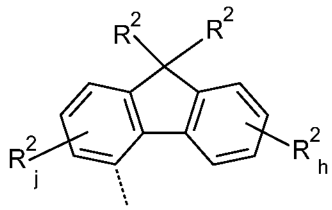

Formel ($R^1$-35)

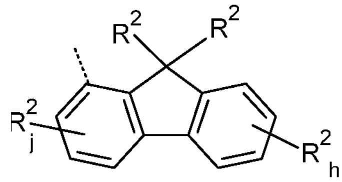

Formel ($R^1$-36)

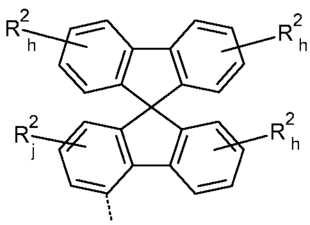

Formel ($R^1$-37)

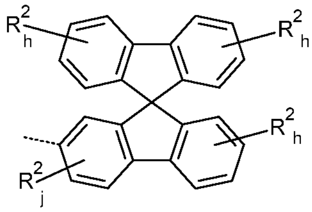

Formel ($R^1$-38)

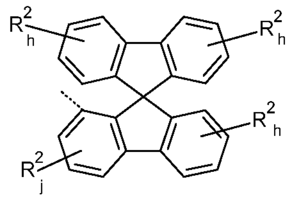

Formel ($R^1$-39)

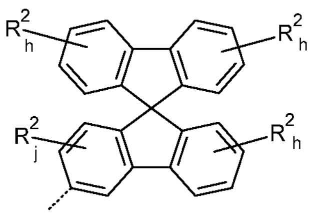

Formel ($R^1$-40)

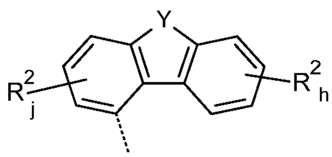

Formel ($R^1$-41)

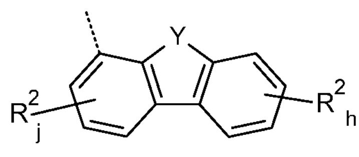

Formel ($R^1$-42)

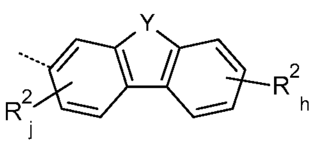

Formel ($R^1$-43)

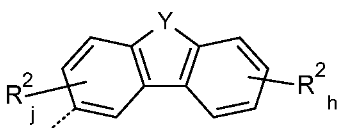

Formel ($R^1$-44)

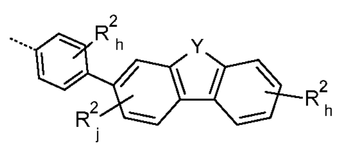

Formel ($R^1$-45)

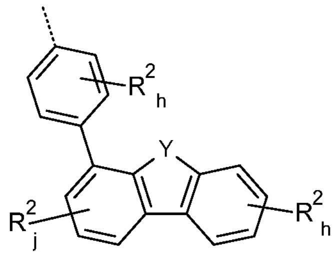

Formel ($R^1$-46)

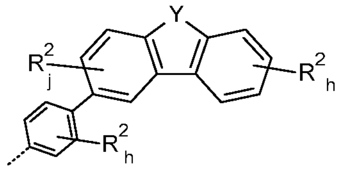

Formel ($R^1$-47)

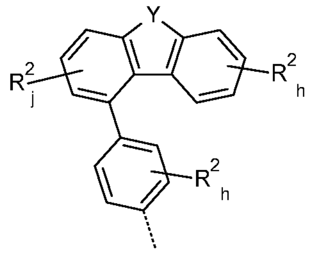

Formel ($R^1$-48)

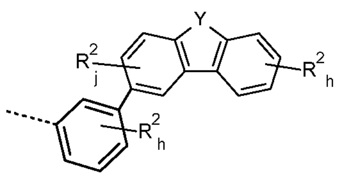

Formel ($R^1$-49)

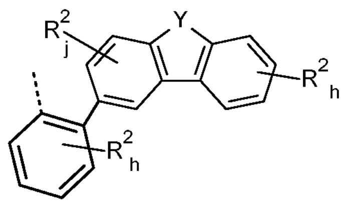

Formel ($R^1$-50)

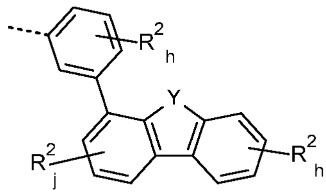

Formel ($R^1$-51)

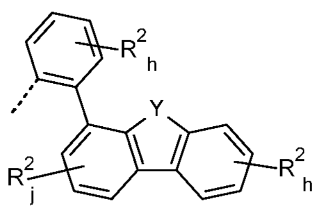

Formel ($R^1$-52)

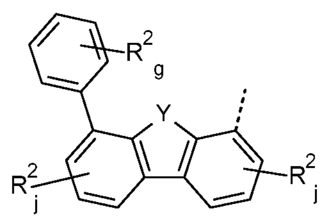

Formel ($R^1$-53)

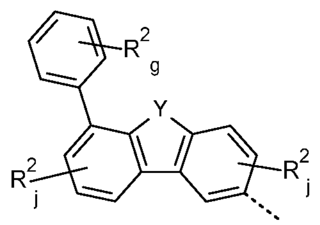

Formel ($R^1$-54)

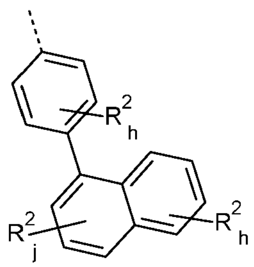

Formel ($R^1$-55)

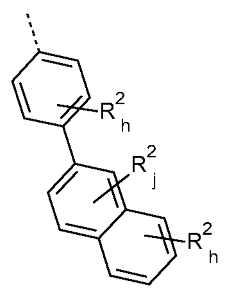

Formel ($R^1$-56)

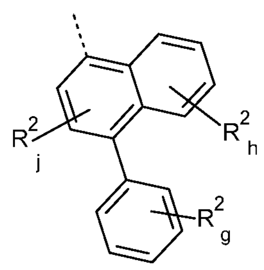

Formel ($R^1$-57)

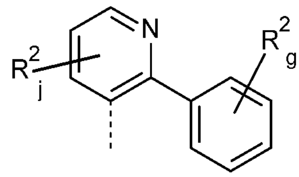

Formel ($R^1$-58)

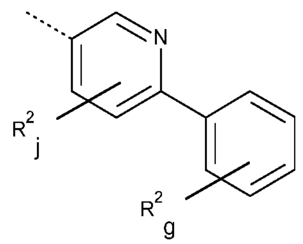

Formel ($R^1$-59)

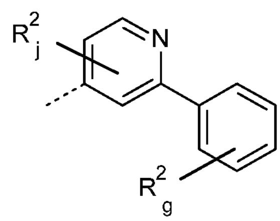

Formel ($R^1$-60)

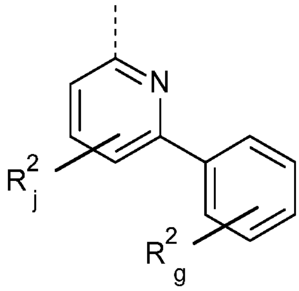

Formel ($R^1$-61)

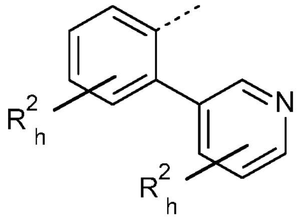

Formel ($R^1$-62)

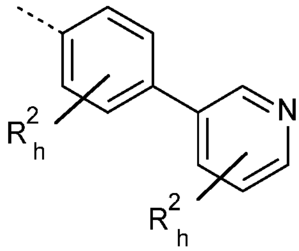

Formel ($R^1$-63)

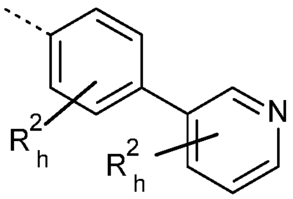

Formel ($R^1$-64)

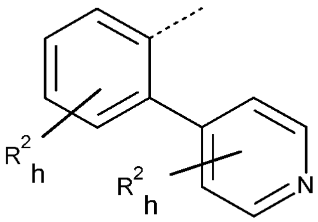

Formel ($R^1$-65)

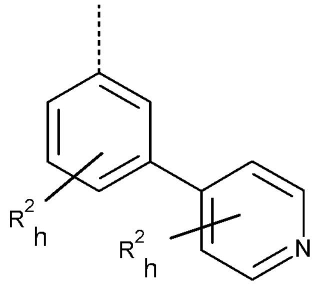

Formel ($R^1$-66)

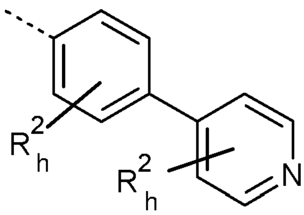

Formel ($R^1$-67)

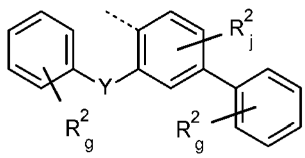

Formel ($R^1$-68)

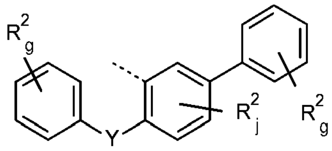

Formel ($R^1$-69)

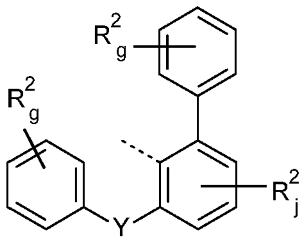

Formel ($R^1$-70)

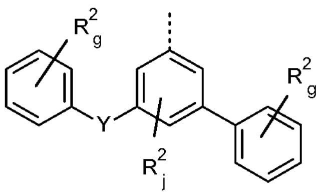

Formel ($R^1$-71)

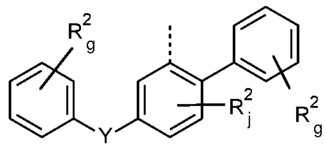

Formel ($R^1$-72)

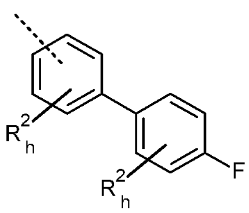

Formel ($R^1$-73)

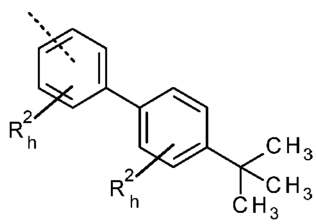

Formel ($R^1$-74)

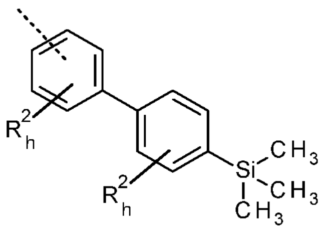

Formel ($R^1$-75)

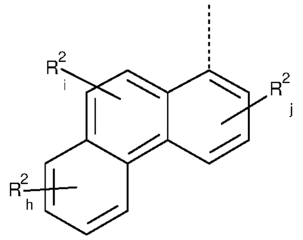

Formel ($R^1$-76)

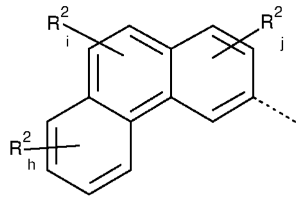

Formel ($R^1$-77)

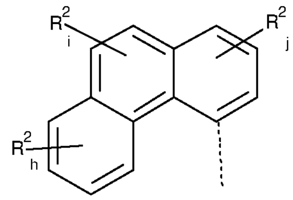

Formel ($R^1$-78)

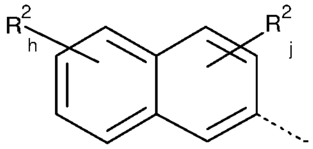

Formel ($R^1$-79)

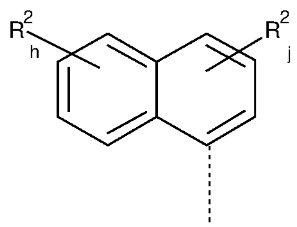

Formel ($R^1$-80)

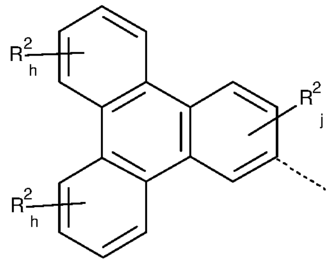

Formel ($R^1$-81)

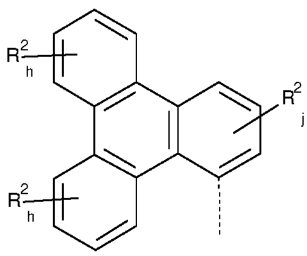

Formel ($R^1$-82)

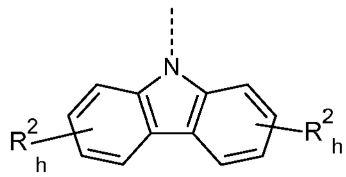

Formel ($R^1$-83)

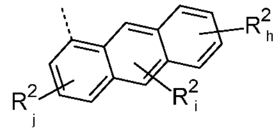

Formel ($R^1$-84)

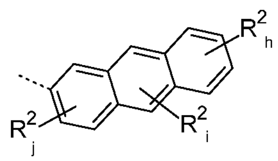

Formel ($R^1$-85)

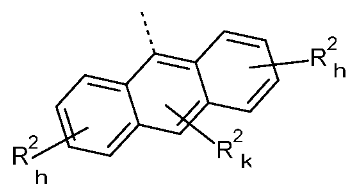

Formel ($R^1$-86)

wobei für die verwendeten Symbole gilt:

Y ist O, S oder $NR^2$, vorzugsweise O oder S;
k ist bei jedem Auftreten unabhängig 0 oder 1;
i ist bei jedem Auftreten unabhängig 0, 1 oder 2;
j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5;
$R^2$ kann die zuvor genannte Bedeutung aufweisen und

die gestrichelte Bindung markiert die Anbindungsposition. Hierbei sind die Gruppen der Formeln R1-1 bis R1-54 bevorzugt und Gruppen der Formeln R1-1, R1-3, R1-5, R1-6, R1-15, R1-29, R1-34, R1-35, R1-45, R1-46, R1-47 und/oder R1-48 besonders bevorzugt.

Vorzugsweise kann vorgesehen sein, dass die Summe der Indices i, j, h und g in den Strukturen der Formel

($R^1$-1) bis ($R^1$-86) jeweils höchstens 3, vorzugsweise höchstens 2 und besonders bevorzugt höchstens 1 beträgt.

Bevorzugt bilden die Reste $R^2$ in den Formeln ($R^1$-1) bis ($R^1$-86) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

Bevorzugt kann die Gruppe $L^1$ mit dem Strukturelement mit drei anellierten aromatischen Ringen (AR) und mit dem Strukturelement mit einem aromatischen Valerolaktam (AV) eine durchgängige Konjugation ausbilden. Eine durchgängige Konjugation der aromatischen beziehungsweise heteroaromatischen Systeme wird ausgebildet sobald direkte Bindungen zwischen benachbarten aromatischen oder heteroaromatischen Ringen gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das $sp^3$ hybridisierte Kohlenstoffatom in Position 9 zwar eine Kondensation dieser Ringe unterbindet, jedoch eine Konjugation erfolgen kann, da dieses $sp^3$ hybridisierte Kohlenstoffatom in Position 9 nicht zwingend zwischen dem Strukturelement mit drei anellierten aromatischen (AR) und dem Strukturelement mit einem aromatischen Valerolaktam (AV) liegt. Im Gegensatz hierzu kann bei einer zweiten Spirobifluorenstruktur eine durchgängige Konjugation ausgebildet werden, falls die Verbindung zwischen dem Strukturelement mit drei anellierten aromatischen Ringen (AR) und dem Strukturelement mit einem aromatischen Valerolaktam (AV) über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die Verbindung zwischen dem Strukturelement mit drei anellierten aromatischen Ringen (AR) und dem Strukturelement mit einem aromatischen Valerolaktam (AV) über verschiedene Phenylgruppen der zweiten Spirobifluorenstruktur erfolgt, die über das $sp^3$ hybridisierte Kohlenstoffatom in Position 9 verbunden sind, ist die Konjugation unterbrochen.

In einer weiteren bevorzugten Ausführungsform der Erfindung steht $L^1$ für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor genannte Bedeutung aufweisen kann. Besonders bevorzugt steht $L^1$ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^2$ substituiert sein kann, bevorzugt aber unsubstituiert ist, wobei $R^2$ die zuvor genannte Bedeutung aufweisen kann.

Weiterhin bevorzugt steht das unter anderem in den Strukturen gemäß Formel (II), (II-1), (II-2), (II-3), (II-4), (II-5), (IIa), (IIa-1), (IIa-2), (IIa-3), (IIa-4), (IIa-5), (IV), (IV-1), (IV-2), (IV-3), (IV-6), (IV-7), (IV-8), (IV-9), (IVa), (IVa-1), (IVa-2), (IVa-3), (IVa-6), (IVa-7), (IVa-8), (IVa-9), (VI), (VI-1), (VI-2), (VI-3), (VI-6), (VI-7), (VI-8), (VI-9), (VIa), (VIa-1), (VIa-2), (VIa-3), (VIa-6), (VIa-7), (VIa-8) und/oder (VIa-9) dargelegte Symbol $L^1$ gleich oder verschieden bei jedem Auftreten für eine Bindung oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die unter anderem in den Strukturen gemäß Formel (II), (II-1), (II-2), (II-3), (II-4), (II-5), (IIa), (IIa-1), (IIa-2), (IIa-3), (IIa-4), (IIa-5), (IV), (IV-1), (IV-2), (IV-3), (IV-6), (IV-7), (IV-8), (IV-9), (IVa), (IVa-1), (IVa-2), (IVa-3), (IVa-6), (IVa-7), (IVa-8), (IVa-9), (VI), (VI-1), (VI-2), (VI-3), (VI-6), (VI-7), (VI-8), (VI-9), (VIa), (VIa-1), (VIa-2), (VIa-3), (VIa-6), (VIa-7), (VIa-8) und/oder (VIa-9) dargelegte Gruppe $L^1$ ein aromatisches Ringsystem mit höchstens zwei kondensierten aromatischen und/oder heteroaromatischen Ringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme $L^1$ sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste $R^2$ substituiert sein können, bevorzugt aber unsubstituiert sind.

Ferner kann vorgesehen sein, dass die unter anderem in den Strukturen gemäß Formel (II), (II-1), (II-2), (II-3), (II-4), (II-5), (IIa), (IIa-1), (IIa-2), (IIa-3), (IIa-4), (IIa-5), (IV), (IV-1), (IV-2), (IV-3), (IV-6), (IV-7), (IV-8), (IV-9), (IVa), (IVa-1), (IVa-2), (IVa-3), (IVa-6), (IVa-7), (IVa-8), (IVa-9), (VI), (VI-1), (VI-2), (VI-3), (VI-6), (VI-7), (VI-8), (VI-9), (VIa), (VIa-1), (VIa-2), (VIa-3), (VIa-6), (VIa-7), (VIa-8) und/oder (VIa-9) dargelegte Gruppe $L^1$ höchstens 1 Stickstoffatom, bevorzugt höchstens 2 Heteroatome, insbesondere bevorzugt höchstens ein Heteroatom und besonders bevorzugt kein Heteroatom aufweist.

Bevorzugt sind Verbindungen umfassend Strukturen der Formeln (II), (II-1), (II-2), (II-3), (II-4), (II-5), (IIa), (IIa-1), (IIa-2), (IIa-3), (IIa-4), (IIa-5), (IV), (IV-1), (IV-2), (IV-3), (IV-6), (IV-7), (IV-8), (IV-9), (IVa), (IVa-1), (IVa-2), (IVa-3), (IVa-6), (IVa-7), (IVa-8), (IVa-9), (VI), (VI-1), (VI-2), (VI-3), (VI-6), (VI-7), (VI-8), (VI-9), (VIa), (VIa-1), (VIa-2), (VIa-3), (VIa-6), (VIa-7), (VIa-8) und/oder (VIa-9), worin die Gruppe $L^1$ für eine Bindung oder für eine Gruppe steht, die ausgewählt ist aus den Formeln ($L^1$-1) bis ($L^1$-108)

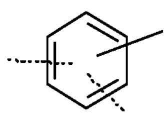

Formel ($L^1$-1)

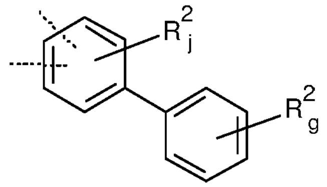

Formel ($L^1$-2)

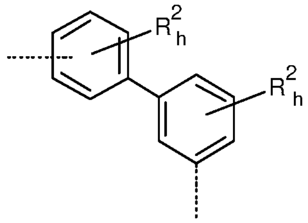

Formel ($L^1$-3)

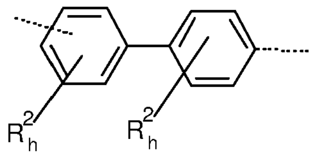

Formel ($L^1$-4)

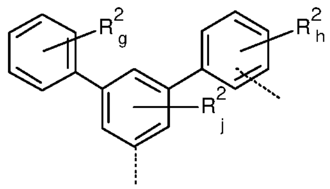

Formel ($L^1$-5)

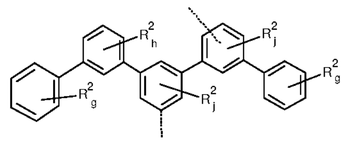

Formel ($L^1$-6)

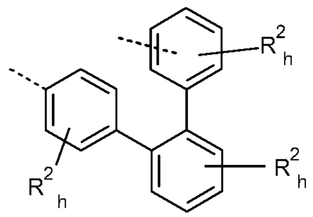

Formel ($L^1$-7)

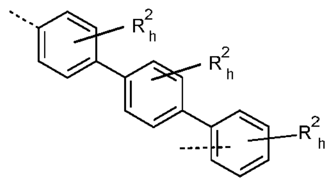

Formel ($L^1$-8)

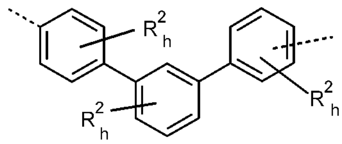

Formel ($L^1$-9)

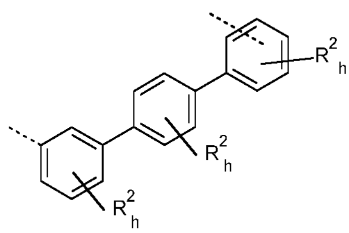

Formel ($L^1$-10)

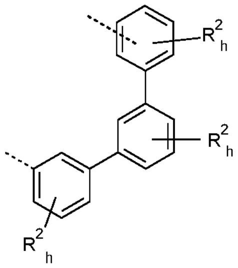

Formel ($L^1$-11)

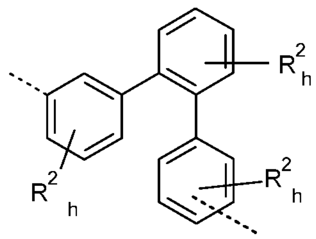

Formel ($L^1$-12)

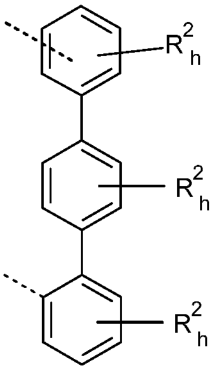

Formel ($L^1$-13)

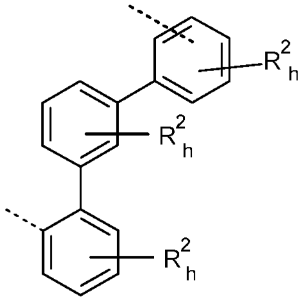

Formel ($L^1$-14)

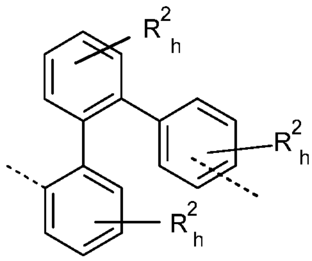

Formel ($L^1$-15)

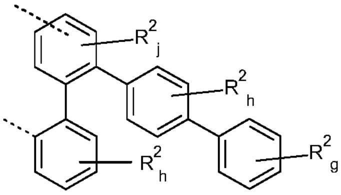

Formel ($L^1$-16)

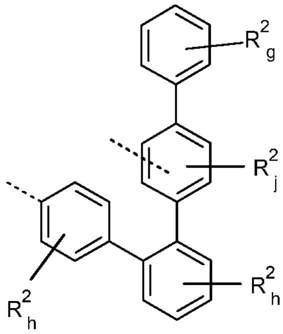

Formel ($L^1$-17)

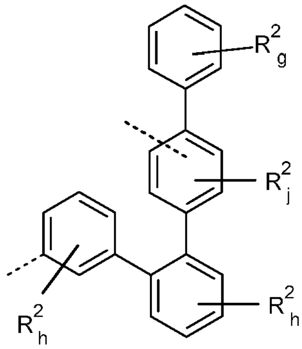

Formel ($L^1$-18)

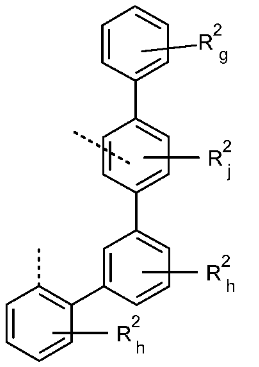

Formel ($L^1$-19)

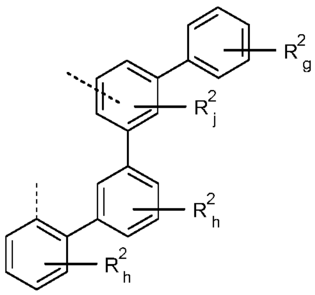

Formel ($L^1$-20)

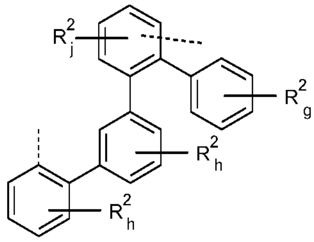

Formel ($L^1$-21)

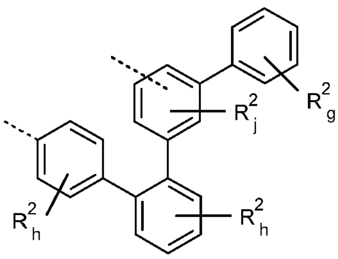

Formel ($L^1$-22)

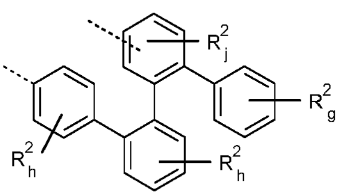

Formel ($L^1$-23)

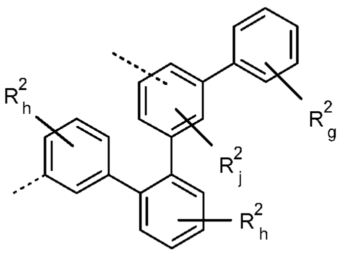

Formel ($L^1$-24)

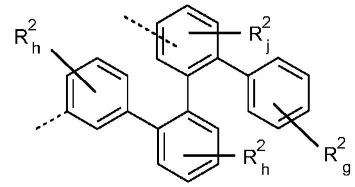

Formel ($L^1$-25)

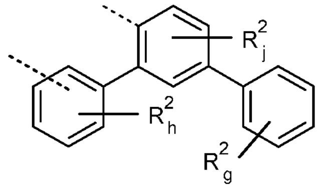

Formel ($L^1$-26)

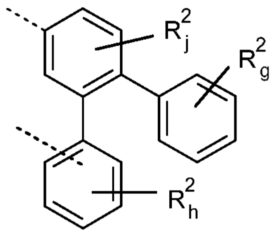

Formel ($L^1$-27)

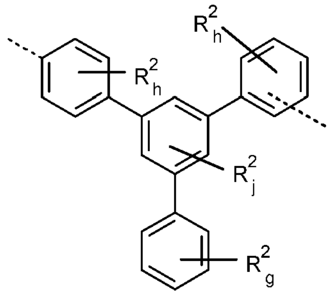

Formel ($L^1$-28)

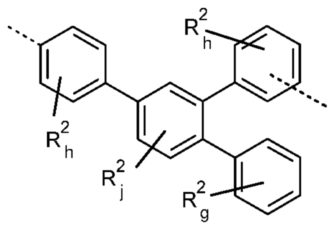

Formel ($L^1$-29)

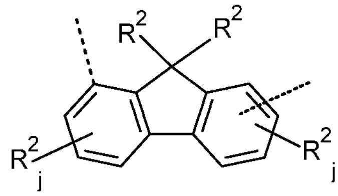

Formel ($L^1$-30)

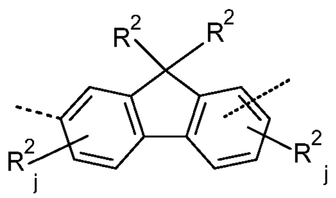

Formel ($L^1$-31)

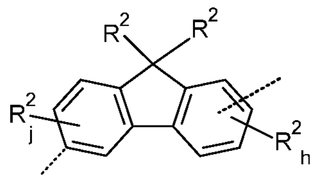

Formel ($L^1$-32)

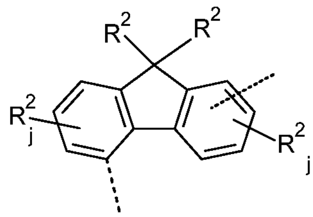

Formel ($L^1$-33)

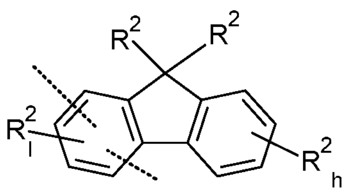

Formel ($L^1$-34)

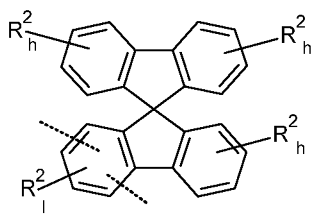

Formel ($L^1$-35)

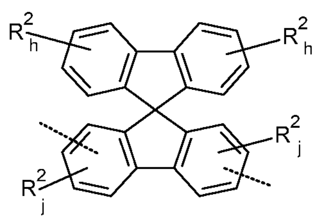

Formel ($L^1$-36)

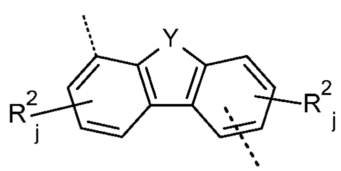

Formel ($L^1$-37)

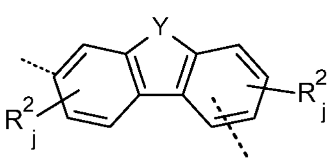

Formel ($L^1$-38)

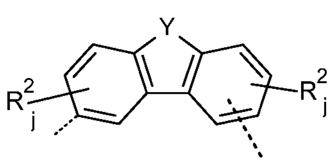

Formel ($L^1$-39)

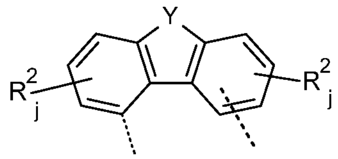

Formel ($L^1$-40)

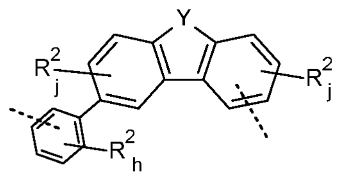

Formel ($L^1$-41)

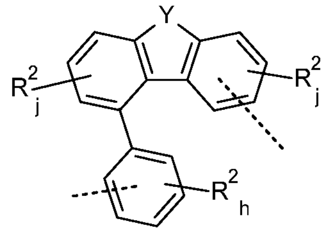

Formel ($L^1$-42)

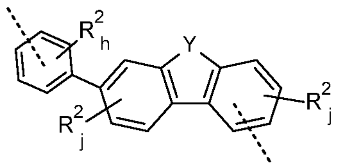

Formel ($L^1$-43)

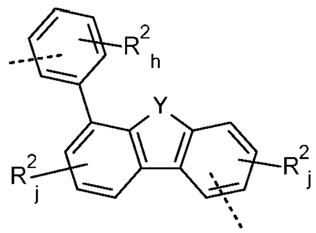

Formel ($L^1$-44)

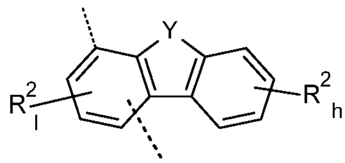

Formel ($L^1$-45)

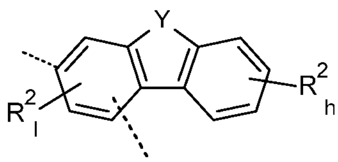

Formel ($L^1$-46)

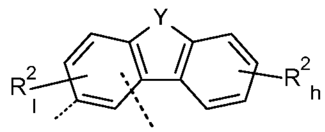

Formel ($L^1$-47)

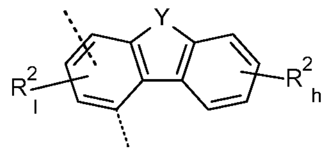

Formel ($L^1$-48)

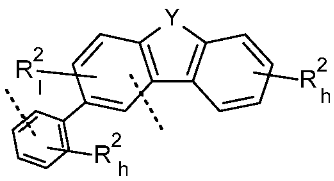

Formel ($L^1$-49)

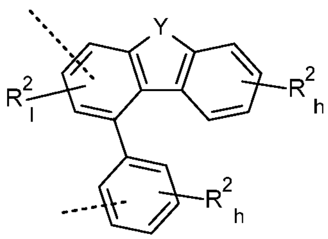

Formel ($L^1$-50)

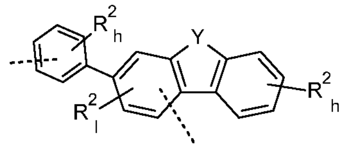

Formel ($L^1$-51)

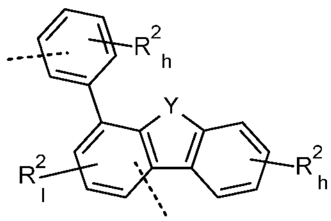

Formel ($L^1$-52)

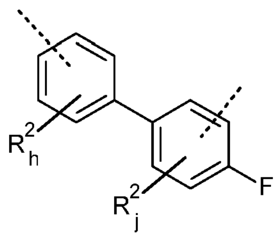

Formel ($L^1$-53)

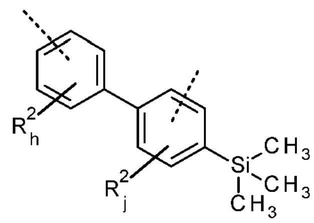

Formel ($L^1$-54)

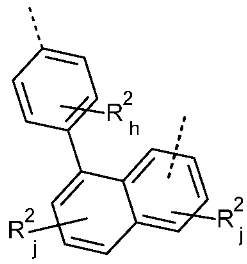

Formel ($L^1$-55)

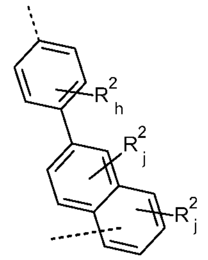

Formel ($L^1$-56)

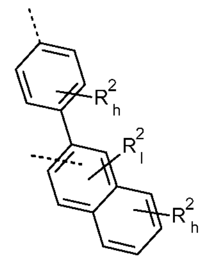

Formel ($L^1$-57)

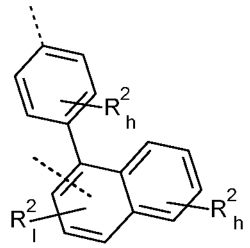

Formel ($L^1$-58)

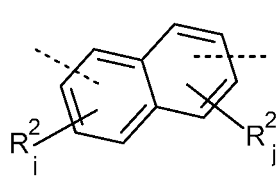

Formel ($L^1$-59)

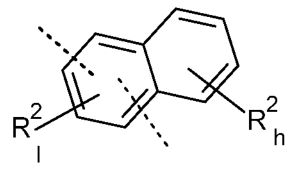

Formel ($L^1$-60)

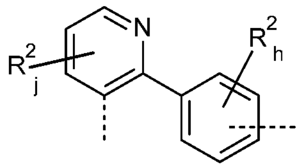

Formel ($L^1$-61)

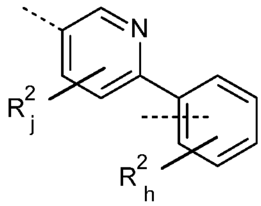

Formel ($L^1$-62)

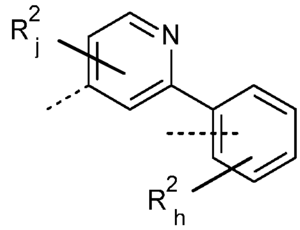

Formel ($L^1$-63)

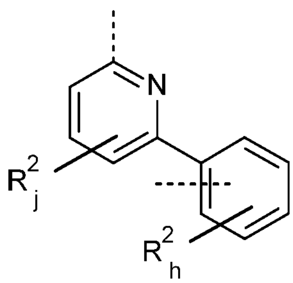

Formel ($L^1$-64)

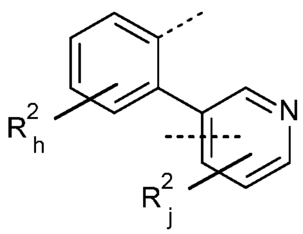

Formel ($L^1$-65)

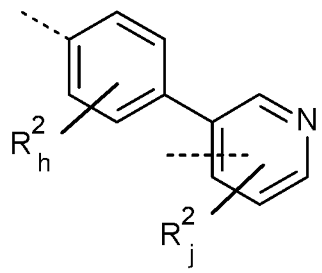

Formel ($L^1$-66)

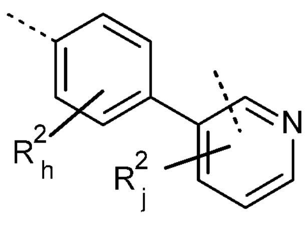

Formel ($L^1$-67)

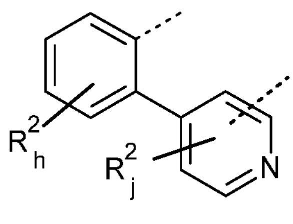

Formel ($L^1$-68)

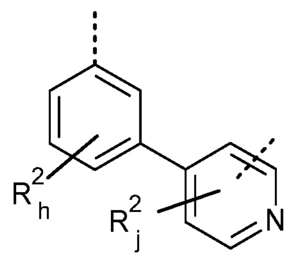

Formel ($L^1$-69)

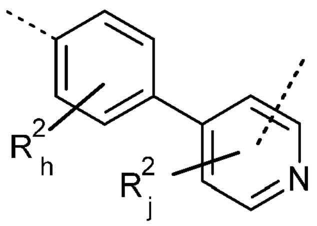

Formel ($L^1$-70)

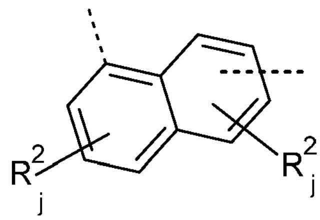

Formel ($L^1$-71)

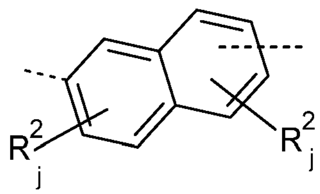

Formel ($L^1$-72)

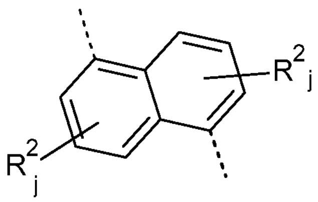

Formel ($L^1$-73)

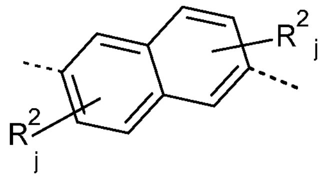

Formel ($L^1$-74)

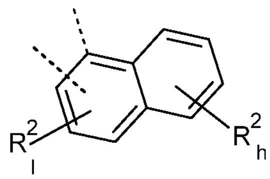

Formel ($L^1$-75)

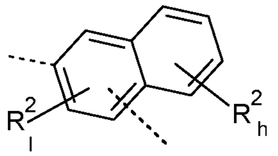

Formel ($L^1$-76)

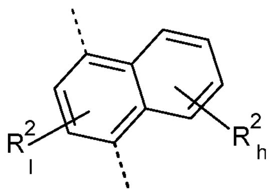

Formel ($L^1$-77)

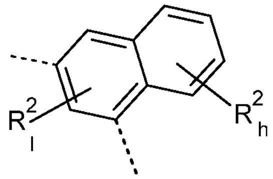

Formel ($L^1$-78)

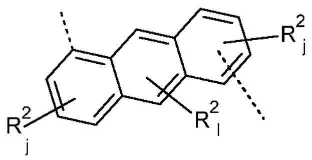

Formel ($L^1$-79)

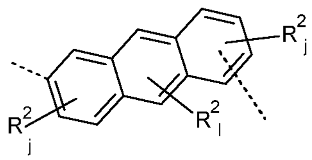

Formel ($L^1$-80)

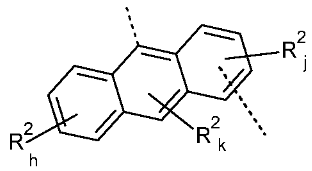

Formel ($L^1$-81)

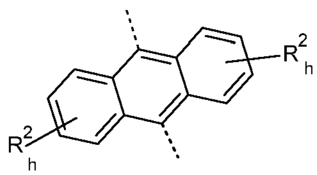

Formel ($L^1$-82)

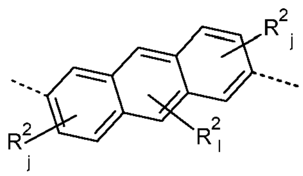

Formel ($L^1$-83)

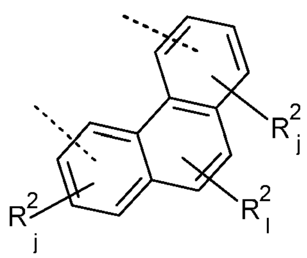

Formel ($L^1$-84)

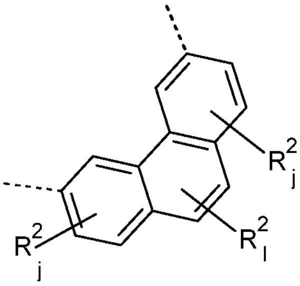

Formel ($L^1$-85)

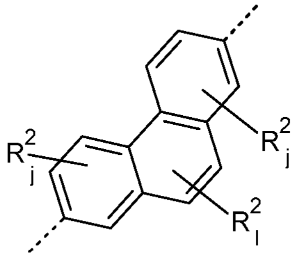

Formel ($L^1$-86)

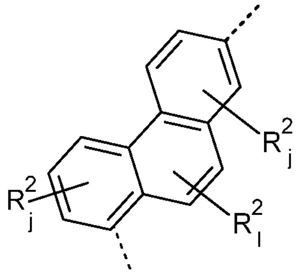

Formel ($L^1$-87)

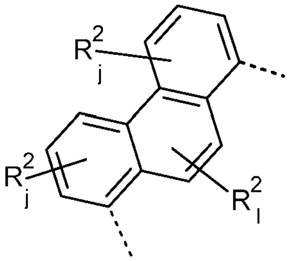

Formel ($L^1$-88)

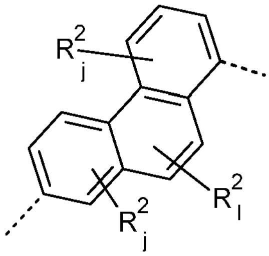

Formel ($L^1$-89)

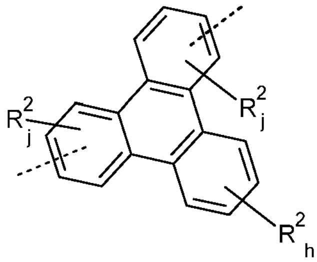

Formel ($L^1$-90)

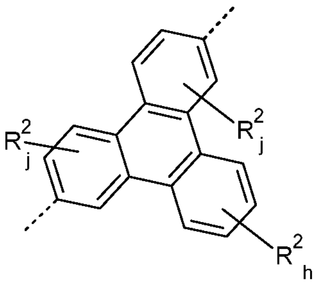

Formel ($L^1$-91)

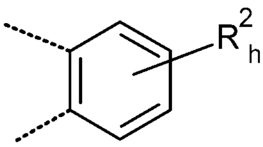

Formel ($L^1$-92)

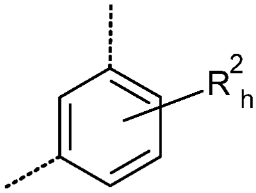

Formel ($L^1$-93)

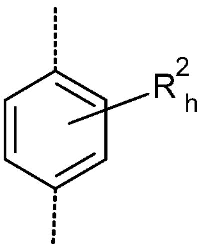

Formel ($L^1$-94)

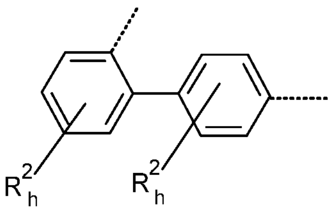

Formel ($L^1$-95)

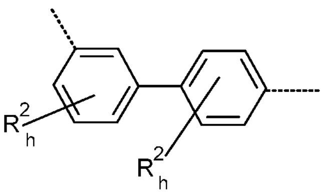

Formel ($L^1$-96)

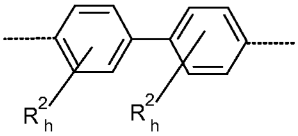

Formel ($L^1$-97)

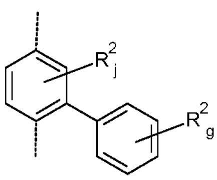

Formel ($L^1$-98)

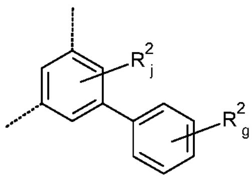

Formel ($L^1$-99)

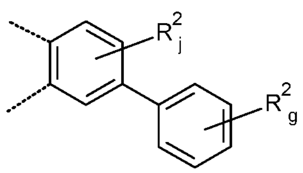

Formel ($L^1$-100)

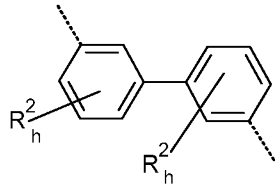

Formel ($L^1$-101)

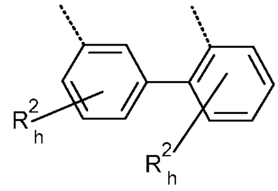

Formel ($L^1$-102)

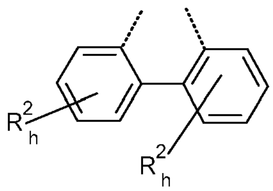

Formel ($L^1$-103)

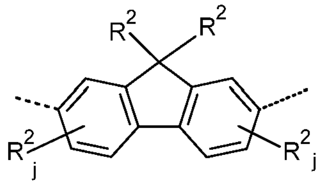

Formel ($L^1$-104)

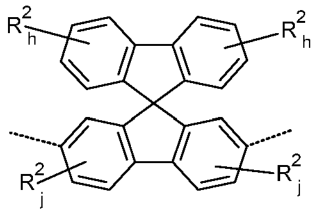

Formel ($L^1$-105)

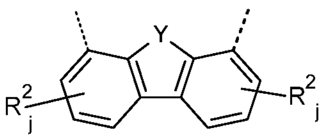

Formel ($L^1$-106)

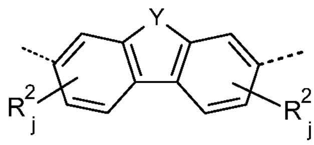

Formel ($L^1$-107)

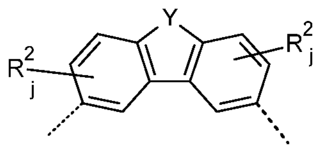

Formel ($L^1$-108)

wobei die gestrichelten Bindungen jeweils die Anbindungspositionen markieren, der Index k 0 oder 1 ist, der Index l 0, 1 oder 2 ist, der Index j bei jedem Auftreten unabhängig 0, 1, 2 oder 3 ist; der Index h bei jedem Auftreten unabhängig 0, 1, 2, 3 oder 4 ist, der Index g 0, 1, 2, 3, 4 oder 5 ist; das Symbol Y O, S oder $NR^2$, vorzugsweise O oder S ist; und das Symbol $R^2$ die zuvor genannte Bedeutung aufweist.

Vorzugsweise kann vorgesehen sein, dass die Summe der Indices k, l, g, h und j in den Strukturen der Formel ($L^1$-1) bis ($L^1$-108) jeweils höchstens 3, vorzugsweise höchstens 2 und besonders bevorzugt höchstens 1 beträgt.

Bevorzugte erfindungsgemäße Verbindungen umfassen eine Gruppe L', die eine Bindung darstellt oder die ausgewählt ist aus einer der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103). Mit Vorteil kann die Summe der Indices k, l, g, h und j in den Strukturen der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103) jeweils höchstens 3, vorzugsweise höchstens 2 und besonders bevorzugt höchstens 1 betragen.

Bevorzugt bilden die Reste $R^2$ in den Formeln ($L^1$-1) bis ($L^1$-108) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

In einer weiteren bevorzugten Ausführungsform der Erfindung ist $R^2$, beispielsweise bei einer Struktur gemäß Formel (II), (IV) und/oder (VI) sowie bevorzugten Ausführungsformen dieser Strukturen oder den Strukturen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

In einer weiteren bevorzugten Ausführungsform der Erfindung ist $R^3$, beispielsweise bei einer Struktur gemäß Formel (II), (IV) und/oder (VI) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, F, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch

ein oder mehrere Alkylgruppen mit jeweils 1 bis 4 Kohlenstoffatomen substituiert sein kann, bevorzugt aber unsubstituiert ist.

Ferner kann vorgesehen sein, dass die erfindungsgemäße Verbindung zwei Strukturelemente mit drei anellierten aromatischen Ringen (AR) aufweist.

Darüber hinaus kann eine erfindungsgemäße Verbindung zwei Strukturelemente mit einem aromatischen oder heteroaromatischen Valerolaktam (AV) umfassen.

Besonders bevorzugt sind Verbindungen der Formeln (IIa), (IIa-1), (IIa-2), (IIa-3), (IIa-4), (IIa-5), (IVa), (IVa-1), (IVa-2), (IVa-3), (IVa-6), (IVa-7), (IVa-8), (IVa-9), (VIa), (VIa-1), (VIa-2), (VIa-3), (VIa-6), (VIa-7), (VIa-8) und/oder (VIa-9), worin $L^1$ eine Bindung darstellt und die Summe der Indices f, k, l, m, n, o und p höchstens 5, vorzugsweise höchstens 3 und besonders bevorzugt höchstens 1 beträgt.

Weiterhin sind Verbindungen gemäß den Formeln (IIa), (IIa-1), (IIa-3), (IIa-4) und/oder (IIa-5) bevorzugt, bei denen die Gruppe $L^1$ eine Bindung darstellt und die Summe der Indices f, k, l, m, n, o und p höchstens 5, vorzugsweise höchstens 3 und besonders bevorzugt höchstens 1 beträgt. Im Falle der Strukturen der Formeln (IIa-1), (IIa-4) und/oder (IIa-5) ist p vorzugsweise 1, wobei die Gruppe $R^1$, für die p 1 ist, vorzugsweise ausgewählt ist aus den Gruppen ($R^1$-1) bis ($R^1$-86), besonders bevorzugt ($R^1$-1) bis ($R^1$-54).

Ferner sind Verbindungen gemäß den Formeln (IVa-1), (IVa-2), (IVa-3), (VIa-1), (VIa-2), (VIa-3) bevorzugt, bei denen die Anbindungsstelle am Strukturelement (AV) in para-Stellung zur Bindungsstelle des Stickstoffatoms der Amidgruppe steht, wie dies in Formel (VIa-12) dargestellt ist, und bei denen die Gruppe $L^1$ eine Bindung darstellt und die Summe der Indices m, n, o und p höchstens 5, vorzugsweise höchstens 3 und besonders bevorzugt höchstens 1 beträgt. Im Falle der Strukturen der Formel (IVa-1) ist p vorzugsweise 1, wobei die Gruppe $R^1$, für die p 1 ist, vorzugsweise ausgewählt ist aus den Gruppen ($R^1$-1) bis ($R^1$-86), besonders bevorzugt ($R^1$-1) bis ($R^1$-54). Im Falle der Strukturen der Formeln (IVa-2), (IVa-3), ist o vorzugsweise 2, wobei die Gruppen $R^1$, für die o 2 ist, vorzugsweise ausgewählt jeweils sind aus den Gruppen ($R^1$-1) bis ($R^1$-86), besonders bevorzugt ($R^1$-1) bis ($R^1$-54).

Besonders bevorzugt sind Verbindungen der Formeln (IIa), (IIa-1), (IIa-2), (IIa-3), (IIa-4), (IIa-5), (IVa), (IVa-1), (IVa-2), (IVa-3), (IVa-6), (IVa-7), (IVa-8), (IVa-9), (VIa), (VIa-1), (VIa-2), (VIa-3), (VIa-6), (VIa-7), (VIa-8) und/oder (VIa-9), worin $L^1$ eine Gruppe der Formel ($L^1$-1) darstellt und die Summe der Indices f, k, l, m, n, o und p höchstens 7, vorzugsweise höchstens 6 und besonders bevorzugt höchstens 5 beträgt.

Weiterhin sind Verbindungen gemäß den Formeln (IIa-1), (IIa-3), (IIa-4) und/oder (IIa-5) bevorzugt, bei denen die Gruppe $L^1$ eine Gruppe der Formel ($L^1$-1) darstellt und die Summe der Indices f, k, l, m, n, o und p höchstens 5, vorzugsweise höchstens 3 und besonders bevorzugt höchstens 1 beträgt. Im Falle der Strukturen der Formeln (IIa-1), (IIa-4) und/oder (IIa-5) ist p vorzugsweise 1, wobei die Gruppe $R^1$, für die p 1 ist, vorzugsweise ausgewählt ist aus den Gruppen ($R^1$-1) bis ($R^1$-86), besonders bevorzugt ($R^1$-1) bis ($R^1$-54).

Ferner sind Verbindungen gemäß den Formeln (IVa-1), (IVa-2), (IVa-3), (VIa-1), (VIa-2), (VIa-3) bevorzugt, bei denen die Anbindungsstelle am Strukturelement (AV) in para-Stellung zur Bindungsstelle des Stickstoffatoms der Amidgruppe steht, wie dies in Formel (VIa-8) dargestellt ist, und bei denen die Gruppe $L^1$ eine Gruppe der Formel ($L^1$-1) darstellt und die Summe der Indices m, n, o und p höchstens 5, vorzugsweise höchstens 3 und besonders bevorzugt höchstens 1 beträgt. Im Falle der Strukturen der Formel (IVa-1) ist p vorzugsweise 1, wobei die Gruppe $R^1$, für die p 1 ist, vorzugsweise ausgewählt ist aus den Gruppen ($R^1$-1) bis ($R^1$-86), besonders bevorzugt ($R^1$-1) bis ($R^1$-54). Im Falle der Strukturen der Formeln (IVa-2), (IVa-3), ist o vorzugsweise 2, wobei die Gruppen $R^1$, für die o 2 ist, vorzugsweise ausgewählt jeweils sind aus den Gruppen ($R^1$-1) bis ($R^1$-86), besonders bevorzugt ($R^1$-1) bis ($R^1$-54).

Beispiele für geeignete erfindungsgemäße Verbindungen sind die nachstehend gezeigten Strukturen gemäß den folgenden Formeln 1 bis 91, 106 bis 135, 141, 154 bis 156, 175, 179, 183 und 185:

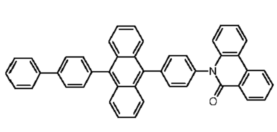

Formel 1

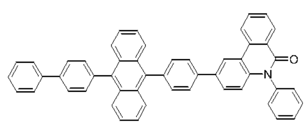

Formel 2

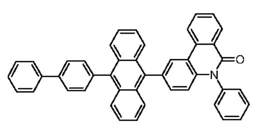

Formel 3

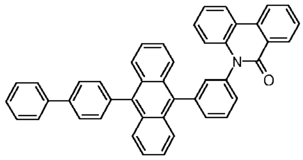

Formel 4

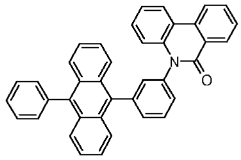

Formel 5

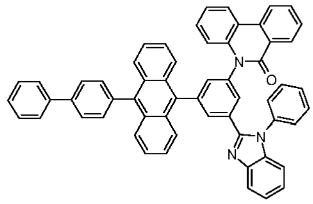

Formel 6

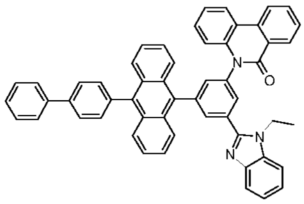

Formel 7

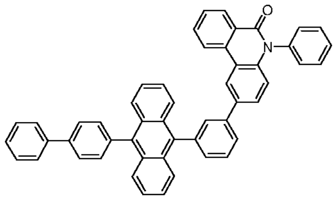

Formel 8

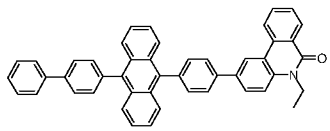

Formel 9

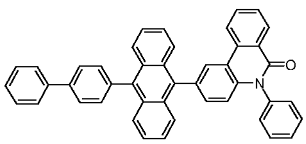

Formel 10

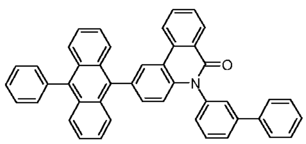

Formel 11

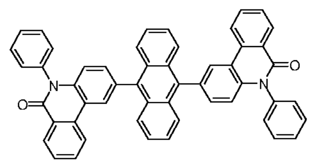

Formel 12

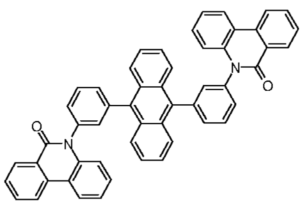

Formel 13

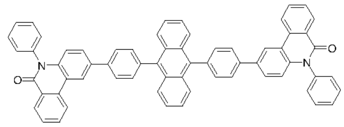

Formel 14

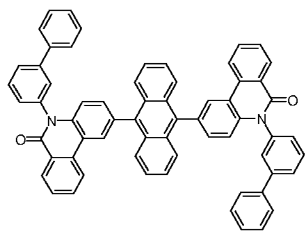

Formel 15

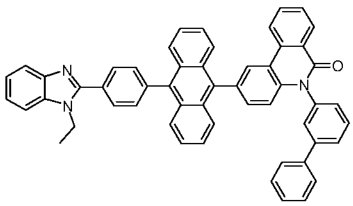

Formel 16

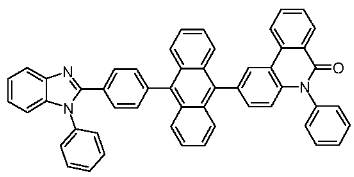

Formel 17

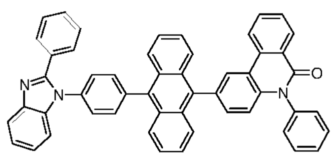

Formel 18

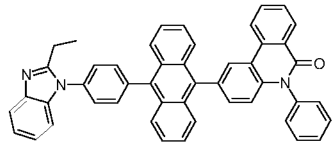

Formel 19

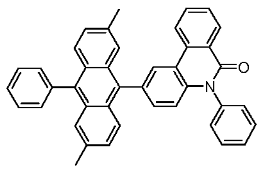

Formel 20

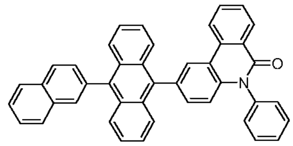

Formel 21

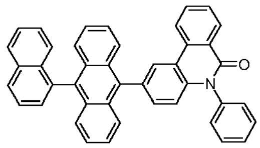

Formel 22

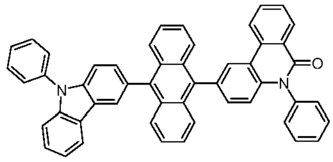

Formel 23

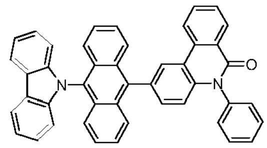

Formel 24

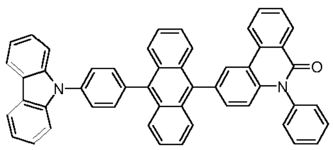

Formel 25

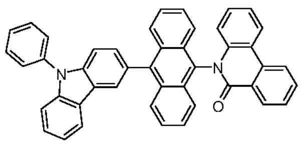

Formel 26

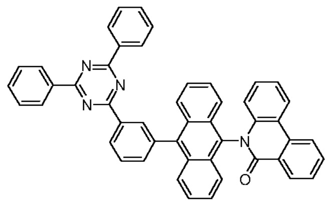

Formel 27

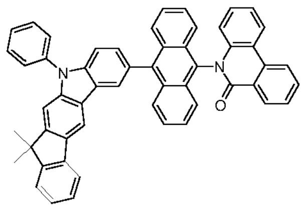

Formel 28

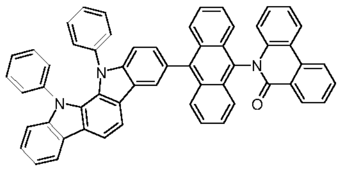

Formel 29

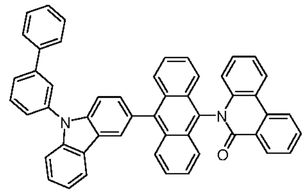

Formel 30

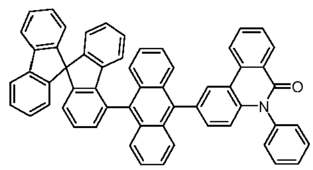

Formel 31

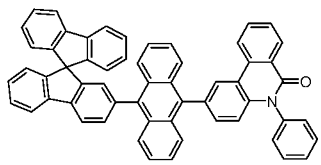

Formel 32

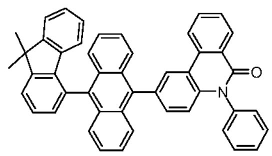

Formel 33

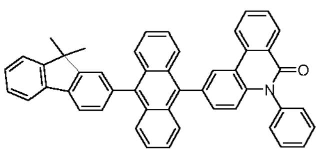

Formel 34

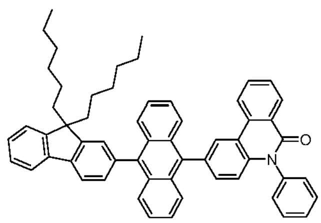

Formel 35

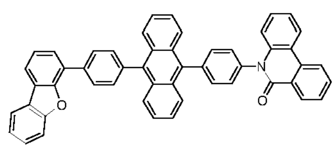

Formel 36

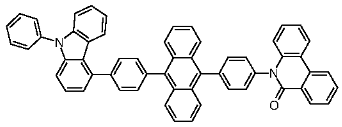

Formel 37

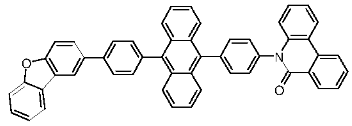

Formel 38

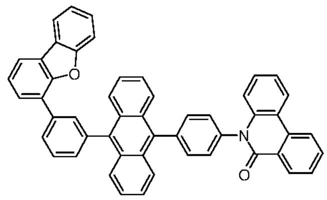

Formel 39

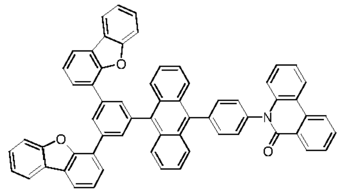

Formel 40

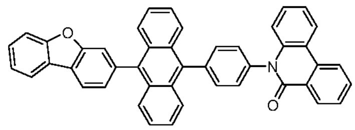

Formel 41

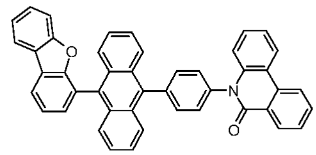

Formel 42

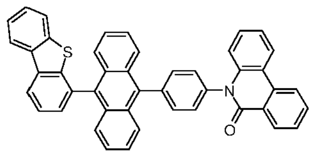

Formel 43

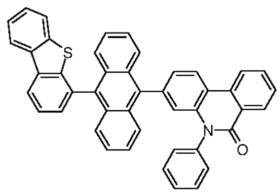

Formel 44

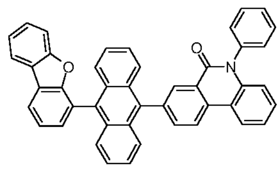

Formel 45

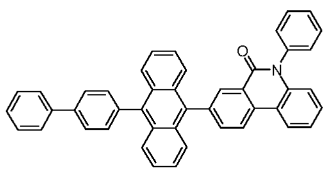

Formel 46

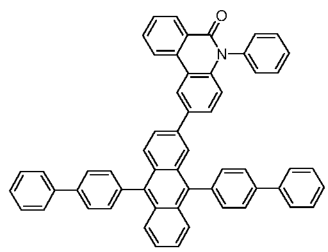

Formel 47

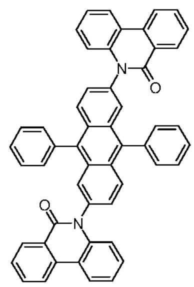

Formel 48

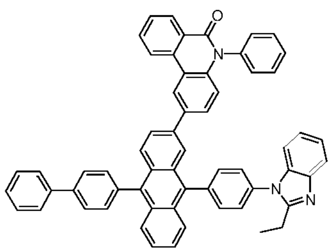

Formel 49

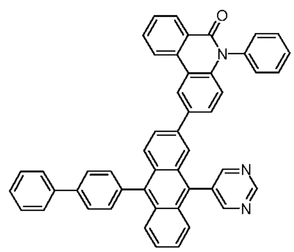

Formel 50

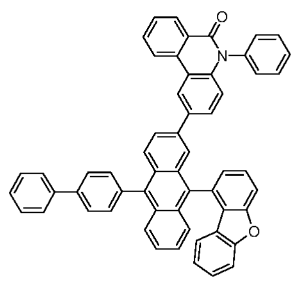

Formel 51

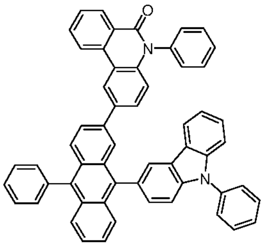

Formel 52

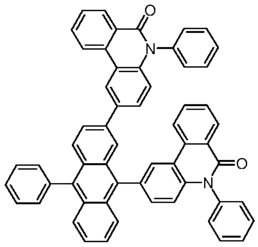

Formel 53

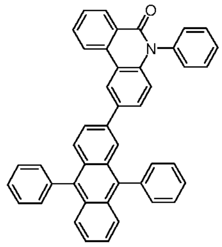

Formel 54

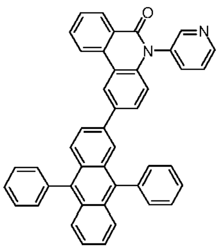

Formel 55

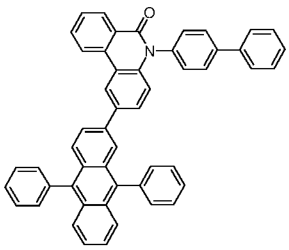

Formel 56

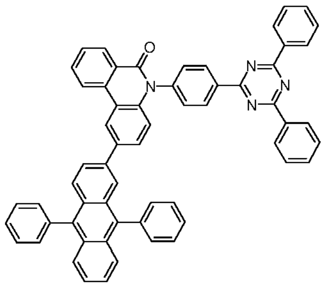

Formel 57

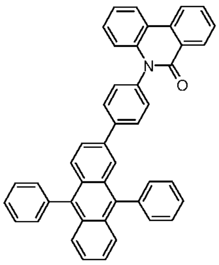

Formel 58

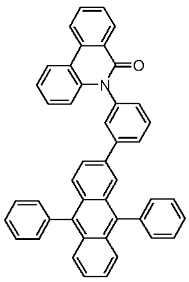

Formel 59

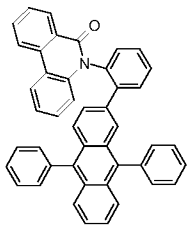

Formel 60

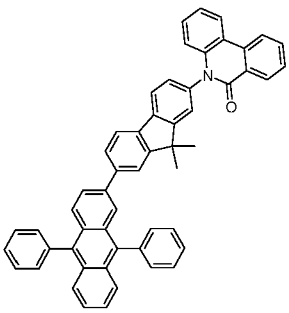

Formel 61

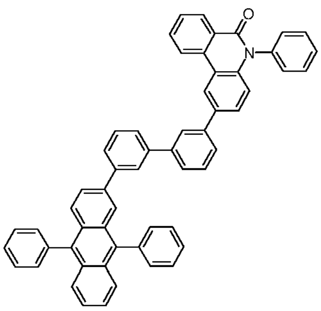

Formel 62

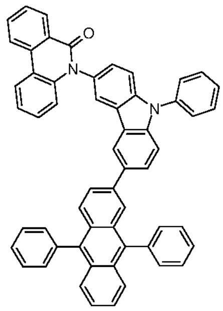

Formel 63

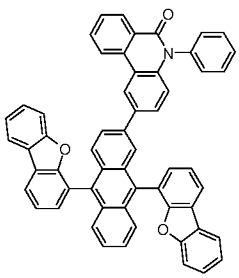

Formel 64

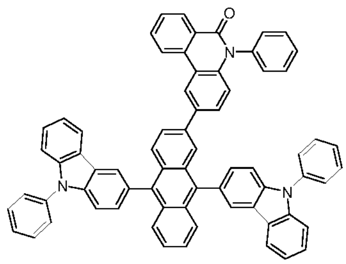

Formel 65

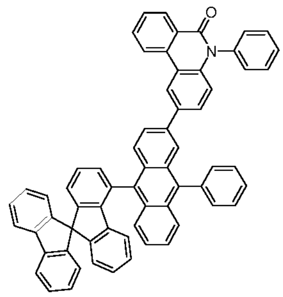

Formel 66

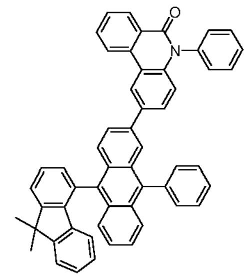

Formel 67

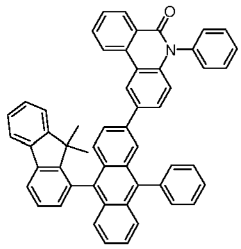

Formel 68

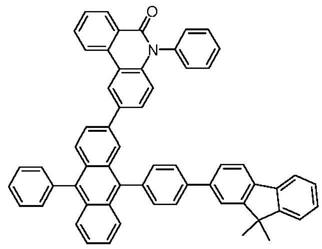

Formel 69

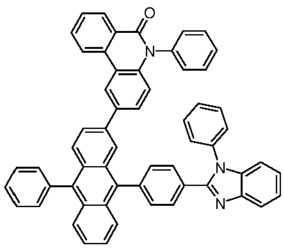

Formel 70

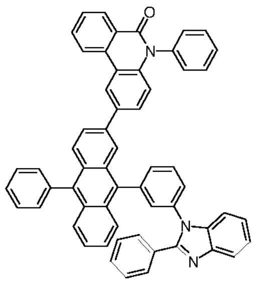

Formel 71

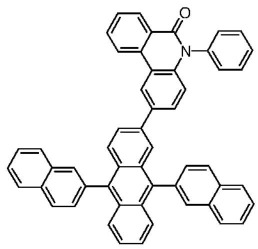

Formel 72

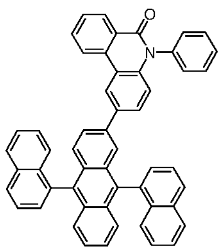

Formel 73

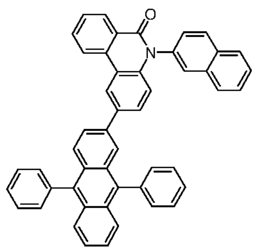

Formel 74

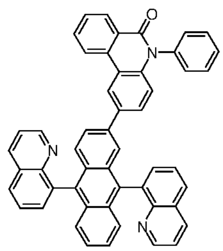

Formel 75

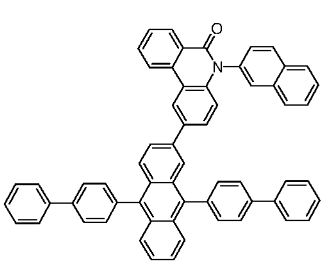

Formel 76

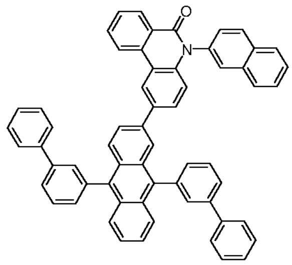

Formel 77

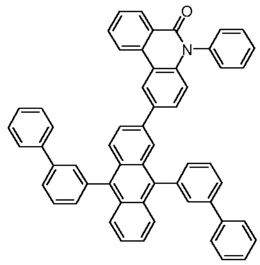

Formel 78

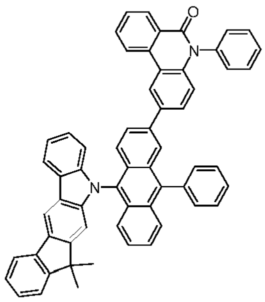

Formel 79

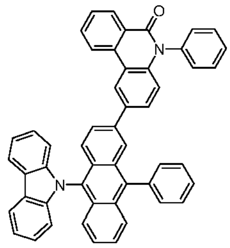

Formel 80

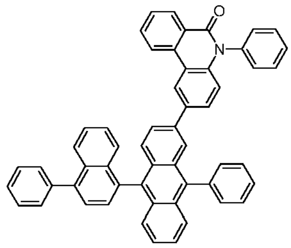

Formel 81

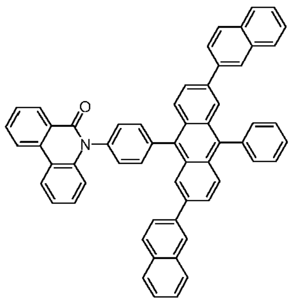

Formel 82

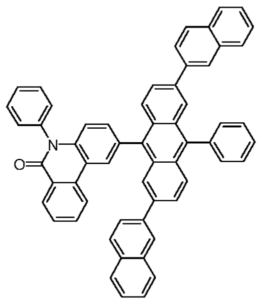

Formel 83

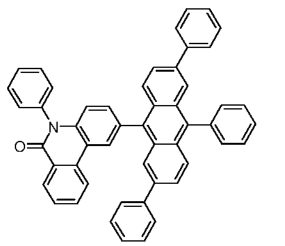

Formel 84

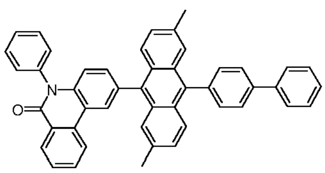

Formel 85

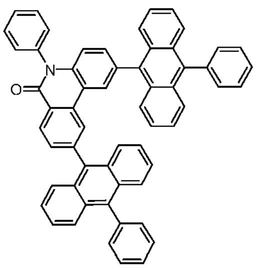

Formel 86

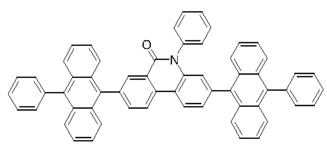

Formel 87

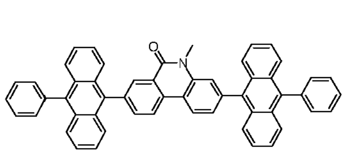

Formel 88

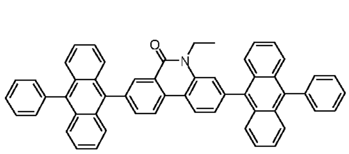

Formel 89

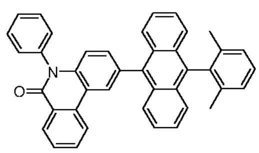

Formel 90

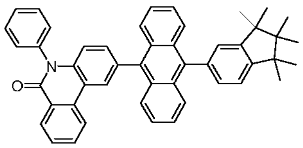

Formel 91

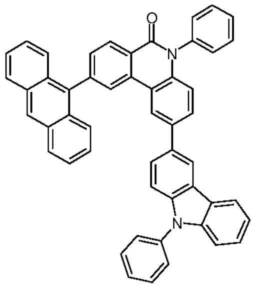

Formel 106

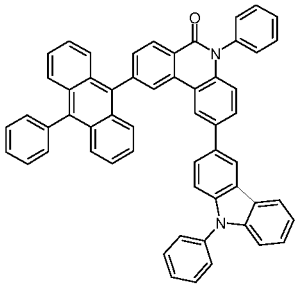

Formel 107

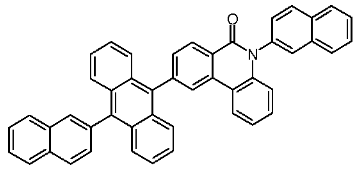

Formel 108

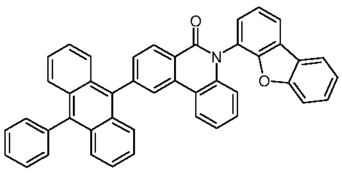

Formel 109

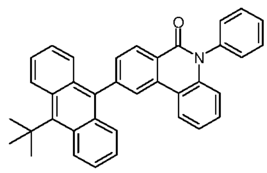

Formel 110

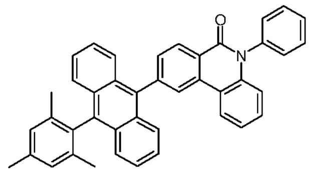

Formel 111

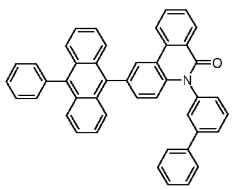

Formel 112

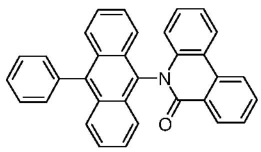

Formel 113

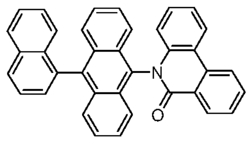

Formel 114

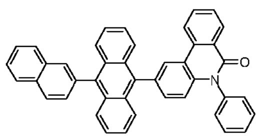

Formel 115

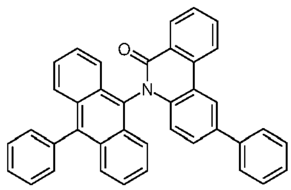

Formel 116

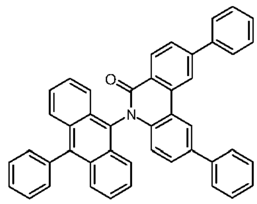

Formel 117

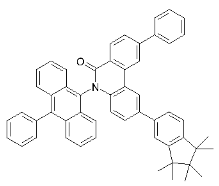

Formel 118

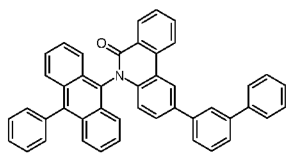

Formel 119

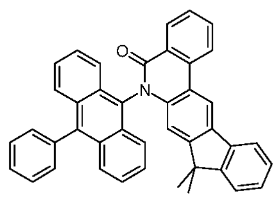

Formel 120

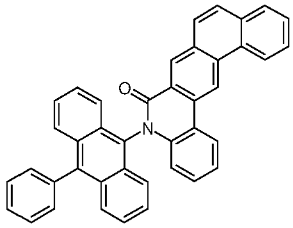

Formel 121

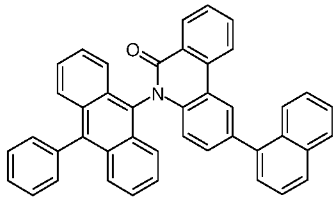

Formel 122

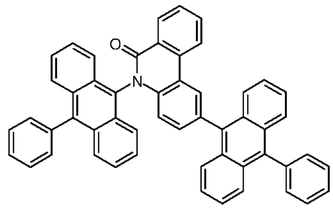

Formel 123

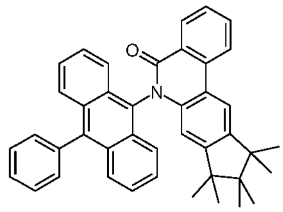

Formel 124

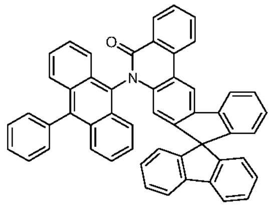

Formel 125

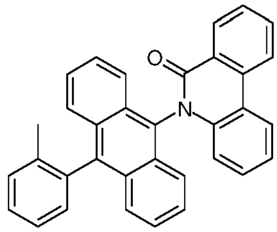

Formel 126

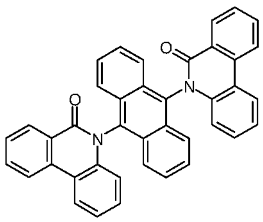

Formel 127

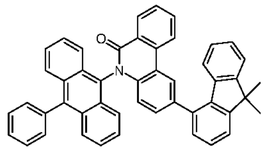

Formel 128

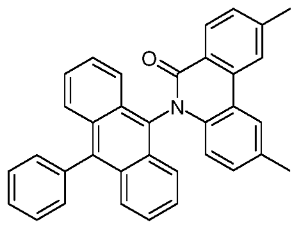

Formel 129

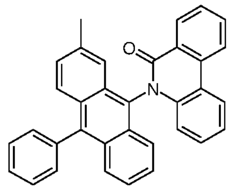

Formel 130

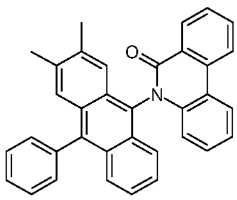

Formel 131

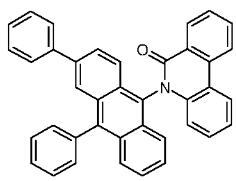

Formel 132

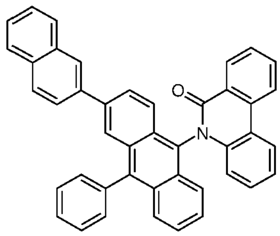

Formel 133

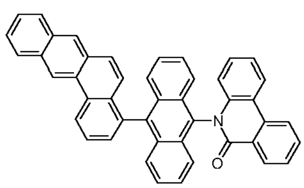

Formel 134

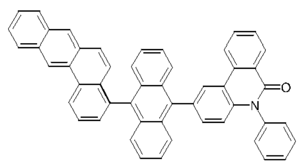

Formel 135

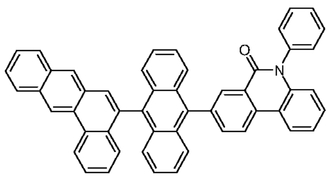

Formel 141

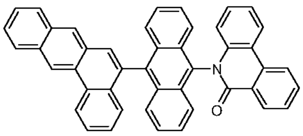

Formel 154

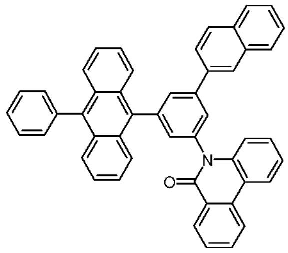

Formel 155

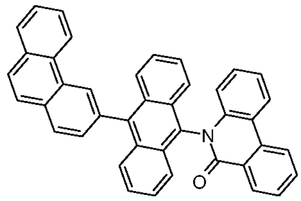

Formel 156

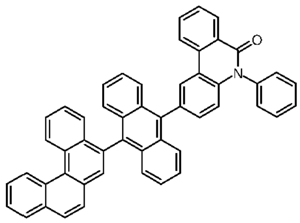

Formel 175

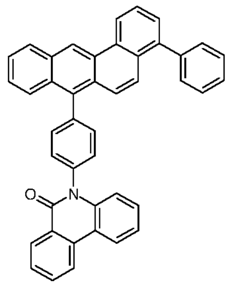

Formel 179

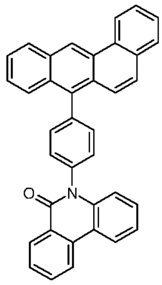

Formel 183

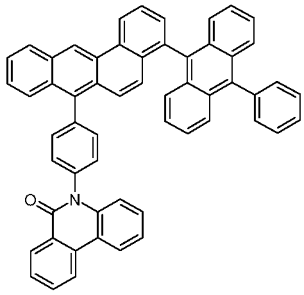

Formel 185

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der erfindungsgemäßen Verbindungen, bei dem in einer Kupplungsreaktion eine Verbindung, umfassend mindestens ein Strukturelement mit drei anellierten aromatischen Ringen (AR), mit einer Verbindung, umfassend mindestens ein Strukturelement mit einem aromatischen Valerolaktam (AV), umgesetzt wird.

Geeignete Verbindungen mit einer aromatischen Valerolaktam-Gruppe können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Arylverbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Durch diese Verfahren, gegebenenfalls gefolgt von Aufreinigung, wie z. B. Umkristallisation oder Sublimation, lassen sich die erfindungsgemäßen Verbindungen, umfassend mindestens ein Strukturelement mit drei anellierten aromatischen Ringen (AR) und mindestens ein Strukturelement mit einem aromatischen Valerolaktam (AV), in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration zu lösen, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens ein Strukturelement mit drei anellierten aromatischen Ringen (AR) und mindestens ein Strukturelement mit einem aromatischen Valerolaktam (AV), bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend ein oder mehrere der oben aufgeführten Strukturelemente mit drei anellierten aromatischen Ringen (AR) und ein oder mehrere

der oben aufgeführten Strukturelemente mit einem aromatischen Valerolaktam (AV) oder erfindungsgemäße Verbindungen, wobei ein oder mehrere Bindungen der erfindungsgemäßen Verbindungen oder der Strukturen der Formel (II), (IV) und/oder (VI) zum Polymer, Oligomer oder Dendrimer vorhanden sind. Je nach Verknüpfung der Strukturen der Formel (II), (IV) und/oder (VI) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (II), (IV) und/oder (VI) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen, umfassend mindestens ein Strukturelement mit drei anellierten aromatischen Ringen (AR) und mindestens ein Strukturelement mit einem aromatischen Valerolaktam (AV), bzw. die zuvor und nachfolgend ausgeführten bevorzugten Ausführungsformen, beispielsweise Verbindungen, umfassend Strukturen der allgemeinen Formel (II), (IV) und/oder (VI), bevorzugt, die eine Glasübergangstemperatur von mindestens 70 °C, beson ders bevorzugt von mindestens 110 °C, ganz besonders bevorzugt von mindestens 125 °C und insbesondere bevorzugt von mindestens 15 0 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethlenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, beispielsweise ein fluoreszierender Dotand, ein phosphoreszierender Dotand oder eine Verbindung, die TADF (thermally activated delayed fluorescence) zeigt, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Host-Materialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden.

Hierbei sind insbesondere Zusammensetzungen von Interesse, die mindestens eine erfindungsgemäße Verbindung und wenigstens ein weiteres Elektroneninjektionsmaterial und/oder Elektronentransportmaterial umfasst.

Das weitere Elektroneninjektionsmaterial und/oder Elektronentransportmaterial unterscheidet sich hierbei von einer erfindungsgemäßen Verbindung. Die vorliegenden Erfindung betrifft daher auch eine Zusammensetzung enthaltend wenigstens eine Verbindung, umfassend mindestens ein Strukturelement mit drei anellierten aromatischen Ringen (AR) und mindestens ein Strukturelement mit einem aromatischen Valerolaktam (AV), bzw. die zuvor und nachfolgend ausgeführten bevorzugten Ausführungsformen sowie wenigstens ein weiteres Elektroneninjektionsmaterial und/oder Elektronentransportmaterial. Vorzugsweise ist das weitere Elektroneninjektionsmaterial und/oder Elektronentransportmaterial ausgewählt aus der Gruppe der Pyridine, Pyrimidine, Triazine, Benzoxazole, Benzimidazole, Anthracene, Lactame, Dibenzofurane, Hydroxychinolinate und Alkalimetallverbindungen. Von den genannten weiteren Verbindungen sind insbesondere sind Triazine, Hydroxychinolinate und Alkalimetallverbindungen bevorzugt, wobei Hydroxychinolinate und Alkalimetallverbindungen besonders bevorzugt sind. Diese Verbindungen sind in der Fachwelt bekannt, wobei bevorzugte Alkalimetallverbindungen insbesondere Lithium enthalten. Bevorzugte Hydroxychinolinate umfassen unter anderem Zr, Al, Hf oder Li. Speziell bevorzugte weitere Elektroneninjektionsmaterialien und/oder Elektronentransportmaterialien sind Hydroxychinolinate, die Lithium enthalten, wobei Hydroxychinolinato-lithium, insbesondere 8-Hydroxychinolinato-lithium (CAS Nr. 25387-93-3) speziell bevorzugt ist.

Hierbei kann das Verhältnis von erfindungsgemäßer Verbindung zu dem weiteren Elektroneninjektionsmaterial und/oder Elektronentransportmaterial vorzugsweise im Bereich von 1:50 bis 50:1, bevorzugt 1:20 bis 20:1, besonders bevorzugt 1:10 bis 10:1, speziell bevorzugt 1:4 bis 4:1 und ganz besonders bevorzugt 1:2 bis 2:1 vorliegen, wobei sich dieses Verhältnis auf das Volumen bezieht, falls alle Verbindungen sublimiert werden können. In anderen Fällen bezieht sich dieses Verhältnis auf das Gewicht der Substanzen.

Die vorliegenden Erfindung betrifft weiterhin auch eine Zusammensetzung enthaltend wenigstens eine Verbindung, umfassend mindestens ein Strukturelement mit drei anellierten aromatischen Ringen (AR) und mindestens ein Strukturelement mit einem aromatischen Valerolaktam (AV), bzw. die zuvor und nachfolgend ausgeführten bevorzugten Ausführungsformen sowie wenigstens ein weiteres Matrixmaterial. Gemäß einem besonderen Aspekt der vorliegenden Erfindung weist das weitere Matrixmaterial lochtransportierende Eigenschaften auf.

Ein weiterer Gegenstand der vorliegenden stellt eine Zusammensetzung dar, enthaltend wenigstens eine Verbindung, umfassend mindestens ein Strukturelement mit drei anellierten aromatischen Ringen (AR) und mindestens ein Strukturelement mit einem aromatischen Valerolaktam (AV), bzw. die zuvor und nachfolgend ausgeführten bevorzugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$\text{HOMO(eV)} = ((\text{HEh}*27.212)-0.9899)/1.1206$$

$$\text{LUMO(eV)} = ((\text{LEh}*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung, umfassend mindestens ein Strukturelement mit drei anellierten aromatischen Ringen (AR) und mindestens ein Strukturelement mit einem aromatischen Valerolaktam (AV), bzw. die zuvor und nachfolgend ausgeführten bevorzugten Ausführungsformen sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angegebenen bevorzugten phosphoreszierenden Dotanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304 und den noch nicht offen gelegten Anmeldungen EP 15182264.0, EP 16179378.1 und EP 16186313.9 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

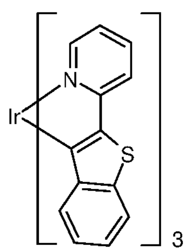
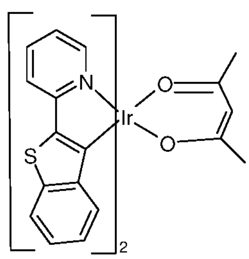
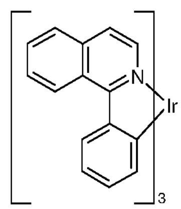
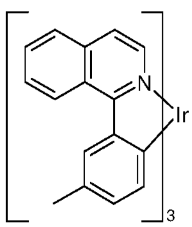
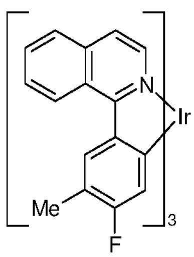
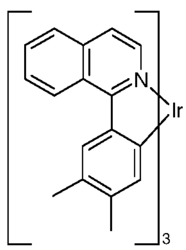

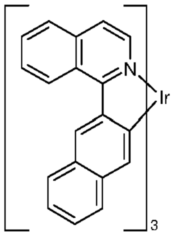
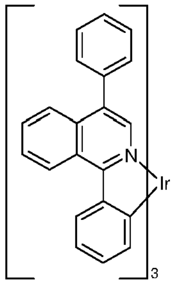
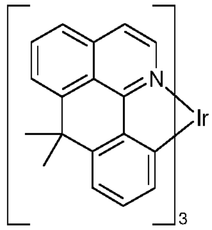

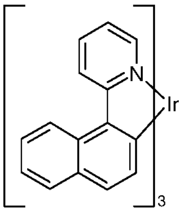
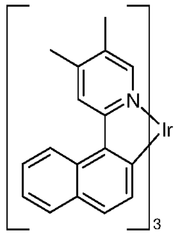
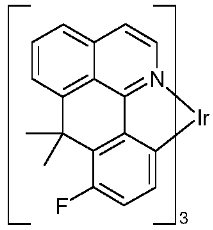

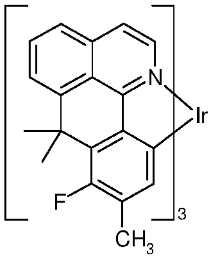
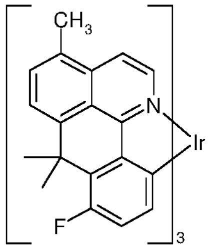
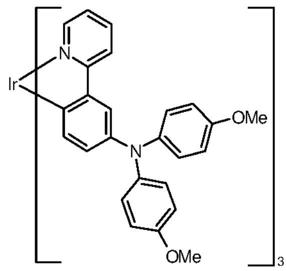

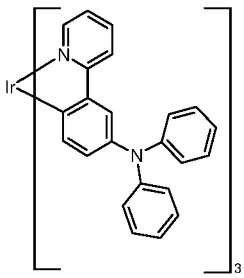
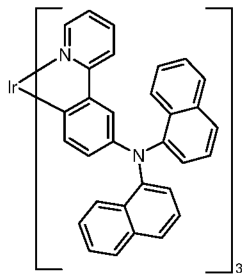
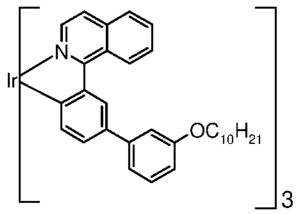

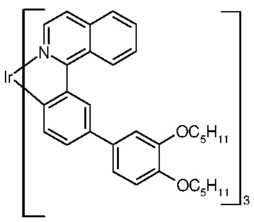
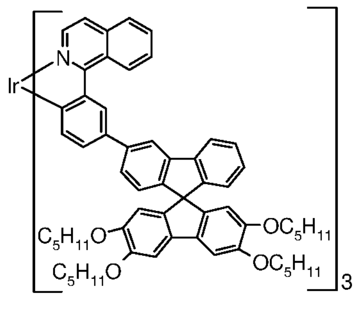
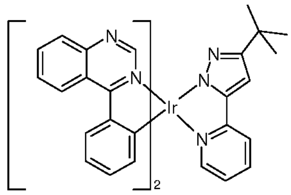

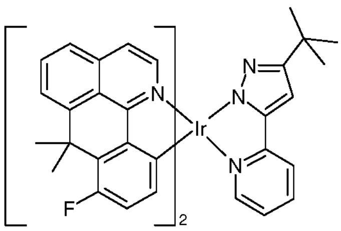
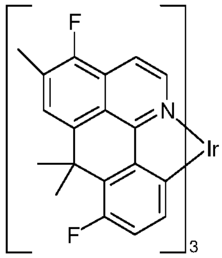
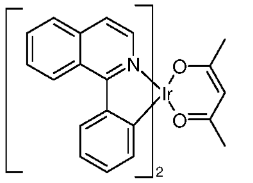

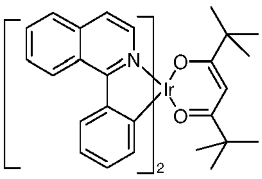
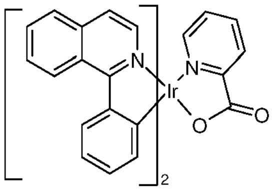
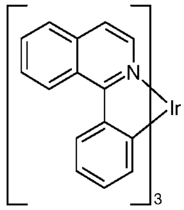

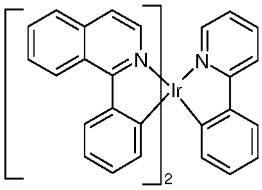
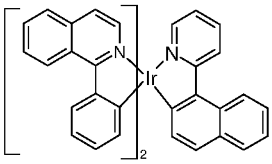
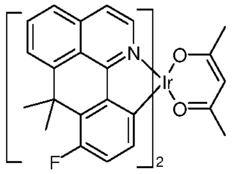

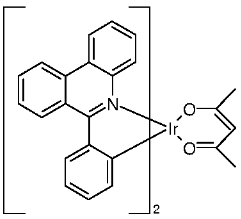
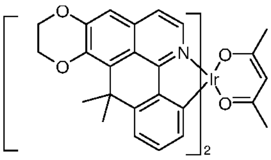
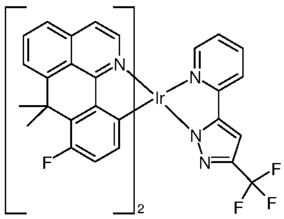

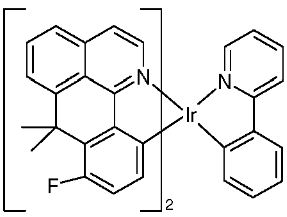
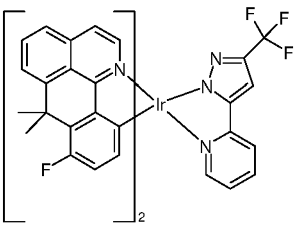
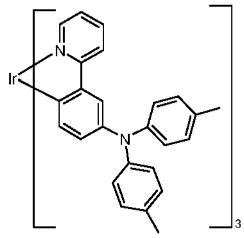

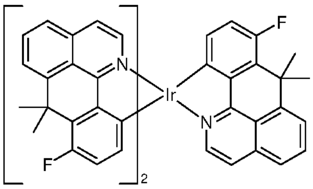
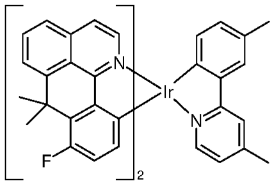
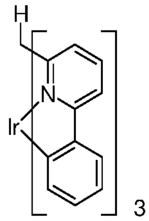

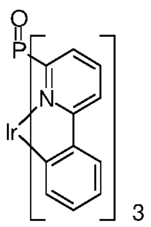
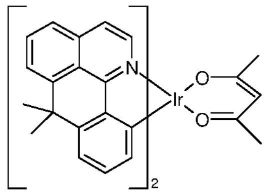
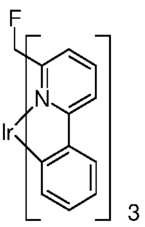

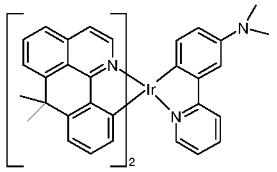
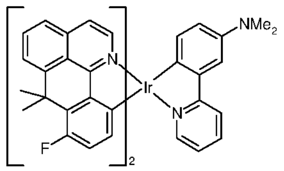
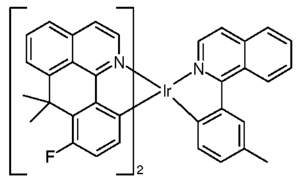

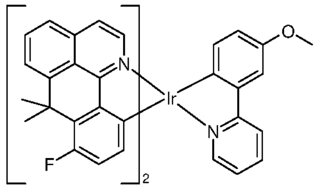
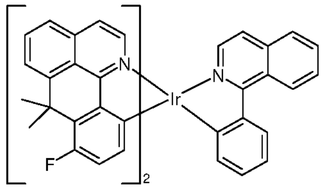
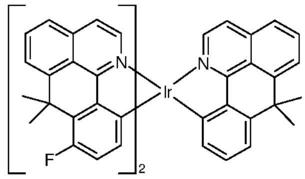

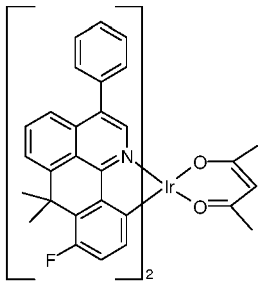
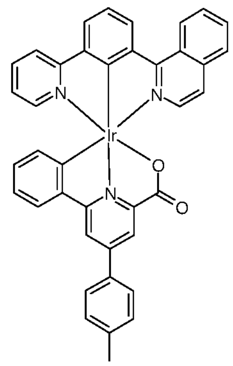
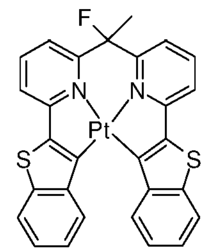

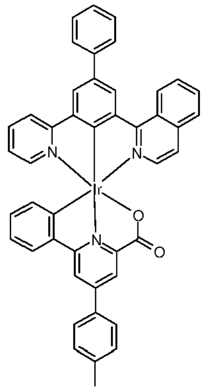
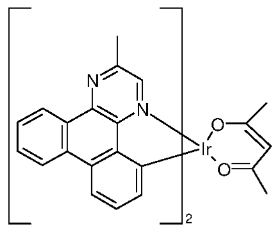
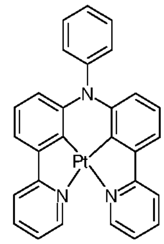

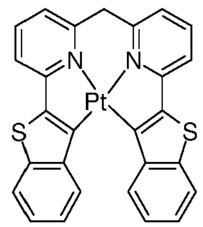
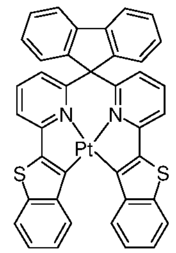
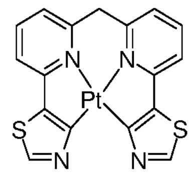

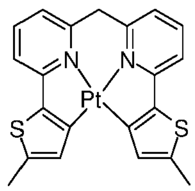
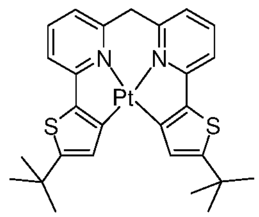
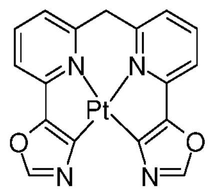

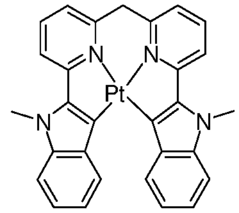
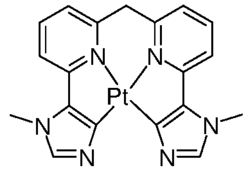
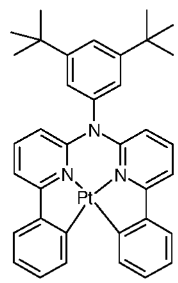

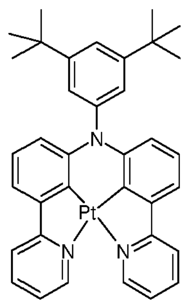
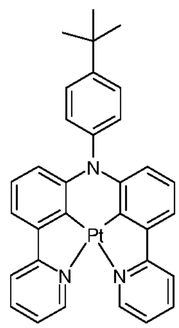
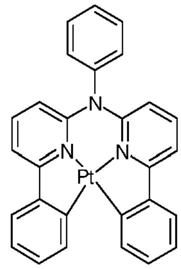

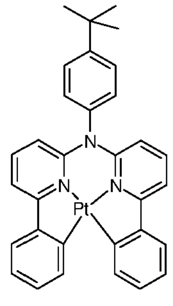
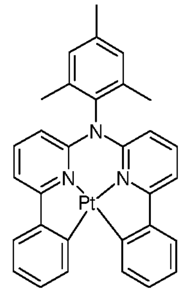
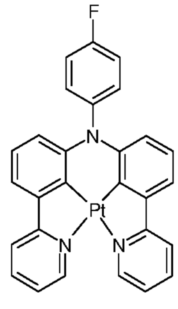

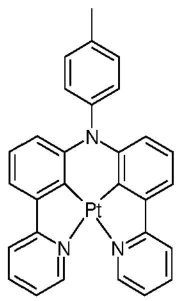
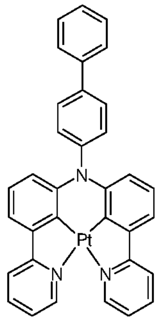
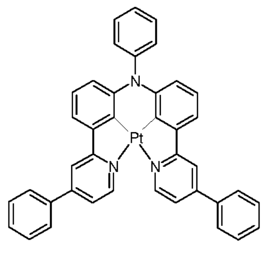
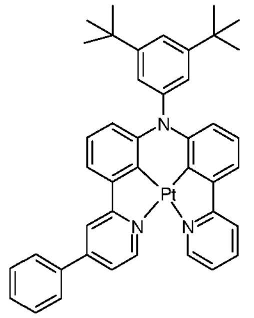
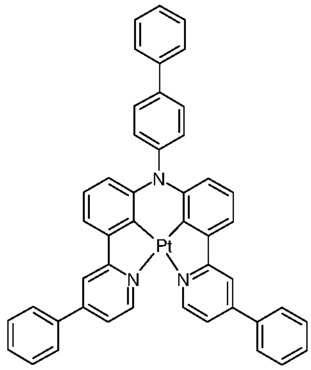
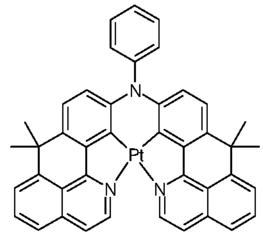
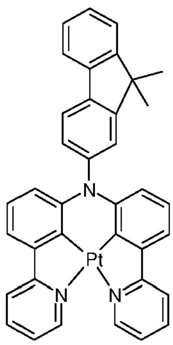
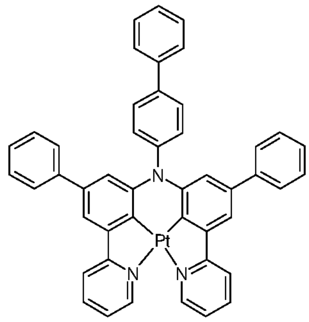
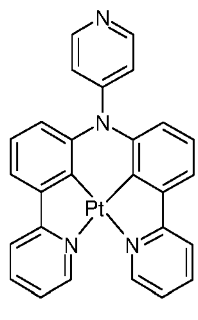
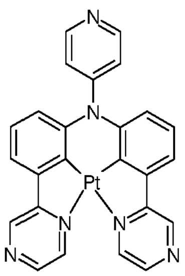
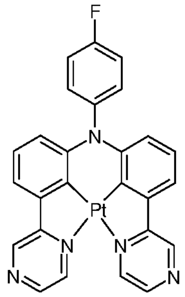
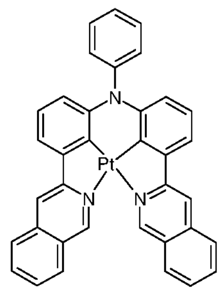
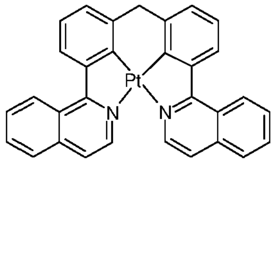
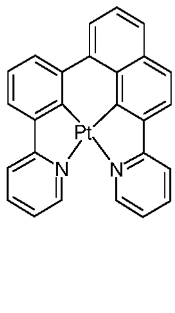
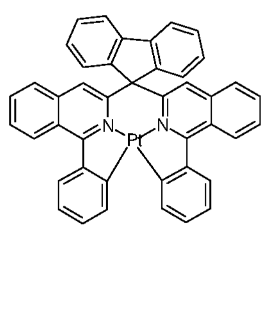

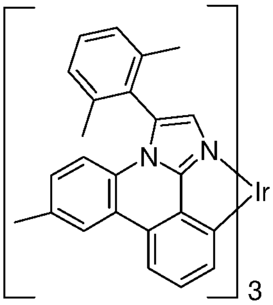
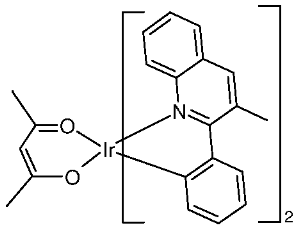
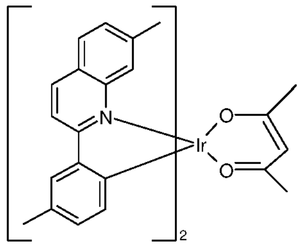

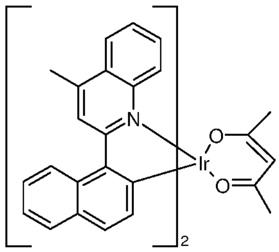
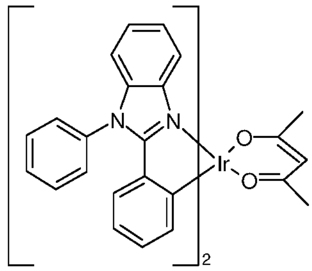
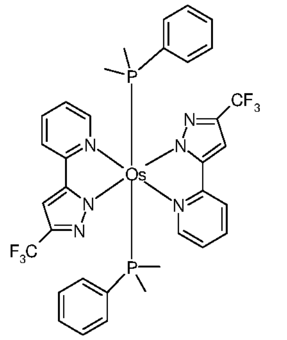

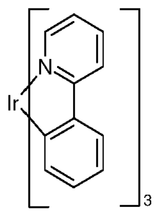
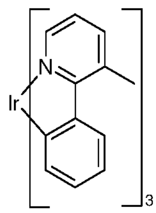
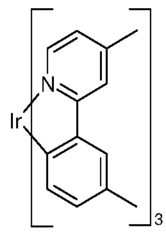

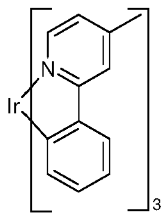
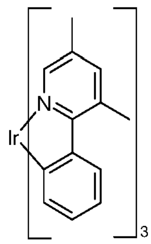
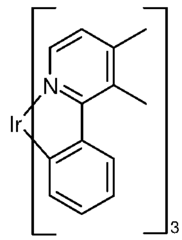

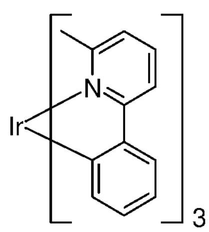
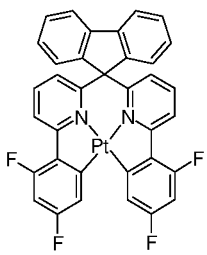
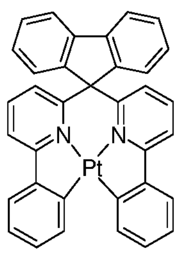

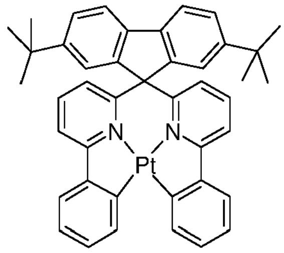

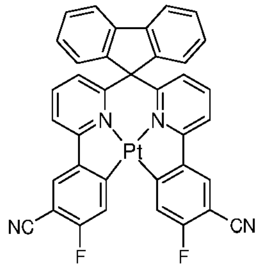

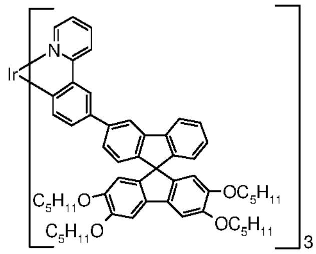

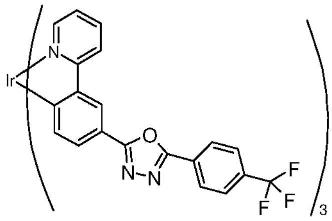

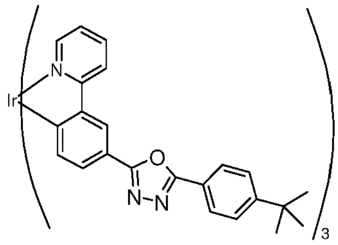

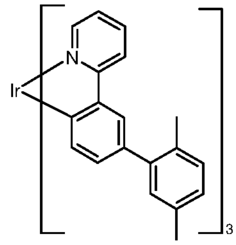

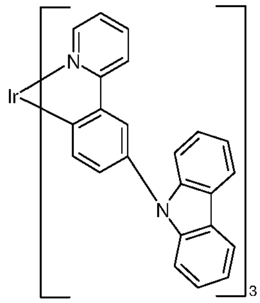

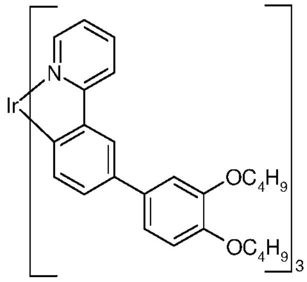

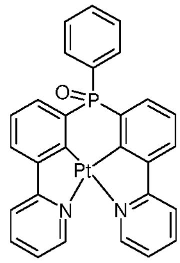

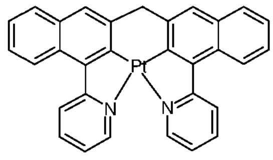

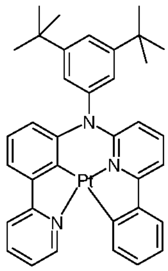

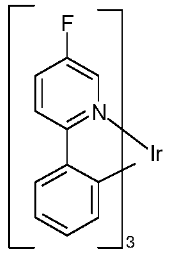

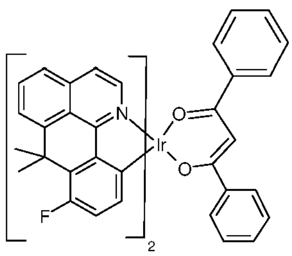

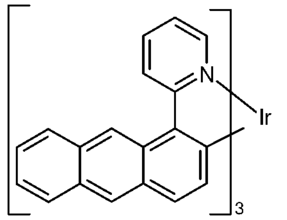

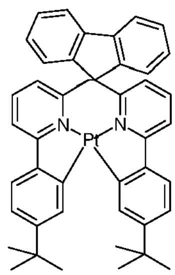

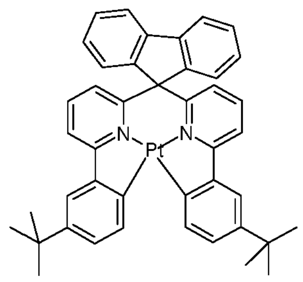
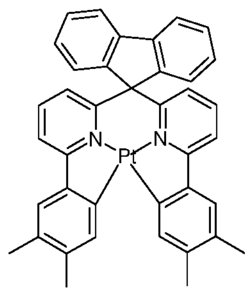
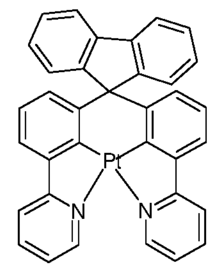

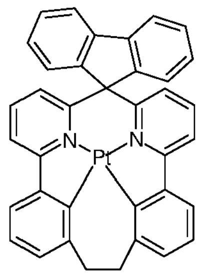
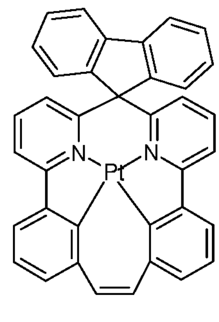
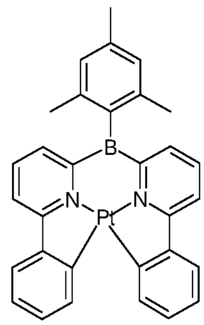

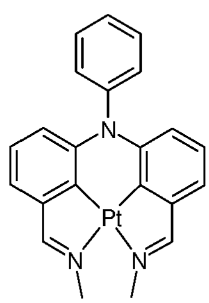
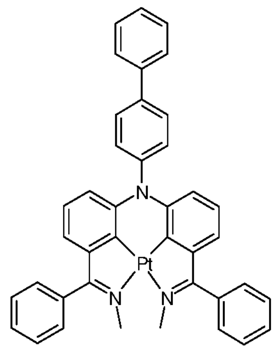
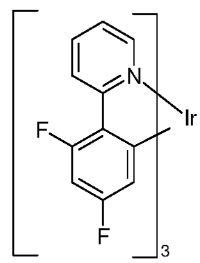

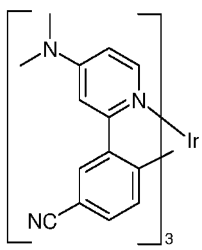
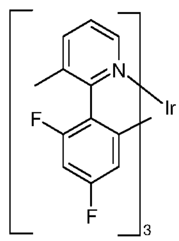
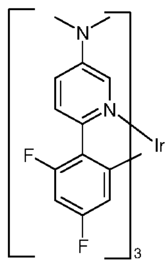

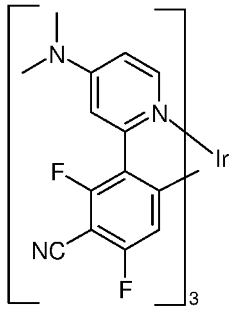
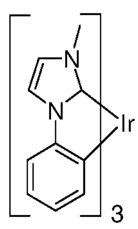
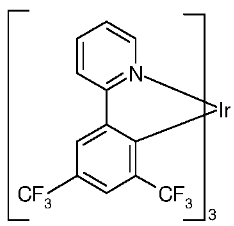

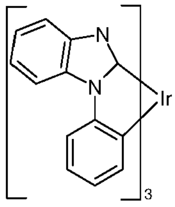
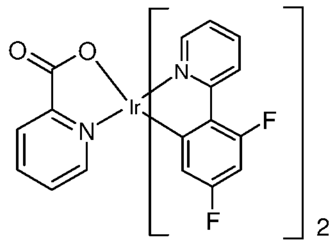
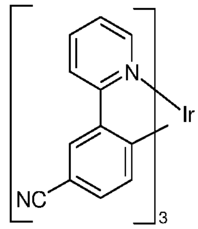

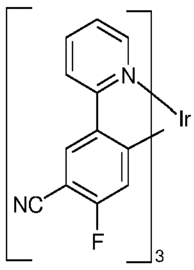
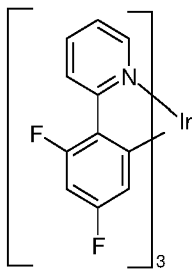
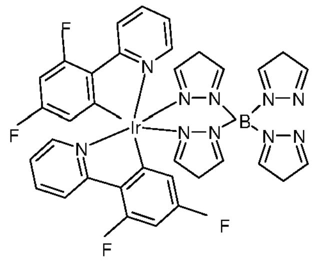

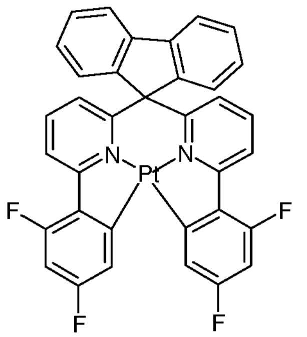
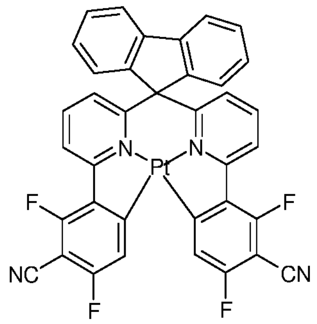
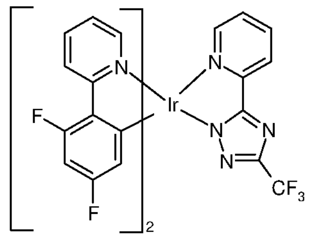

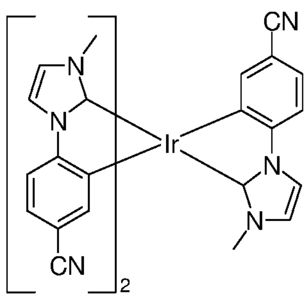
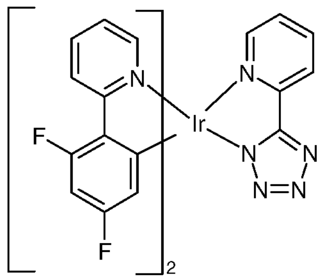
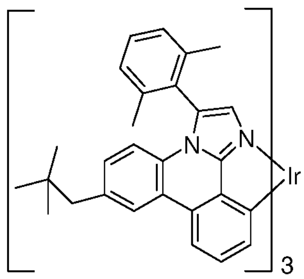

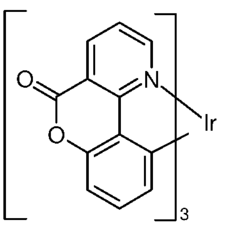
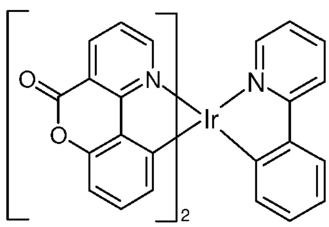
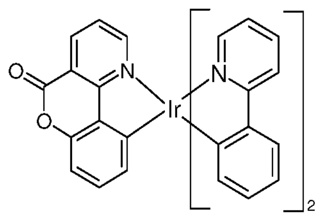

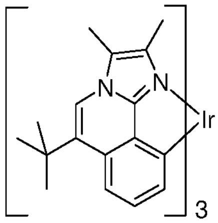
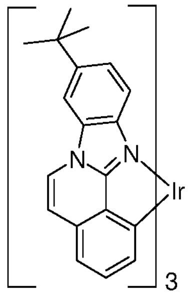
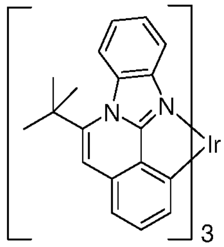

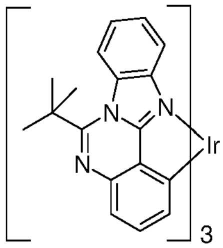
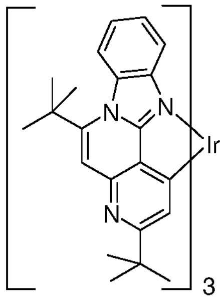
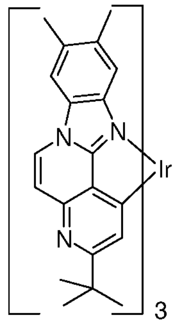

Die oben beschriebenen Verbindungen, umfassend mindestens ein Strukturelement mit drei anellierten aromatischen Ringen (AR) und mindestens ein Strukturelement mit einem aromatischen Valerolaktam (AV), bzw. die oben aufgeführten bevorzugten Ausführungsformen, können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine Verbindung, umfassend mindestens ein Strukturelement mit drei anellierten aromatischen Ringen (AR) und mindestens ein Strukturelement mit einem aromatischen Valerolaktam (AV), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs, enthaltend in mindestens einer Schicht mindestens eine Verbindung, umfassend mindestens ein Strukturelement mit drei anellierten aromatischen Ringen (AR) und mindestens ein Strukturelement mit einem aromatischen Valerolaktam (AV). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoOs oder WOs oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert,

d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen.. Weiterhin bevorzugt sind auch Tandem-OLEDs. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die erfindungsgemäße Verbindung, umfassend mindestens ein Strukturelement mit drei anellierten aromatischen Ringen (AR) und mindestens ein Strukturelement mit einem aromatischen Valerolaktam (AV), bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung.

Geeignete Matrixmaterialien, welche in Kombination mit den Verbindungen, umfassend mindestens ein Strukturelement mit drei anellierten aromatischen Ringen (AR) und mindestens ein Strukturelement mit einem aromatischen Valerolaktam (AV), bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011/137951 oder WO 2013/064206, 4-SpirocarbazolDerivate, z. B. gemäß WO 2014/094963 oder WO 2015/192939 oder Dibenzofuran-Derivate, z. B. gemäß WO 2015/169412, WO 2016/015810, WO 2016/023608 oder den noch nicht offen gelegten Anmeldungen EP 16158460.2 und EP 16159829.7. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1),

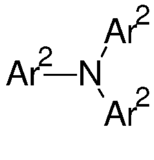

Formel (TA-1)

wobei $Ar^2$ gleich oder verschieden bei jedem Auftreten ein aromatisches oder heteroaromatisches Ringsystem mit 6 bis 40 C-Atomen, das jeweils mit einem oder mehreren Resten $R^2$ substituiert sein kann, darstellt, wobei optional zwei oder mehr benachbarte Substituenten $R^2$ ein mono- oder polycyclisches, aliphatisches, heteroaliphatisches, aromatisches oder heteroaromatisches Ringsystem, vorzugsweise ein mono- oder polycyclisches, aliphatisches Ringsystem bilden können, das mit einem oder mehreren Resten $R^3$ substituiert sein kann, wobei das Symbol $R^2$ die zuvor genannte Bedeutung aufweist. Vorzugsweise stellt $Ar^2$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^2$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Bevorzugt sind die Gruppen $Ar^2$ gleich oder verschieden bei jedem Auftreten ausgewählt aus den oben genannten Gruppen $R^1$-1 bis $R^1$-86, besonders bevorzugt $R^1$-1 bis $R^1$-54.

In einer bevorzugten Ausführungsform der Verbindungen der Formel (TA-1) ist mindestens eine Gruppe $Ar^2$ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe $Ar^2$ ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe $Ar^2$ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe $Ar^2$ ausgewählt aus einer Fluoren- oder Spirobifluorengruppe, insbesondere einer 4-Fluoren- bzw. 4-Spirobifluorengruppe, und eine Gruppe $Ar^2$ ist ausgewählt aus einer Biphenylgruppe, insbesondere einer para-Biphenylgruppe, oder einer Fluorengruppe, insbesondere einer 2-Fluorengruppe, und die dritte Gruppe $Ar^2$ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2),

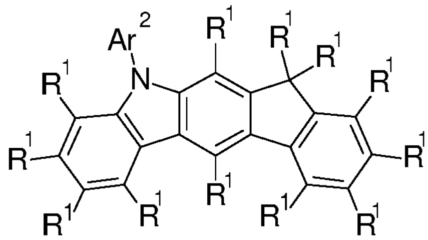

Formel (TA-2)

wobei $Ar^2$ und $R^1$ die oben insbesondere für Formeln (AV-1), (AV-2) und/oder (TA-1) aufgeführten Bedeutungen aufweisen. Dabei sind bevorzugte Ausführungsformen der Gruppe $Ar^2$ die oben aufgeführten Strukturen $R^1$-1 bis $R^1$-86, besonders bevorzugt $R^1$-1 bis $R^1$-54.

Eine bevorzugte Ausführungsform der Verbindungen der Formel (TA-2) sind die Verbindungen der folgenden Formel (TA-2a),

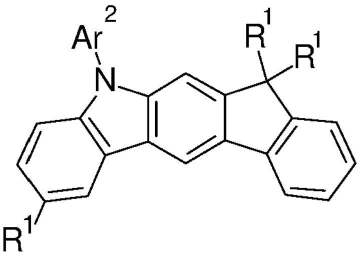

Formel (TA-2a)

wobei $Ar^2$ und $R^1$ die oben aufgeführten Bedeutungen aufweisen. Dabei stehen die beiden Gruppen $R^1$, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Be-sonders bevorzugt stehen die beiden Gruppen $R^1$, die an das Indenokohlenstoffatom gebunden sind, für Methylgruppen. Weiterhin bevorzugt steht der Substituent $R^1$, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3),

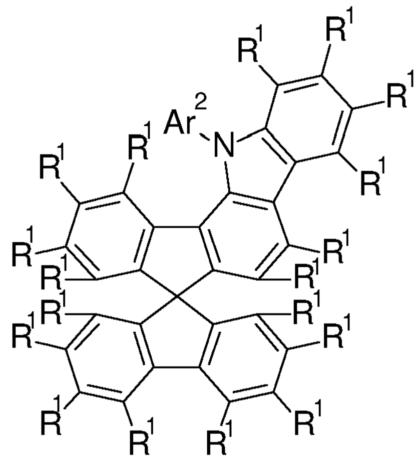

Formel (TA-3)

wobei $Ar^2$ und $R^1$ die oben aufgeführten Bedeutungen aufweisen. Dabei sind bevorzugte Ausführungsformen der Gruppe $Ar^2$ die oben aufgeführten Strukturen $R^1$-1 bis $R^1$-86, besonders bevorzugt $R^1$-1 bis $R^1$-54.

Eine bevorzugte Ausführungsform der Verbindungen der Formel (TA-3) sind die Verbindungen der folgenden Formel (TA-3a),

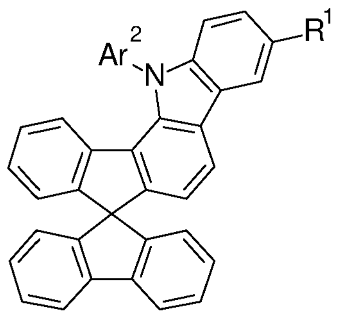

Formel (TA-3a)

wobei $Ar^2$ und $R^1$ die oben aufgeführten Bedeutungen aufweisen. Dabei sind bevorzugte Ausführungsformen der Gruppe $Ar^2$ die oben aufgeführten Strukturen $R^1$-1 bis $R^1$-86, besonders bevorzugt $R^1$-1 bis $R^1$-54.

Bevorzugte Lactame, die als Co-Host-Materialien zusammen mit den erfindungsgemäßen Verbindungen eingesetzt werden, sind ausgewählt aus den Verbindungen der folgenden Formel (LAC-1),

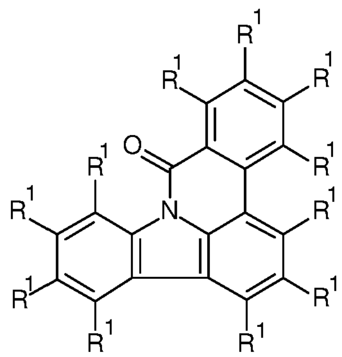

Formel (LAC-1)

wobei $R^1$ die oben aufgeführte Bedeutung aufweist.

Eine bevorzugte Ausführungsform der Verbindungen der Formel (LAC-1) sind die Verbindungen der folgenden Formel (LAC-1a),

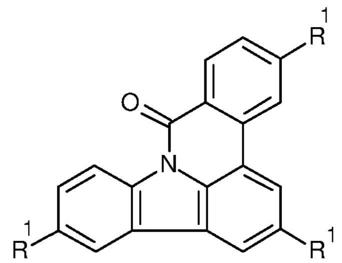

Formel (LAC-1a)

wobei $R^1$ die oben genannte Bedeutung aufweist. Dabei steht $R^1$ bevorzugt gleich oder verschieden bei jedem Auftreten für H oder ein aromatisches oder heteroaromatisches Ringsystem mit 5 bis 40 aromatischen Ringatomen, das mit einem oder mehreren Resten $R^2$ substituiert sein kann, wobei $R^2$ die zuvor genannte Bedeutung aufweisen kann. Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei sind geeignete Strukturen $R^1$ die gleichen Strukturen, wie sie zuvor für R-1 bis R-86 abgebildet sind, besonders bevorzugt $R^1$-1 bis $R^1$-54.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben. Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung, umfassend mindestens ein Strukturelement mit drei anellierten aromatischen Ringen (AR) und mindestens ein Strukturelement mit einem aromatischen Valerolaktam (AV), in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung, umfassend mindestens ein Strukturelement mit drei anellierten aromatischen Ringen (AR) und mindestens ein Strukturelement mit einem aromatischen Valerolaktam (AV), bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung, umfassend mindestens ein Strukturelement mit drei anellierten aromatischen Ringen (AR) und mindestens ein Strukturelement mit einem aromatischen Valerolaktam (AV), bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoOs oder WOs, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine erfindungsgemäße Verbindung, umfassend mindestens ein Strukturelement mit drei anellierten aromatischen Ringen (AR) und mindestens ein Strukturelement mit einem aromatischen Valerolaktam (AV), und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend erfindungsgemäße Verbindungen, umfassend mindestens ein Strukturelement mit drei anellierten aromatischen Ringen (AR) und mindestens ein Strukturelement mit einem aromatischen Valerolaktam (AV), bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronische Vorrichtungen, insbesondere organische Elektrolumineszenzvorrichtungen enthaltend Verbindungen, Oligomere, Polymere oder Dendrimere mit mindestens einem Strukturelement, enthaltend drei anellierte aromatische Ringe (AR) und mit mindestens einem Strukturelement, enthaltend mindestens ein aromatisches Valerolaktam (AV), bzw. die zuvor und nachfolgend ausgeführten bevorzugten Ausführungsformen, insbesondere als elektronenleitende Materialien, weisen eine sehr gute Lebensdauer auf.

2. Elektronische Vorrichtungen, insbesondere organische Elektrolumineszenzvorrichtungen enthaltend Verbindungen, Oligomere, Polymere oder Dendrimere mit mindestens einem Strukturelement, enthaltend drei anellierte aromatische Ringe (AR) und mit mindestens einem Strukturelement, enthaltend mindestens ein aromatisches Valerolaktam (AV), bzw. die zuvor und nachfolgend ausgeführten bevorzugten Ausführungsformen weisen als elektronenleitende Materialien, Elektroneninjektionsmaterialien und/oder Hostmaterialien eine hervorragende Effizienz auf. Insbesondere ist die Effizienz deutlich höher gegenüber analogen Verbindungen, die keine Struktureinheiten gemäß (AV) oder (AR) enthalten. Hierbei bewirken erfindungsgemäßen Verbindungen, Oligomere, Polymere oder Dendrimere mit mindestens einem Strukturelement, enthaltend drei anellierte aromatische Ringe (AR) und mit mindestens einem Strukturelement, enthaltend mindestens ein aromatisches Valerolaktam (AV), bzw. die zuvor und nachfolgend ausgeführten bevorzugten Ausführungsformen eine geringe Betriebsspannung bei Verwendung in elektronischen Vorrichtungen. Hierbei bewirken diese Verbindungen insbesondere einen geringen Roll-off, d.h. einen geringen Abfall der Leistungseffizienz der Vorrichtung bei hohen Leuchtdichten.

3. Die erfindungsgemäßen Verbindungen, Oligomere, Polymere oder Dendrimere mit mindestens einem Strukturelement, enthaltend drei anellierte aromatische Ringe (AR) und mit mindestens einem Strukturelement, enthaltend mindestens ein aromatisches Valerolaktam (AV), bzw. die zuvor und nachfolgend ausgeführten bevorzugten Ausführungsformen zeigen eine sehr hohe Stabilität und führen zu Verbindungen mit einer sehr hohen Lebensdauer.

4. Mit Verbindungen, Oligomeren, Polymeren oder Dendrimeren mit mindestens einem Strukturelement, enthaltend

drei anellierte aromatische Ringe (AR) und mit mindestens einem Strukturelement, enthaltend mindestens ein aromatisches Valerolaktam (AV), bzw. die zuvor und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

5. Die Verwendung von Verbindungen, Oligomeren, Polymeren oder Dendrimeren mit mindestens einem Strukturelement, enthaltend drei anellierte aromatische Ringe (AR) und mit mindestens einem Strukturelement, enthaltend mindestens ein aromatisches Valerolaktam (AV), bzw. die zuvor und nachfolgend ausgeführten bevorzugten Ausführungsformen in Schichten elektronischer Vorrichtungen, insbesondere organischer Elektrolumineszenzvorrichtungen führt zu einer hohen Mobilität der Elektronenleiterstrukturen.

6. Verbindungen, Oligomere, Polymere oder Dendrimere mit mindestens einem Strukturelement, enthaltend drei anellierte aromatische Ringe (AR) und mit mindestens einem Strukturelement, enthaltend mindestens ein aromatisches Valerolaktam (AV), bzw. die zuvor und nachfolgend ausgeführten bevorzugten Ausführungsformen zeichnen sich durch eine ausgezeichnete thermische Stabilität aus, wobei Verbindungen mit einer Molmasse von weniger als ca. 1200 g/mol gut sublimierbar sind.

7. Verbindungen, Oligomere, Polymere oder Dendrimere mit mindestens einem Strukturelement, enthaltend drei anellierte aromatische Ringe (AR) und mit mindestens einem Strukturelement, enthaltend mindestens ein aromatisches Valerolaktam (AV), bzw. die zuvor und nachfolgend ausgeführten bevorzugten Ausführungsformen weisen eine ausgezeichnete Glasfilmbildung auf.

8. Verbindungen, Oligomere, Polymere oder Dendrimere mit mindestens einem Strukturelement, enthaltend drei anellierte aromatische Ringe (AR) und mit mindestens einem Strukturelement, enthaltend mindestens ein aromatisches Valerolaktam (AV), bzw. die zuvor und nachfolgend ausgeführten bevorzugten Ausführungsformen bilden aus Lösungen sehr gute Filme.

9. Die Verbindungen, Oligomere, Polymere oder Dendrimere, umfassend mindestens ein Strukturelement mit drei anellierten aromatischen Ringen (AR) und mindestens ein Strukturelement mit einem aromatischen Valerolaktam (AV), bzw. die zuvor und nachfolgend ausgeführten bevorzugten Ausführungsformen weisen ein überraschend hohes Triplett-Niveau $T_1$ auf, wobei dies insbesondere Verbindungen gilt, die als elektronenleitende Materialien eingesetzt werden.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als Hostmaterial, Lochblockiermaterial, Elektroneninjektionsmaterial und/oder Elektronentransportmaterial, vorzugsweise als Hostmaterial und/oder Elektronentransportmaterial. Besonders bevorzugt kann eine erfindungsgemäße Verbindung in Kombination mit einem weiteren Elektroneninjektionsmaterial und/oder Elektronentransportmaterial in einer elektronenleitenden Schicht und/oder Elektroneninjektionsschicht eingesetzt werden, wie dies zuvor in Zusammenhang mit einer erfindungsgemäßen Zusammensetzung dargelegt wurde.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen. Besonders bevorzugt ist eine elektronische Vorrichtung ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochblockier- oder Elektronentransportschicht einzusetzen. Dies gilt insbesondere für erfindungsgemäße Verbindungen, die keine Carbazolstruktur aufweisen. Diese können bevorzugt auch mit einer oder mehreren weiteren elektronentransportierenden Gruppen substituiert sein, beispielsweise Benzimidazolgruppen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen bzw. gemäß den bevorzugten Ausführungsformen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

## Beispiele

### Syntheseschema:

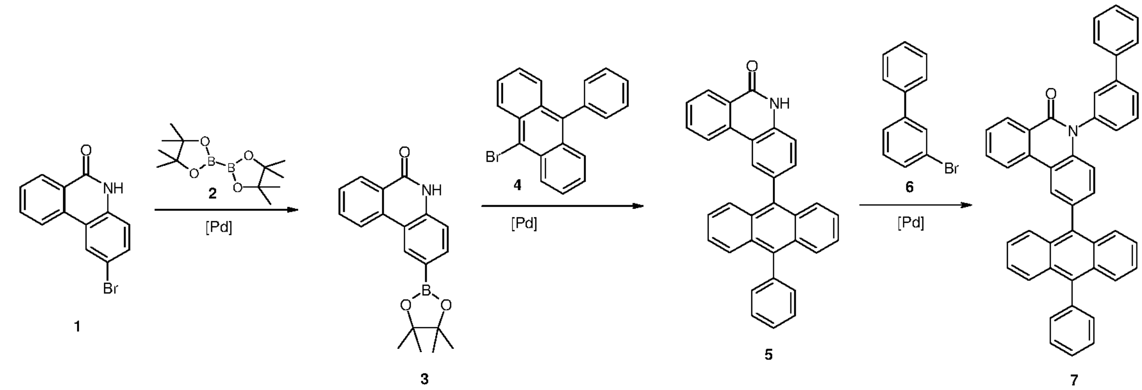


### 1. Herstellung von 2-(4,4,5,5-Tetramethyl-[1,3,2]dioxaborolan-2-yl)-5H-phenanthridin-6-on (3)

In einem Vierhalskolben werden 20.0g (73.0mmol, 1.00eq) 2-Bromo-5H-phenanthridin-6-on [27353-48-6] 1 zusammen mit 22.2g (87.6mmol, 1.20eq) Bis(pinacolato)diboran [73183-34-3] 2 und 21.5g (219mmol, 3.00eq) Kaliumacetat in 800ml Dioxan gelöst und mit Argon inertisiert. Anschließend werden 1.79g (2.19mmol, 0.03eq) 1,1-Bis(diphenylphosphino)ferrocen-dichloropalladium(II)-Komplex [95464-05-4] zugegeben und das Reaktionsgemisch bei 115°C Badtem peratur über Nacht gerührt. Nach beendeter Reaktion wird der Ansatz auf Raumtemperatur abgekühlt und das Lösungsmittel am Rotationsverdampfer entfernt. Der Rückstand wird in 250ml Dichlormethan aufgenommen und mit 250ml Wasser ausgeschüttelt. Die wässrige Phase wird dreimal mit 250ml Dichlormethan extrahiert, die vereinten organischen Phasen über Natriumsulfat getrocknet und das Lösungsmittel am Rotationsverdampfer entfernt. Der erhaltene Feststoff wird mit Ethanol bei 60°C ausgewaschen. Nach Trocknung werden 20.6g (64.0mmol, 88%) des gewünschten Produkts **3** erhalten.

Analog können die folgenden Verbindungen hergestellt werden:

| Verbin-dung | Edukt | Produkt | Aus-beute [%] |
|---|---|---|---|
| **3b** | 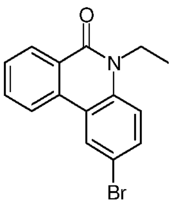 [855353-07-0] | 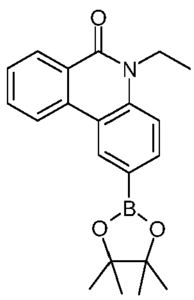 | 82 |
| **3c** | 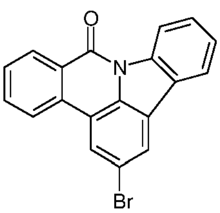 [1346571-39-8] | 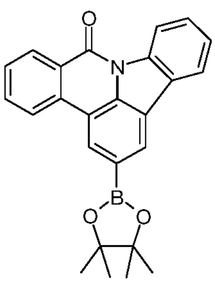 | 97 |

(fortgesetzt)

| Verbin-dung | Edukt | Produkt | Aus-beute [%] |
|---|---|---|---|
| **3d** | 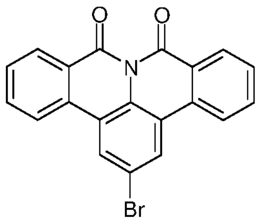 [1598462-32-8] | 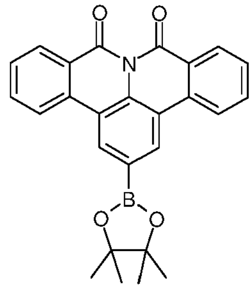 | 66 |
| **3e** | 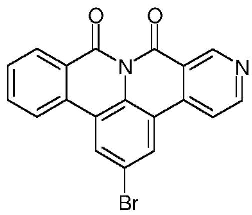 [1598462-34-0] | 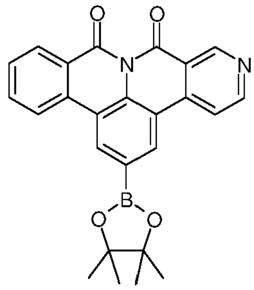 | 45 |
| **3f** | 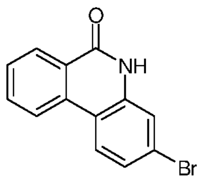 [500350-01-6] | 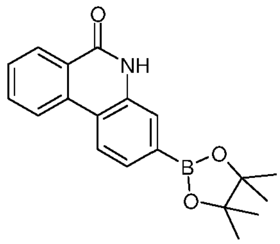 | 78 |
| **3g** | 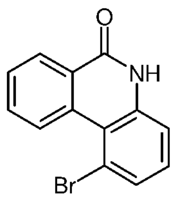 [17613-45-5] | 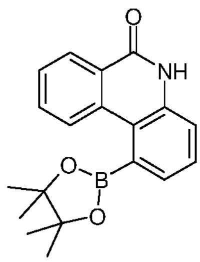 | 47 |
| **3h** | 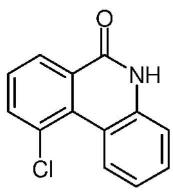 [27353-63-5] | 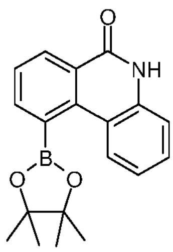 | 33 |
| **3i** | 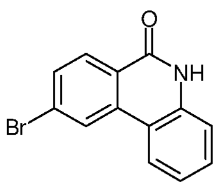 [145548-23-8] | 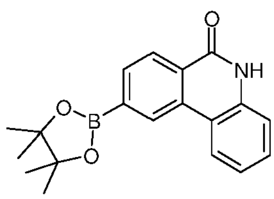 | 93 |

(fortgesetzt)

| Verbin-dung | Edukt | Produkt | Aus-beute [%] |
|---|---|---|---|
| 3j | 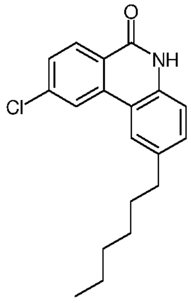 [1346693-50-2] | 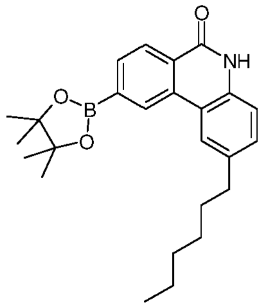 | 68 |
| 3k | 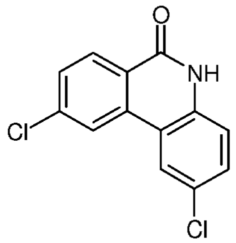 [20851-89-2] | 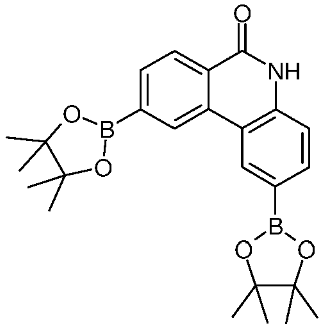 | 27 |
| 3l | 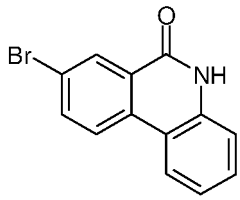 [26689-66-7] | 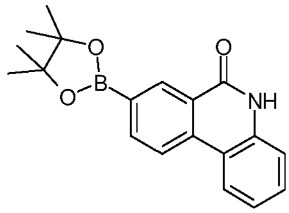 | 95 |
| 3m | 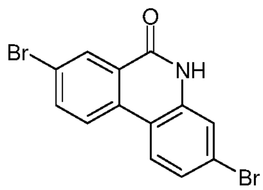 [23818-37-3] | 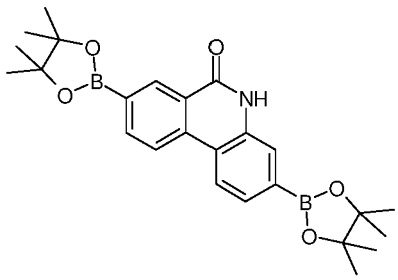 | 74 |

**2. Herstellung von 2-(10-Phenyl-anthracen-9-yl)-5H-phenanthridin-6-on (5)**

In einem Vierhalskolben werden 14.4g (44.9mmol, 1.00eq) der Zwischenstufe **3,** 15.7g (47.1 mmol, 1.05eq) 9-Brom-10-phenylanthracen [23674-20-6] und 4.80g (44.9mmol, 1.00eq) Natriumcarbonat in 185ml Toluol, 375ml 1,4-Dioxan und 375ml Wasser vorgelegt und das Gemisch mit Argon entgast. Anschließend werden 1.03g (0.891 mmol, 0.02eq) Tetrakis(triphenylphosphin)-palladium(0) [14221-01-3] zugegeben und der Ansatz bei 120°C über Nacht gerührt. Nach beendeter Reaktion wird der ausgefallene Feststoff mit Wasser und Ethanol gewaschen und im Vakuumtrockenschrank getrocknet. Es werden 11.9g (26.7mmol, 59%) der gewünschten Zielverbindung **5** erhalten.

Analog können die folgenden Verbindungen hergestellt werden:

| Verb. | Edukt | Edukt | Produkt | Ausb. [%] |
|---|---|---|---|---|
| **5b** | 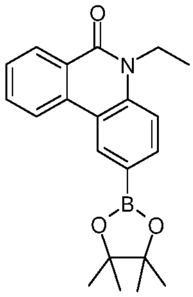 | 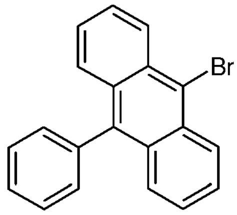 | 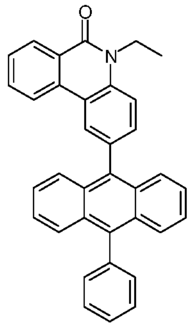 | 43% |
| **5c** | 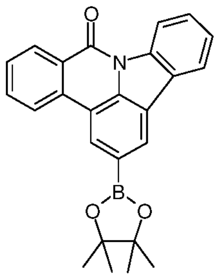 | 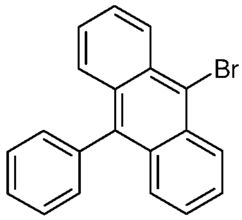 | 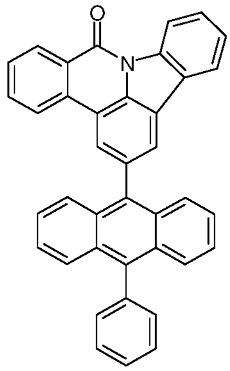 | 47% |
| **5d** | 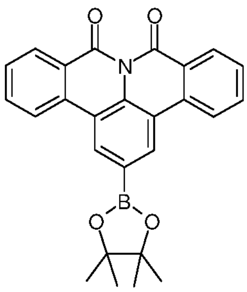 | 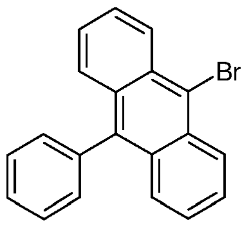 | 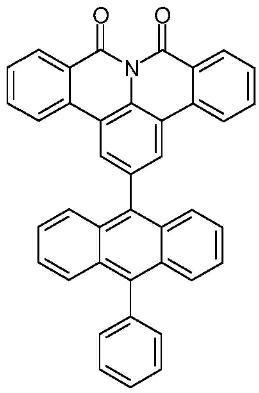 | 53% |
| **5e** | 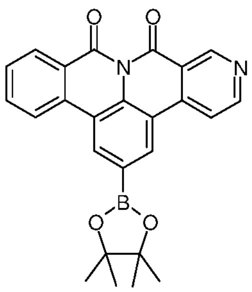 | 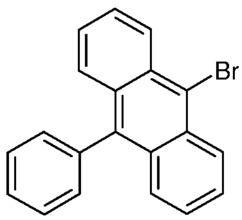 | 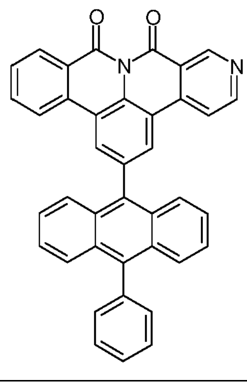 | 37% |

(fortgesetzt)

| Verb. | Edukt | Edukt | Produkt | Ausb. [%] |
|---|---|---|---|---|
| **5f** | 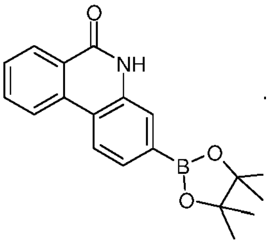 | 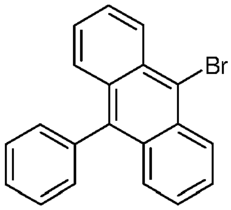 | 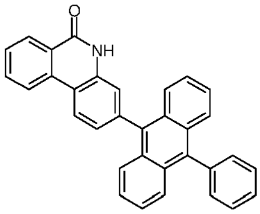 | 42% |
| **5g** | 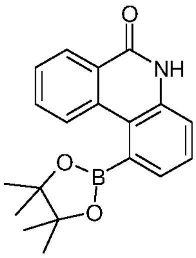 | 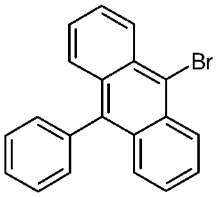 | 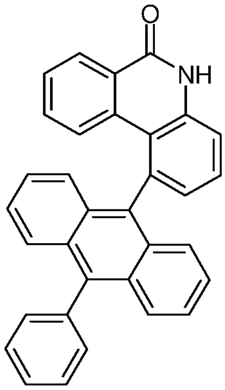 | 51% |
| **5h** | 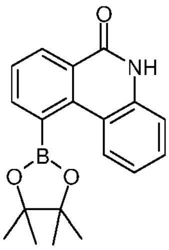 | 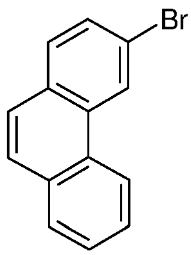 | 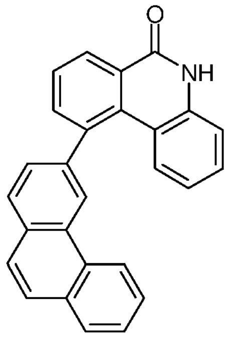 | 53% |
| **5i** | 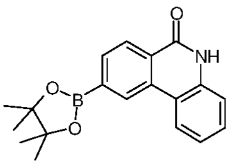 | 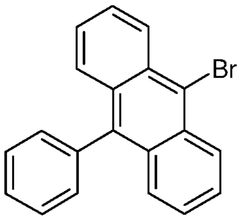 | 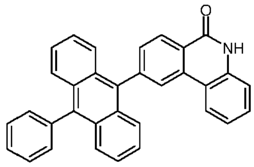 | 47% |
| **5j** | 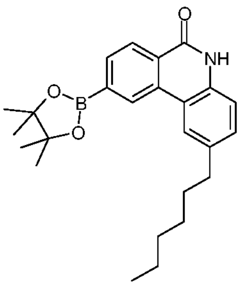 | 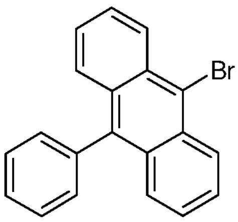 | 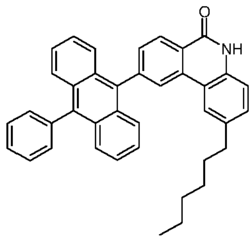 | 59% |

(fortgesetzt)

| Verb. | Edukt | Edukt | Produkt | Ausb. [%] |
|---|---|---|---|---|
| 5k | 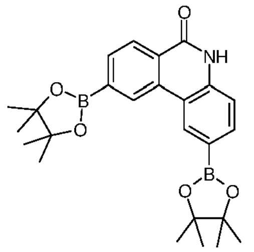 | 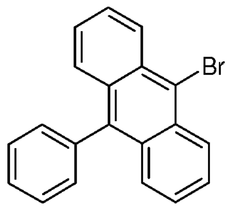 | 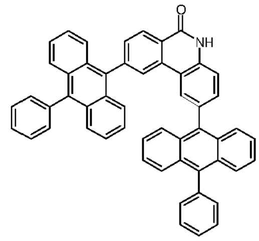 | 57% |
| 5l | 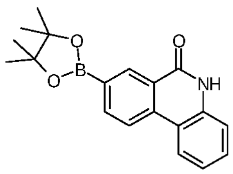 | 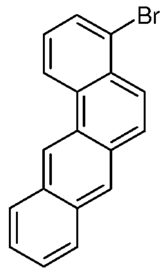 | 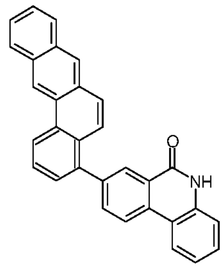 | 53% |
| 5m | 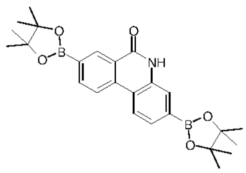 | 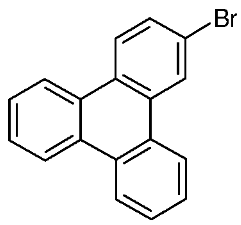 | 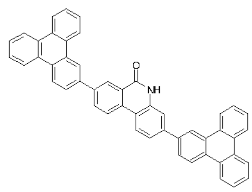 | 37% |

### 3. Herstellung von 5-Biphenyl-3-yl-2-(10-phenyl-anthracen-9-yl)-5H-phenanthridin-6-on (7)

11.1g (24.8mmol, 1.00eq) 2-(10-Phenyl-anthracen-9-yl)-5H-phenanthridin-6-on **5,** 21.3ml (128mmol, 5.2eq) 3-Bromobiphenyl [2113-57-7]und 7.20g Kaliumcarbonat (52.1mmol, 2.10eq) Kaliumcarbonat werden in 220ml getrocknetem DMF vorgelegt und mit Argon inertisiert. Anschließend werden 0.62g (2.7 mmol, 0.11eq) 1,3-Di(2-pyridyl)-1,3-propandion und 0.52g (2.7mmol, 0.11eq) Kupfer(I)-iodid zugegeben und das Gemisch für drei Tage bei 140°C erhitzt. Nach beendeter Reaktio n wird der Ansatz vorsichtig am Rotationsverdampfer eingeengt, der ausgefallene Feststoff abgesaugt und mit Wasser und Ethanol gewaschen. Das Rohprodukt wird zweimal mittels Heißextraktor (Toluol/Heptan 1:1) aufgereinigt und der erhaltene Feststoff aus einem Toluol umkristallisiert. Nach Sublimation werden 5.3g (8.8mmol, 36%) der gewünschten Zielverbindung 7 erhalten.

Analog können die folgenden Verbindungen hergestellt werden:

| Verb. | Edukt | Edukt | Produkt | Ausb. [%] |
|---|---|---|---|---|
| **7b** | 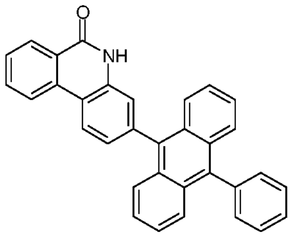 | 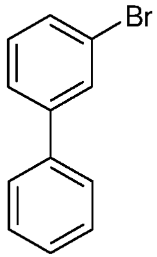 | 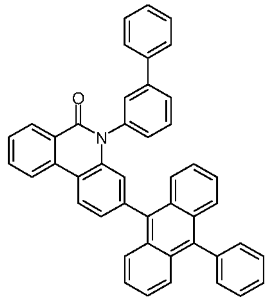 | 42% |
| **7c** | 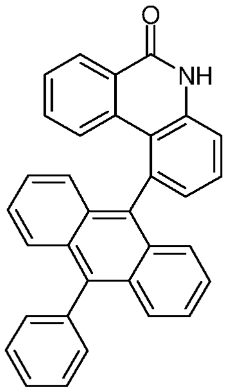 | 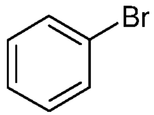 | 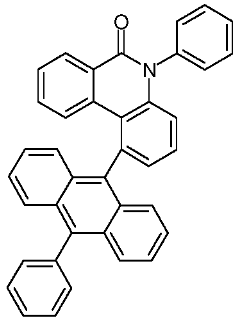 | 47% |
| **7d** | 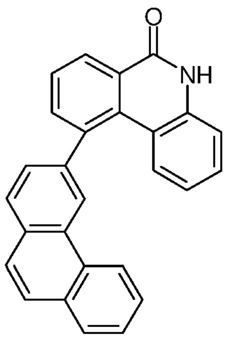 | 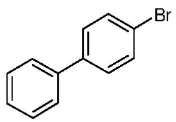 | 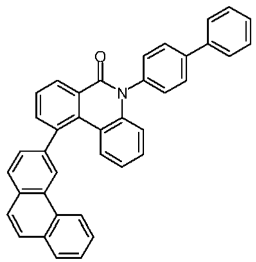 | 53% |

(fortgesetzt)

| Verb. | Edukt | Edukt | Produkt | Ausb. [%] |
|---|---|---|---|---|
| **7e** | 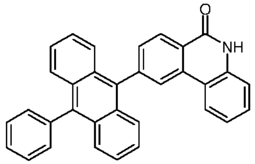 | 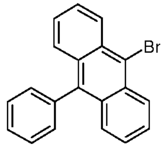 | 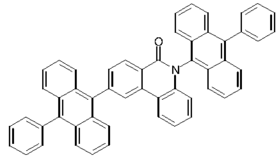 | 51% |
| **7f** | 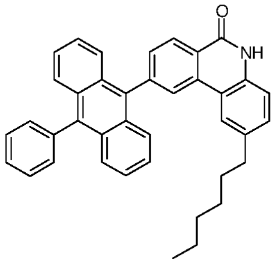 | 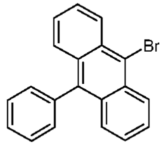 | 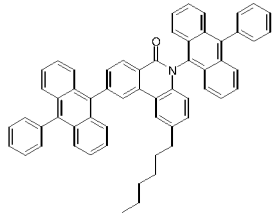 | 47% |
| **7g** | 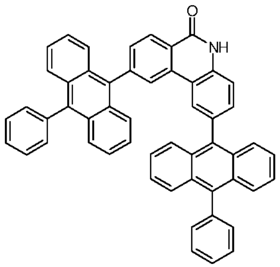 | 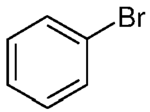 | 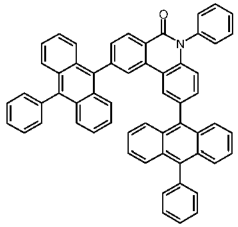 | 49% |
| **7h** | 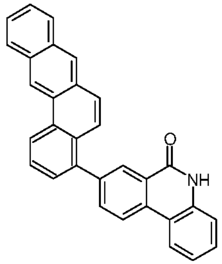 | 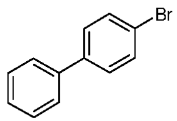 | 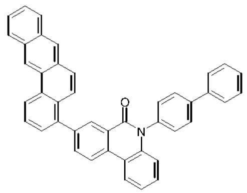 | 59% |

(fortgesetzt)

| Verb. | Edukt | Edukt | Produkt | Ausb. [%] |
|---|---|---|---|---|
| 7i | 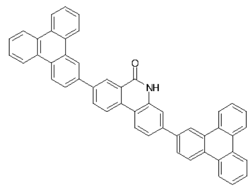 | 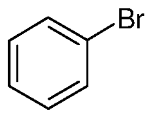 | 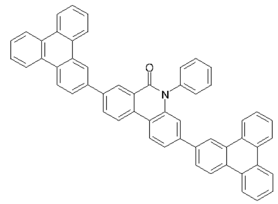 | 42% |
| 7j | 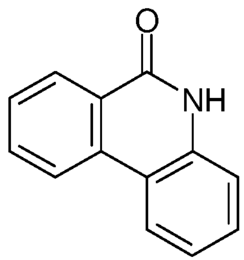 1015-89-0 | 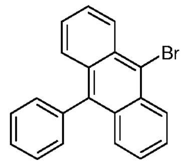 | 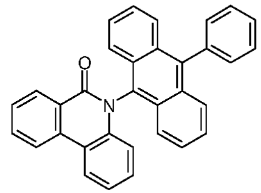 | 59% |
| 7k | 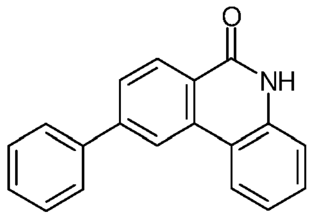 1071752-97-0 | 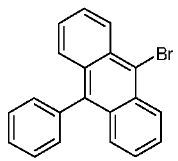 | 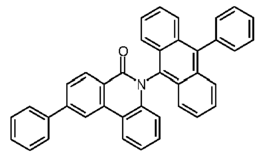 | 52% |

(fortgesetzt)

| Verb. | Edukt | Edukt | Produkt | Ausb. [%] |
|---|---|---|---|---|
| **7l** | 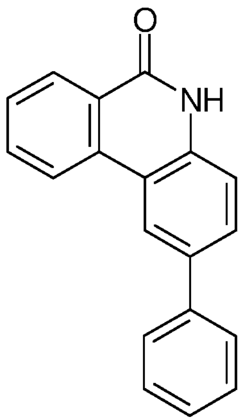 157848-49-2 | 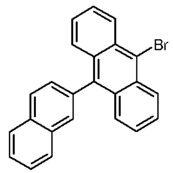 | 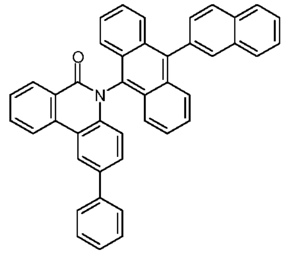 | 41% |
| **7m** | 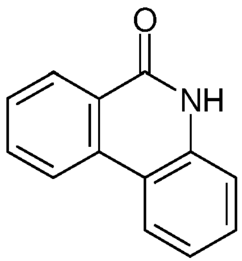 1015-89-0 | 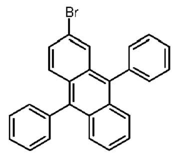 201731-79-5 | 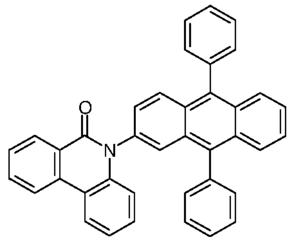 | 40% |

**Herstellung der Vergleichsverbindung (COMP)**

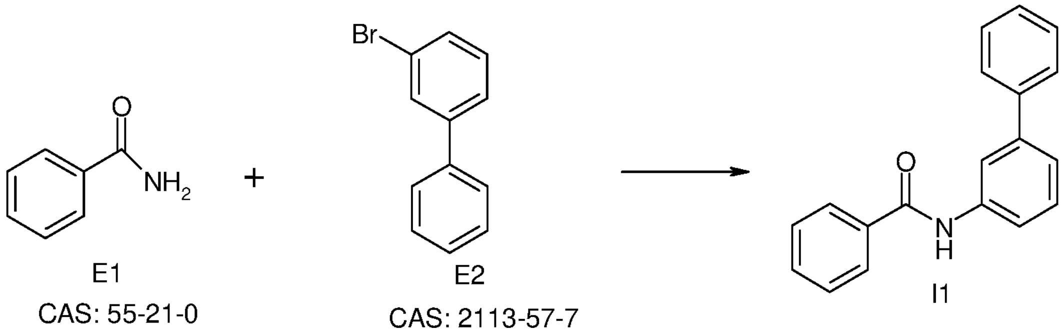


In 200 ml wasserfreiem Toluol werden 20 g (86 mmol) E2, 11.4 g (94 mmol) E1, 23.6 g (171 mmol) Kaliumcarbonat, 1.72 g (20 mmol) N,N'-dimethylethylendiamin, 1.62 g (9 mmol) CuI vorgelegt und 5 Tage refluxiert. Nach vollständiger Reaktion wird die Reaktionsmischung auf Raumtemperatur abgekühlt und über ein kurzes Kieselgelbett filtriert und mit Dichlormethan nachgewaschen. Die Lösungsmittel werden unter vermindertem Druck entfernt und der Rückstand mit Ethanol gewaschen. Ausbeute: 22.4 g (82 mmol; 95 %)

Finaler Schritt zur Vergleichsverbindung **(COMP):**

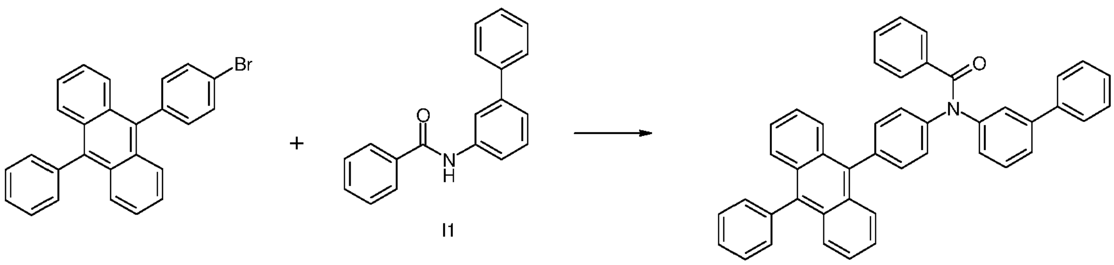


GAS: 625854-02-6

Synthese erfolgte analog der Verbindung **7.** Ausbeute 33 %

**Herstellung der OLEDs**

In den folgenden Beispielen V1 sowie E2 bis E6 (siehe Tabellen 1 und 2) werden die Daten verschiedener OLEDs vorgestellt.

**Vorbehandlung für die Beispiele V1 und E2-E6:** Glasplättchen, die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylendioxythiophen)poly(styrolsulfonat))., bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H:SEB (95%:5%) bedeutet hierbei, dass das Material H in einem Volumenanteil von

95% und SEB in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik, bestimmt. Die Elektrolumineszenzspektren werden bei einer Leuchtdichte von 1000 $cd/m^2$ bestimmt und daraus die CIE 1931 x und y Farbkoordinaten berechnet. Die Angabe U1000 in Tabelle 2 bezeichnet die Spannung, die für eine Leuchtdichte von 1000 $cd/m^2$ benötigt wird. SE1000 und LE1000 bezeichnen die Strom- bzw. Leistungseffizienz, die bei 1000 $cd/m^2$ erreicht werden. EQE1000 schließlich bezeichnet die externe Quanteneffizienz bei einer Betriebsleuchtdichte von 1000 $cd/m^2$.

Die Daten der verschiedenen OLEDs sind in Tabelle 2 zusammengefasst. Das Beispiel V1 dient als Vergleichsbeispiel, die Beispiele E2 bis E4 und E6 zeigen Daten von erfindungsgemäßen OLEDs.

Im Folgenden werden einige der Beispiele näher erläutert, um die Vorteile der erfindungsgemäßen OLEDs zu verdeutlichen.

**Verwendung von erfindungsgemäßen Materialien als Elektronentransportmaterial in OLEDs**

Die erfindungsgemäßen Materialien ergeben bei Einsatz als Elektronentransportmaterial (ETL) in OLEDs eine wesentliche Verbesserung der Leistungseffizienz gegenüber dem Stand der Technik. Durch Einsatz der erfindungsgemäßen Verbindung 7 in Beispiel E2 lässt sich eine Erhöhung der Leistungseffizienz um 50% gegenüber der Vergleichsverbindung **(COMP)** im Beispiel V1 beobachten.

Tabelle 1: Aufbau der OLEDs

| Bsp | HTL Dicke | IL Dicke | EBL Dicke | EML Dicke | ETL Dicke |
|---|---|---|---|---|---|
| V1 | SpA 140nm | HATCN 5nm | SpMA 20nm | H:SEB (95%:5%) 20nm | **COMP**:LiQ (50%:50%) 30nm |
| E2 | SpA 140nm | HATCN 5nm | SpMA 20nm | H:SEB (95%:5%) 20nm | **7**:LiQ (50%:50%) 30nm |
| E3 | SpA 140nm | HATCN 5nm | SpMA 20nm | H:SEB (95%:5%) 20nm | **7g**:LiQ (50%:50%) 30nm |
| E4 | SpA 140nm | HATCN 5nm | SpMA 20nm | H:SEB (95%:5%) 20nm | **7j**:LiQ (50%:50%) 30nm |
| E5(*) | SpA 140nm | HATCN 5nm | SpMA 20nm | H:SEB (95%:5%) 20nm | **7d**:LiQ (50%:50%) 30nm |
| E6 | SpA 140nm | HATCN 5nm | SpMA 20nm | H:SEB (95%:5%) 20nm | **7l**:LiQ (50%:50%) 30nm |
| (*) Referenzbeispiel | | | | | |

Tabelle 2: Daten der OLEDs

| Bsp. | U1000 (V) | SE1000 (cd/A) | LE1000 (Im/W) | EQE 1000 ([%]) | CIE x/y bei 1000 $cd/m^2$ |
|---|---|---|---|---|---|
| V1 | 5.5 | 5.2 | 3.0 | 4.5 | 0.14/0.15 |
| E2 | 5.0 | 7.5 | 4.6 | 6.5 | 0.13 / 0.14 |
| E3 | 5.1 | 7.2 | 4.5 | 6.0 | 0.14/0.15 |

(fortgesetzt)

| Bsp. | U1000 (V) | SE1000 (cd/A) | LE1000 (lm/W) | EQE 1000 ([%]) | CIE x/y bei 1000 cd/m$^2$ |
|---|---|---|---|---|---|
| E4 | 5.2 | 6.5 | 3.9 | 5.6 | 0.14/0.15 |
| E5(*) | 5.2 | 6.9 | 4.2 | 5.9 | 0.13 / 0.14 |
| E6 | 5.3 | 6.6 | 3.9 | 5.7 | 0.14/0.15 |
| (*) Referenzbeispiel | | | | | |

Tabelle 3: Strukturformeln der Materialien für die OLEDs

| | |
|---|---|
| 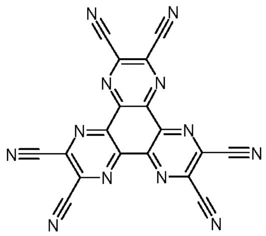 | 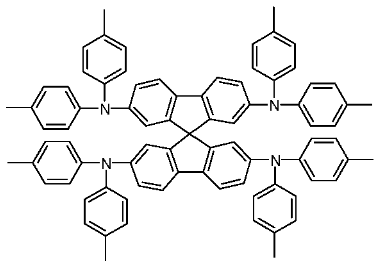 |
| HATCN | SpA |
| 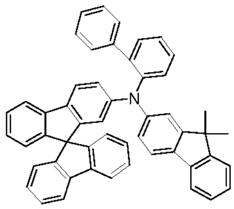 | 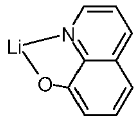 |
| SpMA | LiQ |
| 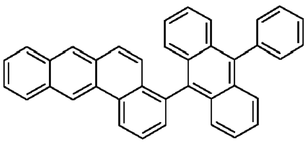 | 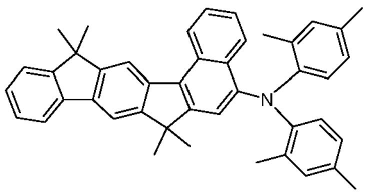 |
| H | SEB |
| 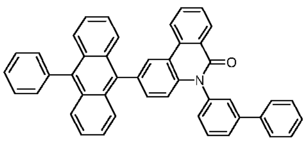 | 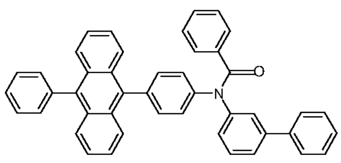 |
| **7** | **COMP** |
| 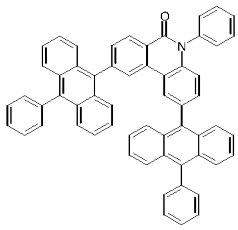 | 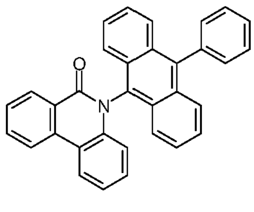 |
| **7g** | **7j** |

(fortgesetzt)

| | |
|---|---|
| 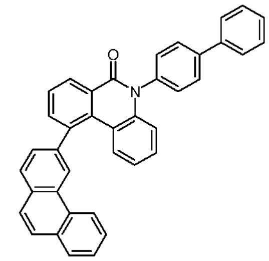 | 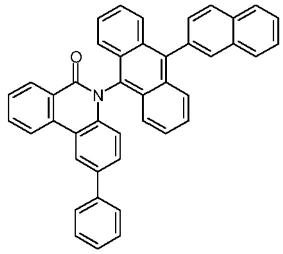 |
| **7d (Referenzverbindung)** | **7l** |

## Patentansprüche

**1.** Verbindung gemäß der Formel (II), (IV), oder (VI):

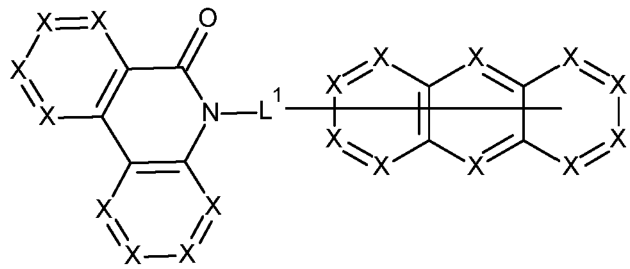

Formel (II)

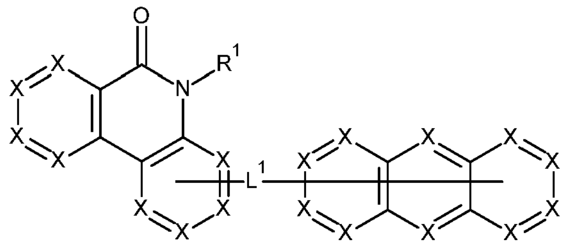

Formel (IV)

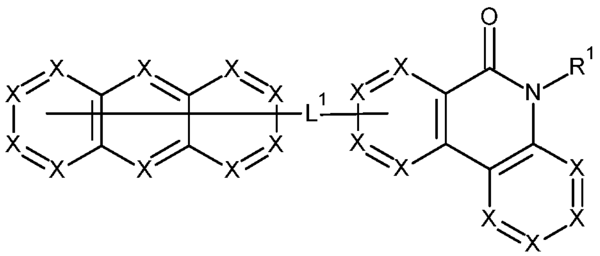

Formel (VI)

wobei für die Symbol und Indizes gilt:

X ist bei jedem Auftreten gleich oder verschieden $CR^1$ oder C für die Anbindungsstelle des Strukturelements mit drei anellierten Ringen (AR);

$R^1$ ist bei jedem Auftreten gleich oder verschieden H, D, CN, eine geradkettige Alkylgruppe mit 1 bis 40 C-Atomen oder eine verzweigte oder cyclische Alkylgruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C≡C-, C=O, C=S, C=Se, $C=NR^2$, -C(=O)O-, $-C(=O)NR^2-$, $NR^2$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann;

$R^2$ ist bei jedem Auftreten gleich oder verschieden H, D, F, CN, eine geradkettige Alkylgruppe mit 1 bis 40 C-Atomen oder eine verzweigte oder cyclische Alkylgruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C≡C-, C=O, C=S, C=Se, $C=NR^3$, -C(=O)O-, $-C(=O)NR^3-$, $NR^3$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können;

$L^1$ ist eine Bindung oder eine Arylgruppe mit 6 bis 40 C-Atomen oder eine Heteroarylgruppe mit 3 bis 40 C-Atomen, welche jeweils durch einen oder mehrere Reste $R^1$ substituiert sein kann.

2. Verbindung gemäß Anspruch 1, **dadurch gekennzeichnet, dass** die Verbindung eine Struktur der Formel (II-1), (II-2), (II-3), (II-4) oder (II-5) aufweist:

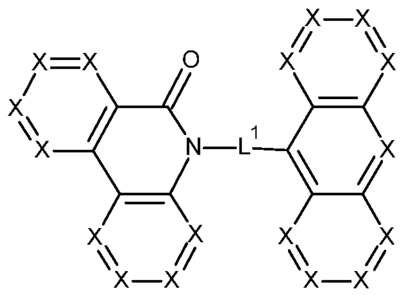

Formel (II-1)

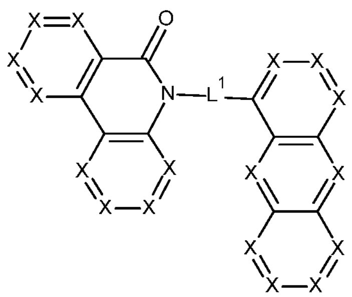

Formel (II-2)

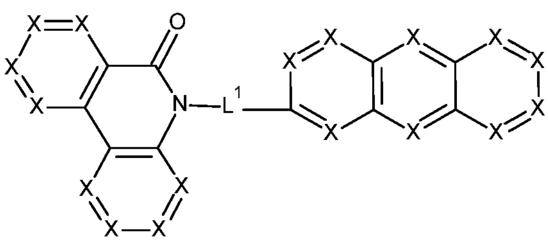

Formel (II-3)

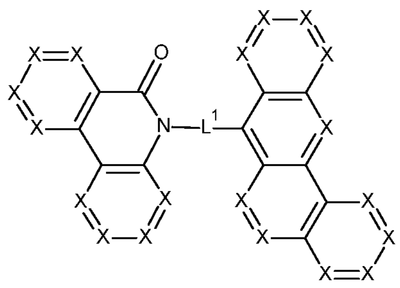

Formel (II-4)

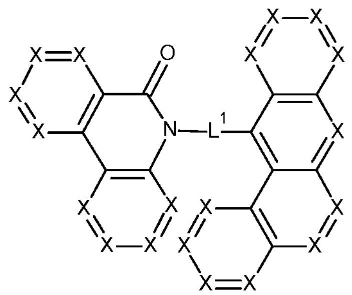

Formel (II-5)

wobei die Symbole X, $L^1$ und $R^1$ die in Anspruch 1 genannte Bedeutung aufweisen.

3. Verbindung gemäß Anspruch 1, **dadurch gekennzeichnet, dass** die Verbindung eine Struktur der Formel (IV-1), (IV-2) oder (IV-3) aufweist:

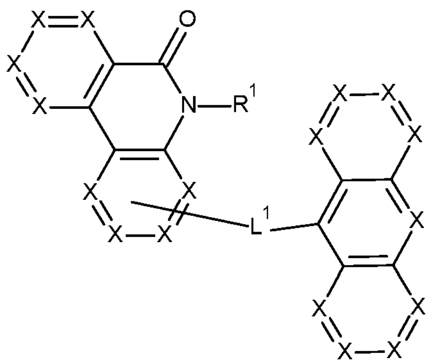

Formel (IV-1)

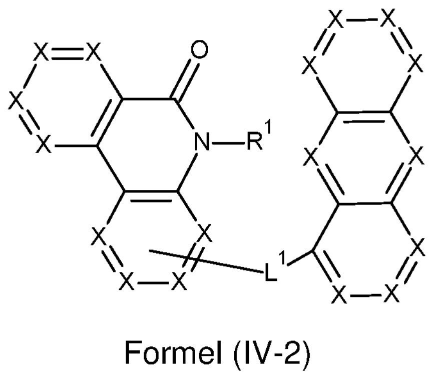

Formel (IV-2)

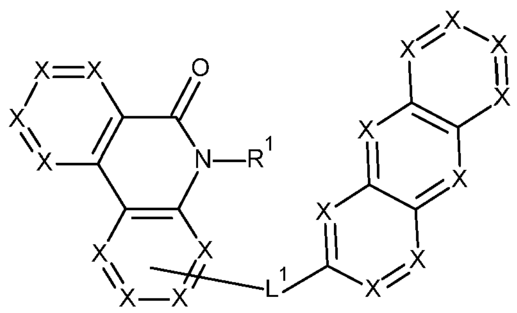

Formel (IV-3)

wobei die Symbole X, $L^1$ und $R^1$ die in Anspruch 1 genannte Bedeutung aufweisen.

4. Verbindung gemäß Anspruch 1, **dadurch gekennzeichnet, dass** die Verbindung eine Struktur der Formel (VI-1), (VI-2) oder (VI-3) aufweist:

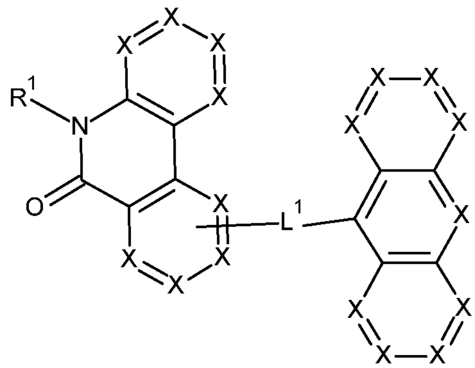

Formel (VI-1)

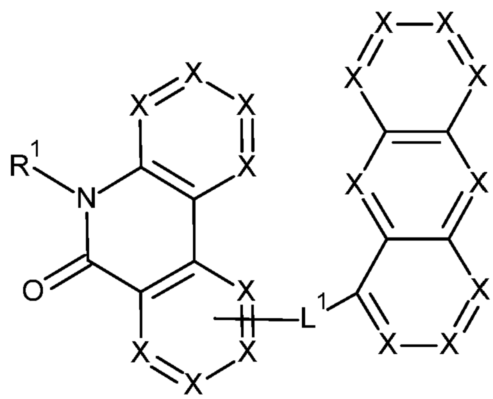

Formel (VI-2)

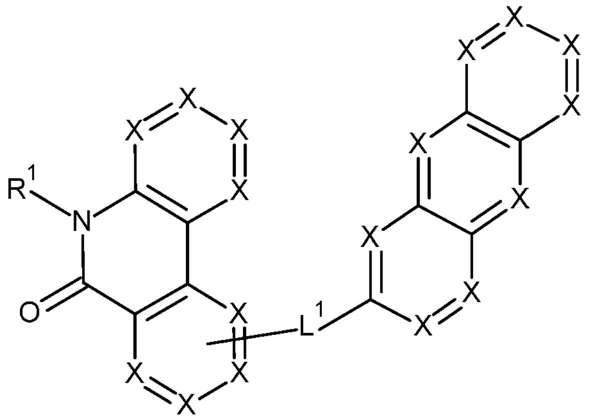

Formel (VI-3)

wobei die Symbole X, $L^1$ und $R^1$ die in Anspruch 1 genannte Bedeutung aufweisen.

**5.** Verbindungen gemäß Anspruch 1, **dadurch gekennzeichnet, dass** die Verbindung eine Struktur der Formel (IV-6), (IV-7), (IV-8) oder (IV-9) aufweist:

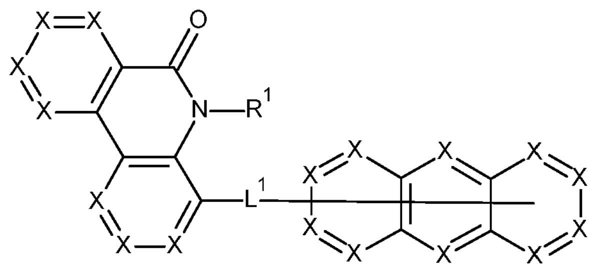

Formel (IV-6)

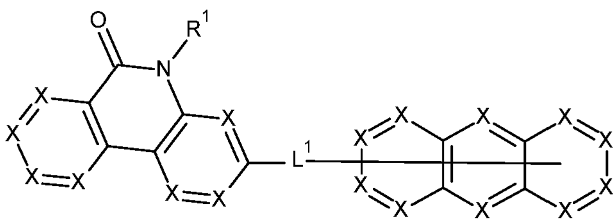

Formel (IV-7)

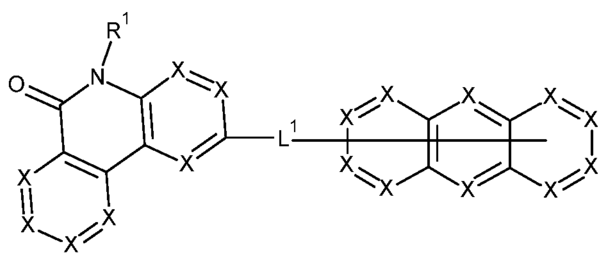

Formel (IV-8)

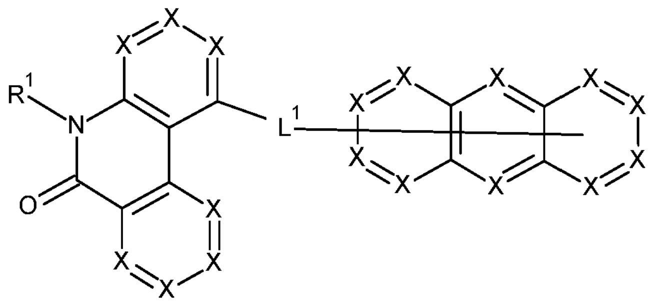

Formel (IV-9)

wobei die Symbole X, $L^1$ und $R^1$ die in Anspruch 1 genannte Bedeutung aufweisen.

6. Verbindungen gemäß Anspruch 1, **dadurch gekennzeichnet, dass** die Verbindung eine Struktur der Formel (VI-6), (VI-7), (VI-8) oder (VI-9) aufweist:

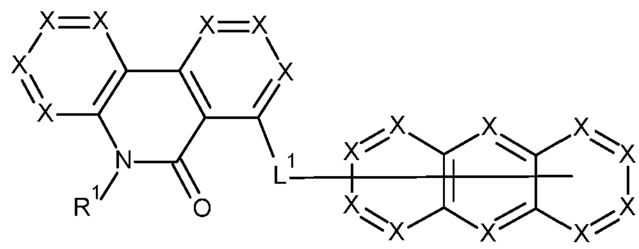

Formel (VI-6)

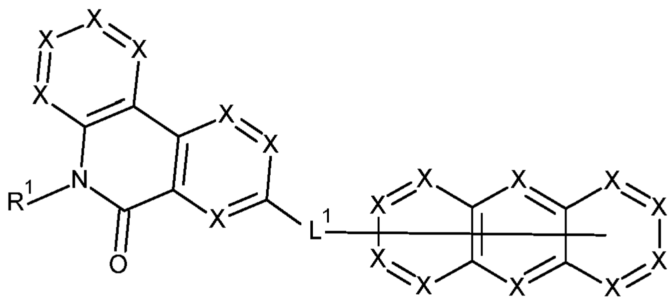

Formel (VI-7)

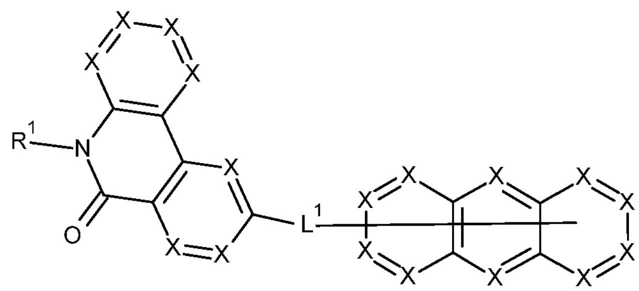

Formel (VI-8)

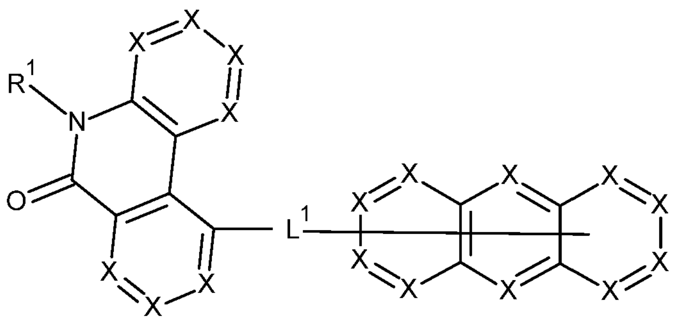

Formel (VI-9)

wobei die Symbole X, $L^1$ und $R^1$ die in Anspruch 1 genannte Bedeutung aufweisen.

7. Verbindung gemäß Anspruch 1, **dadurch gekennzeichnet, dass** die Verbindung eine Struktur der Formel (IIa), (IVa), oder (VIa) aufweist:

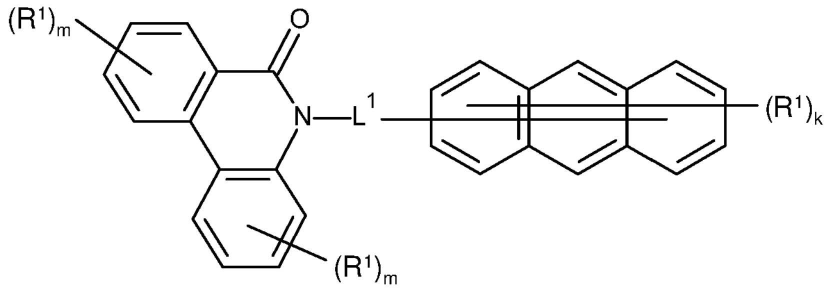

Formel (IIa)

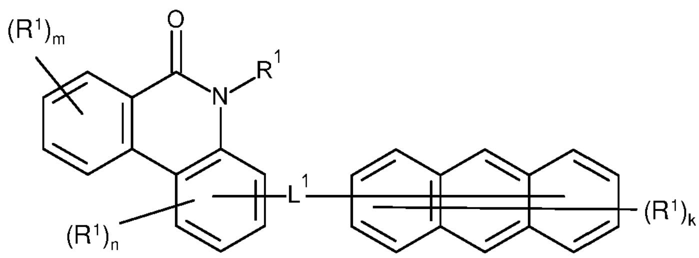

Formel (IVa)

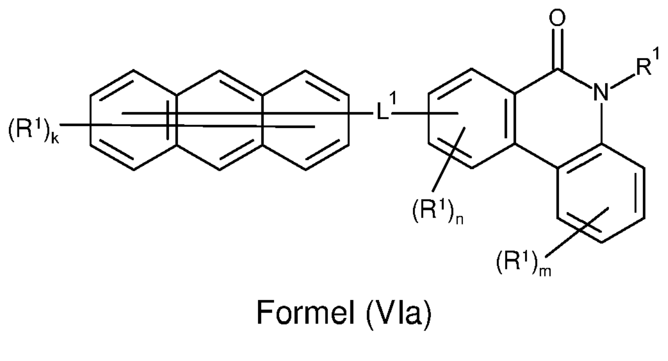

Formel (VIa)

worin die Symbole $R^1$ und $L^1$ die in Anspruch 1 dargelegte Bedeutung haben und der Index k 0, 1, 2, 3, 4, 5, 6, 7, 8 oder 9, vorzugsweise 0, 1, 2 oder 3; der Index m 0, 1, 2, 3 oder 4, vorzugsweise 0, 1 oder 2; und der Index n 0,

1, 2 oder 3, vorzugsweise 0 oder 1 ist.

**8.** Verbindung gemäß Anspruch 1 oder 7, **dadurch gekennzeichnet, dass** die Verbindung eine Struktur der Formel (IIa-1), (IIa-2) oder (IIa-3) aufweist:

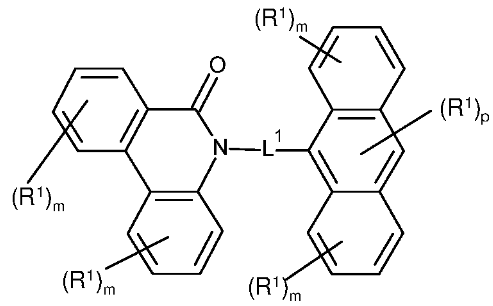

Formel (IIa-1)

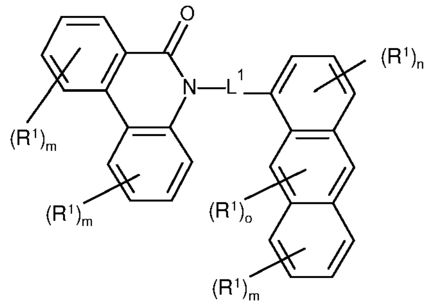

Formel (IIa-2)

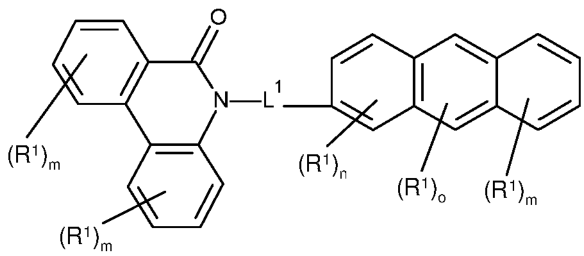

Formel (IIa-3)

wobei die Symbole $L^1$ und $R^1$ sowie die Indices m und n die in Anspruch 7 genannte Bedeutung aufweisen, der Index o 0, 1 oder 2, vorzugsweise 0 oder 1, und der Index p 0 oder 1, vorzugsweise 1 ist.

**9.** Zusammensetzung enthaltend wenigstens eine Verbindung nach einem oder mehreren der Ansprüche 1 bis 8 und wenigstens eine weitere Verbindung ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

**10.** Formulierung enthaltend mindestens eine Verbindung nach einem oder mehreren der Ansprüche 1 bis 8 und/oder wenigstens eine Zusammensetzung nach Anspruch 9 und mindestens ein Lösungsmittel.

**11.** Verfahren zur Herstellung einer Verbindung nach einem oder mehreren der Ansprüche 1 bis 8, **dadurch gekennzeichnet, dass** in einer Kupplungsreaktion eine Verbindung mit drei anellierten aromatischen Ringen mit einer Verbindung, umfassend mindestens ein Strukturelement mit einem aromatischen Valerolaktam, umgesetzt wird.

12. Verwendung einer Verbindung nach einem oder mehreren der Ansprüche 1 bis 8 oder einer Zusammensetzung nach Anspruch 9 in einer elektronischen Vorrichtung als Elektronentransportmaterial, Elektroneninjektionsmaterial und/oder Lochblockiermaterial.

13. Elektronische Vorrichtung, enthaltend mindestens eine Verbindung nach einem oder mehreren der Ansprüche 1 bis 8 oder eine Zusammensetzung gemäß Anspruch 9, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden.

## Claims

1. Compound of the formula (II), (IV) or (VI):

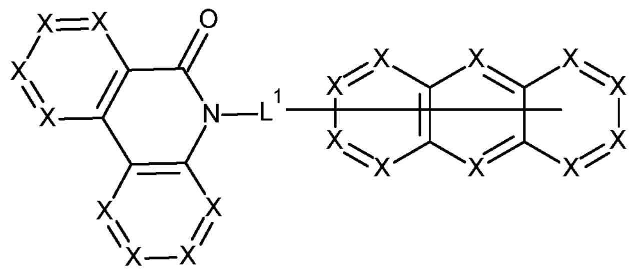

formula (II)

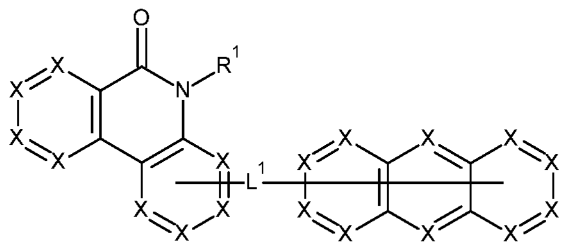

formula (IV)

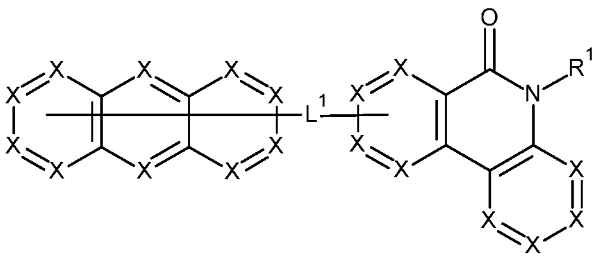

formula (VI)

where the following applies to the symbols and indices:

X is on each occurrence, identically or differently, $CR^1$ or C for the bonding point of the structural element having three fused rings (AR);

$R^1$ is on each occurrence, identically or differently, H, D, CN, a straight-chain alkyl group having 1 to 40 C atoms or a branched or cyclic alkyl group having 3 to 40 C atoms, which may in each case be substituted by one or more radicals $R^2$, where one or more non-adjacent $CH_2$ groups may be replaced by $-R^2C=CR^2-$, $-C\equiv C-$, C=O,

C=S, C=Se, C=$NR^2$, -C(=O)O-, -C(=O)$NR^2$-, $NR^2$, -O-, -S-, SO or $SO_2$ and where one or more H atoms may be replaced by D, F, Cl, Br, I, CN or $NO_2$, or an aromatic or hetero-aromatic ring system having 5 to 40 aromatic ring atoms, which may in each case be substituted by one or more radicals $R^2$;

$R^2$ is on each occurrence, identically or differently, H, D, F, CN, a straight-chain alkyl group having 1 to 40 C atoms or a branched or cyclic alkyl group having 3 to 40 C atoms, which may in each case be substituted by one or more radicals $R^3$, where one or more non-adjacent $CH_2$ groups may be replaced by -$R^3C$=$CR^3$-, -C≡C-, C=O, C=S, C=Se, C=$NR^3$, -C(=O)O-, -C(=O)$NR^3$-, $NR^3$, -O-, -S-, SO or $SO_2$ and where one or more H atoms may be replaced by D, F, Cl, Br, I, CN or $NO_2$, or an aromatic or hetero-aromatic ring system having 5 to 40 aromatic ring atoms, which may in each case be substituted by one or more radicals $R^3$;

$R^3$ is on each occurrence, identically or differently, H, D, F or an aliphatic, aromatic and/or heteroaromatic hydrocarbon radical having 1 to 20 C atoms, in which, in addition, H atoms may be replaced by F;

$L^1$ is a bond or an aryl group having 6 to 40 C atoms or a heteroaryl group having 3 to 40 C atoms, which may in each case be substituted by one or more radicals $R^1$.

2. Compound according to Claim 1, **characterised in that** the compound has a structure of the formula (II-1), (II-2), (II-3), (II-4) or (II-5):

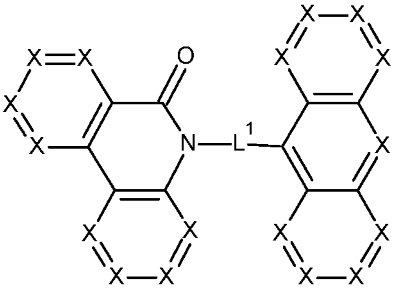

formula (II-1)

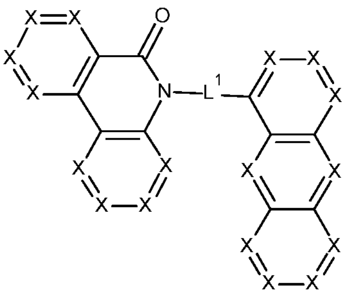

formula (II-2)

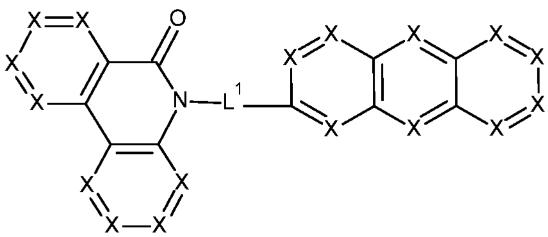

formula (II-3)

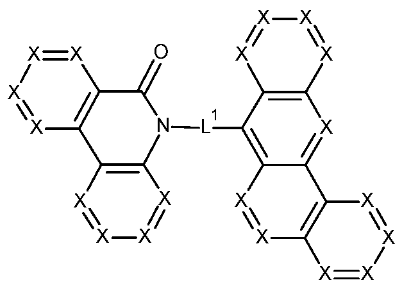

formula (II-4)

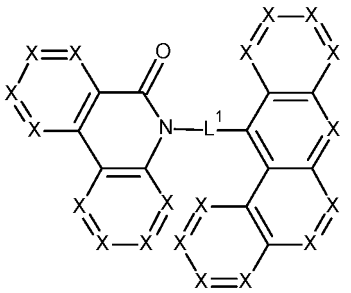

formula (II-5)

where the symbols X, $L^1$ and $R^1$ have the meaning given in Claim 1.

3. Compound according to Claim 1, **characterised in that** the compound has a structure of the formula (IV-1), (IV-2) or (IV-3):

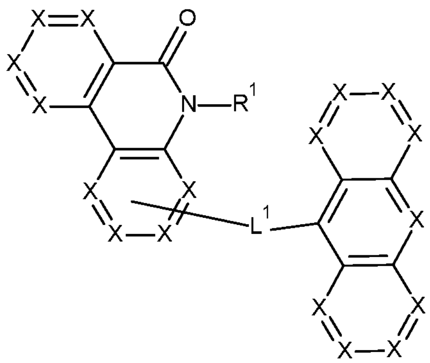

formula (IV-1)

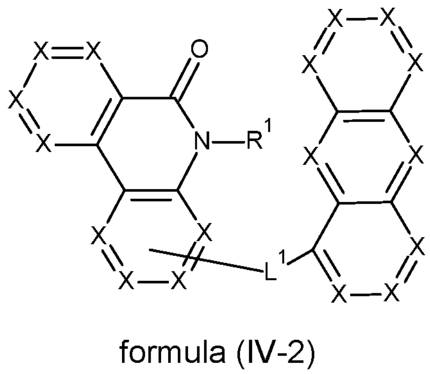

formula (IV-2)

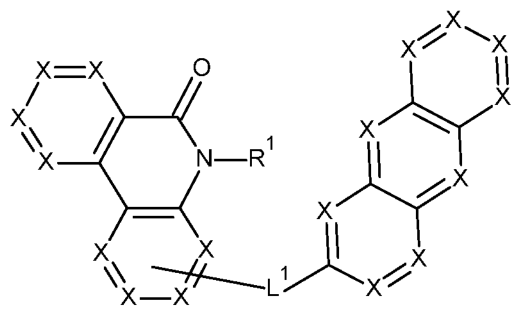

formula (IV-3)

where the symbols X, $L^1$ and $R^1$ have the meaning given in Claim 1.

**4.** Compound according to Claim 1, **characterised in that** the compound has a structure of the formula (VI-1), (VI-2) or (VI-3):

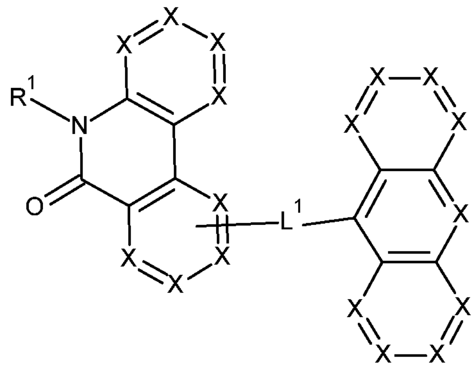

formula (VI-1)

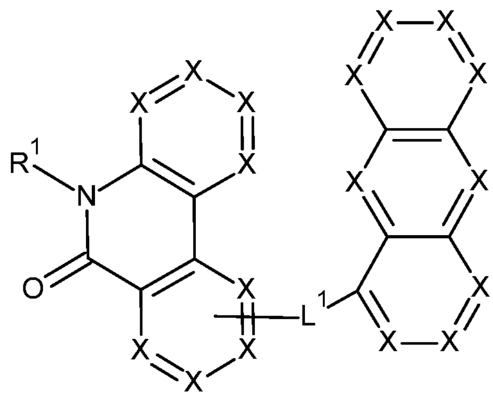

formula (VI-2)

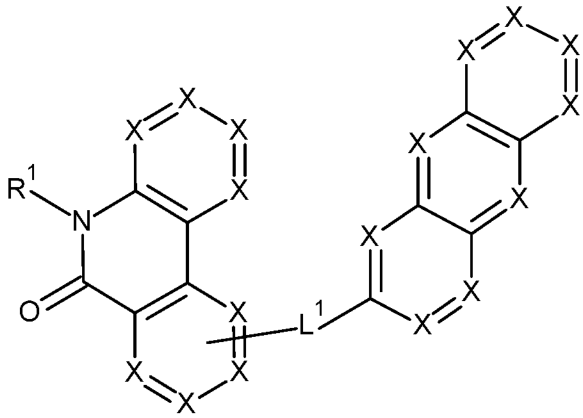

formula (VI-3)

where the symbols X, $L^1$ and $R^1$ have the meaning given in Claim 1.

5. Compound according to Claim 1, **characterised in that** the compound has a structure of the formula (IV-6), (IV-7), (IV-8) or (IV-9):

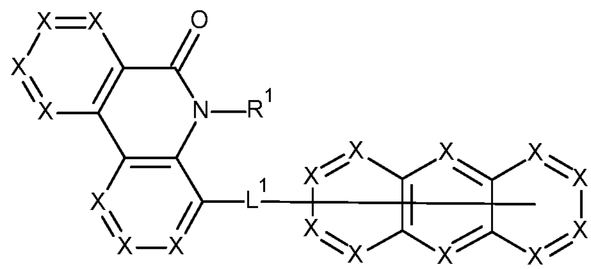

formula (IV-6)

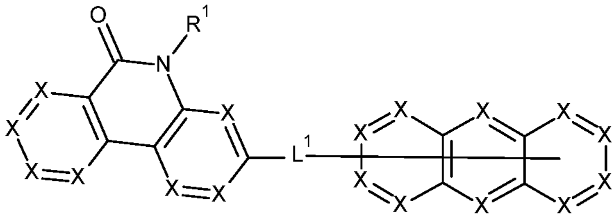

formula (IV-7)

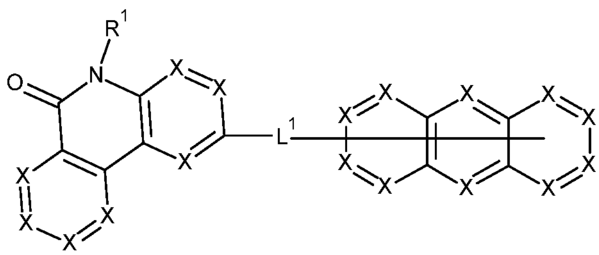

formula (IV-8)

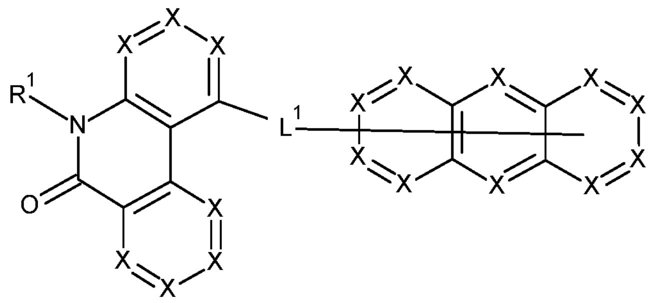

formula (IV-9)

where the symbols X, $L^1$ and $R^1$ have the meaning given in Claim 1.

**6.** Compound according to Claim 1, **characterised in that** the compound has a structure of the formula (VI-6), (VI-7), (VI-8) or (VI-9):

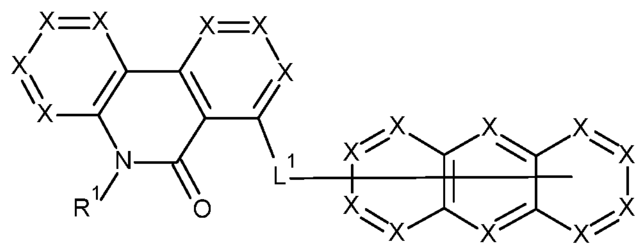

formula (VI-6)

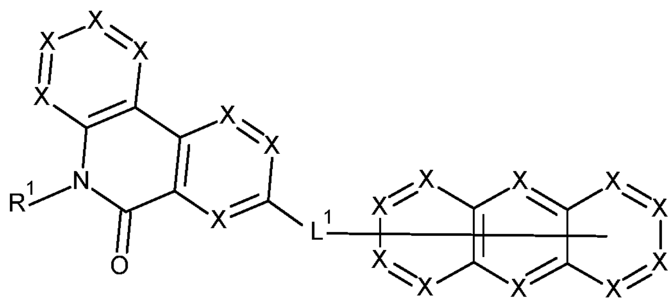

formula (VI-7)

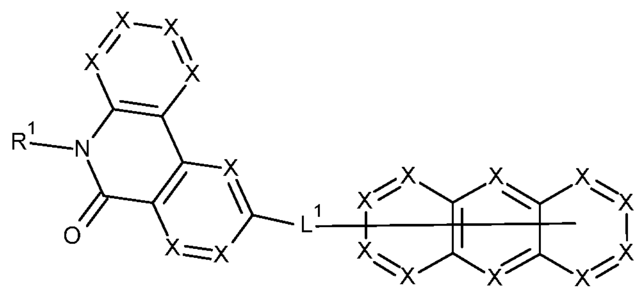

formula (VI-8)

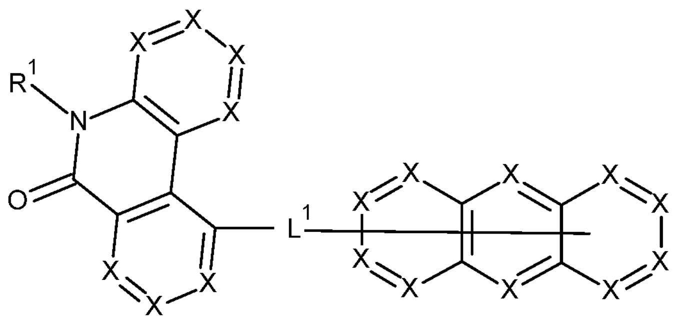

formula (VI-9)

where the symbols X, $L^1$ and $R^1$ have the meaning given in Claim 1.

7. Compound according to Claim 1, **characterised in that** the compound has a structure of the formula (IIa), (IVa) or (VIa):

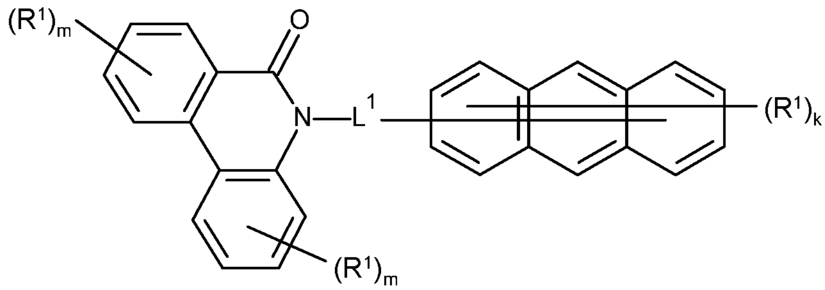

formula (IIa)

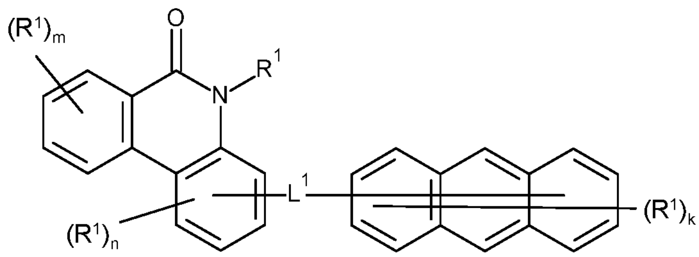

formula (IVa)

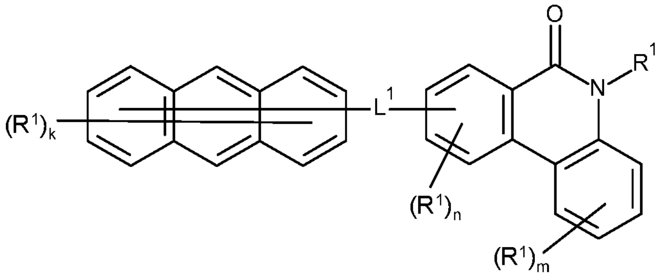

formula (VIa)

in which the symbols $R^1$ and $L^1$ have the meaning described in Claim 1 and the index k is 0, 1, 2, 3, 4, 5, 6, 7, 8 or 9, preferably 0, 1, 2 or 3; the index m is 0, 1, 2, 3 or 4, preferably 0, 1 or 2; and the index n is 0, 1, 2 or 3, preferably 0 or 1.

8. Compound according to Claim 1 or 7, **characterised in that** the compound has a structure of the formula (IIa-1), (IIa-2) or (IIa-3):

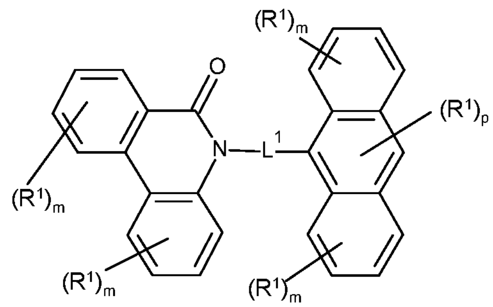

formula (IIa-1)

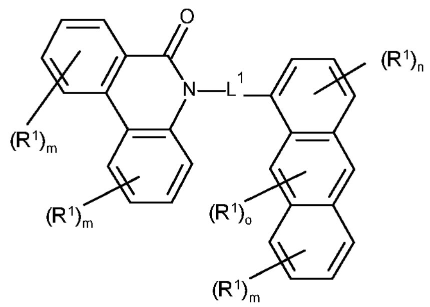

formula (IIa-2)

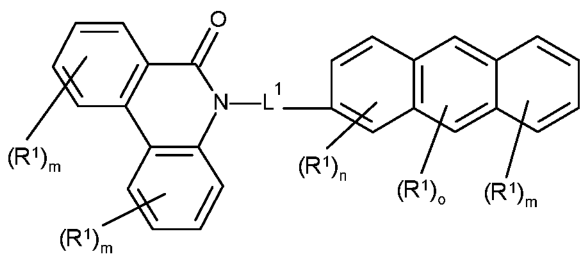

formula (IIa-3)

where the symbols $L^1$ and $R^1$ and the indices m and n have the meaning given in Claim 7, the index o is 0, 1 or 2, preferably 0 or 1, and the index p is 0 or 1, preferably 1.

9. Composition comprising at least one compound according to one or more of Claims 1 to 8 and at least one further compound selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

10. Formulation comprising at least one compound according to one or more of Claims 1 to 8 and/or at least one composition according to Claim 9 and at least one solvent.

11. Process for the preparation of a compound according to one or more of Claims 1 to 8, **characterised in that** a compound having three fused aromatic rings is reacted in a coupling reaction with a compound comprising at least one structural element containing an aromatic valerolactam.

12. Use of a compound according to one or more of Claims 1 to 8 or a composition according to Claim 9 in an electronic device as electron-transport material, electron-injection material and/or hole-blocking material.

**13.** Electronic device containing at least one compound according to one or more of Claims 1 to 8 or a composition according to Claim 9, where the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes.

## Revendications

**1.** Composé de formule (II), (IV) ou (VI) :

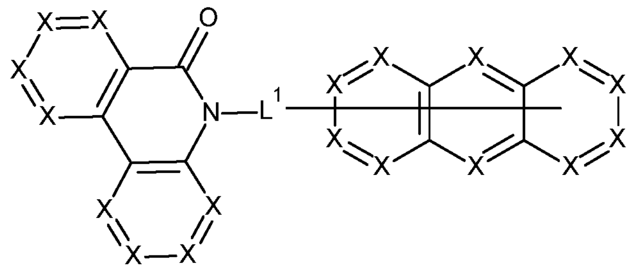

formule (II)

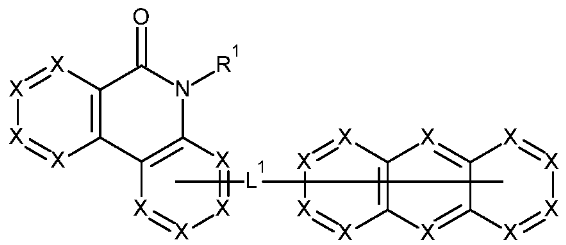

formule (IV)

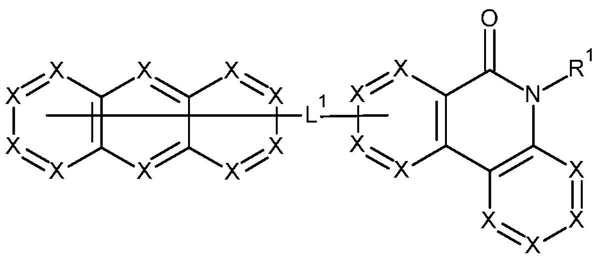

formule (VI)

dans laquelle ce qui suit s'applique aux symboles et aux indices :

X est à chaque occurrence, de manière identique ou différente, $CR^1$ ou C pour le point de liaison à l'élément structurel ayant trois cycles condensés (AR) ;
$R^1$ est à chaque occurrence, de manière identique ou différente, H, D, CN, un groupement alkyle à chaîne linéaire ayant de 1 à 40 atomes de C ou un groupement alkyle ramifié ou cyclique ayant de 3 à 40 atomes de C, pouvant dans chaque cas être substitué par un ou plusieurs radicaux $R^2$, où un ou plusieurs groupements $CH_2$ non adjacents peuvent être remplacés par $-R^2C=CR^2-$, -CEC-, C=O, C=S, C=Se, $C=NR^2$, -C(=O)O-, $-C(=O)NR^2-$, $NR^2$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, pouvant dans chaque cas être substitué par un ou plusieurs radicaux $R^2$ ;
$R^2$ est à chaque occurrence, de manière identique ou différente, H, D, F, CN, un groupement alkyle à chaîne

linéaire ayant de 1 à 40 atomes de C ou un groupement alkyle ramifié ou cyclique ayant de 3 à 40 atomes de C, pouvant dans chaque cas être substitué par un ou plusieurs radicaux $R^3$, où un ou plusieurs groupements $CH_2$ non adjacents peuvent être remplacés par $-R^3C=CR^3-$, -CEC-, C=O, C=S, C=Se, $C=NR^3$, -C(=O)O-, $-C(=O)NR^3-$, $NR^3$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, pouvant dans chaque cas être substitué par un ou plusieurs radicaux $R^3$;

$R^3$ est à chaque occurrence, de manière identique ou différente, H, D, F ou un radical hydrocarboné aliphatique, aromatique et/ou hétéroaromatique ayant de 1 à 20 atomes de C, où, de plus, les atomes de H peuvent être remplacés par F ;

$L^1$ est une liaison ou un groupement aryle ayant de 6 à 40 atomes de C ou un groupement hétéroaryle ayant de 3 à 40 atomes de C, pouvant dans chaque cas être substitué par un ou plusieurs radicaux $R^1$.

2. Composé selon la revendication 1, **caractérisé en ce que** le composé possède une structure de formule (II-1), (II-2), (II-3), (II-4) ou (II-5) :

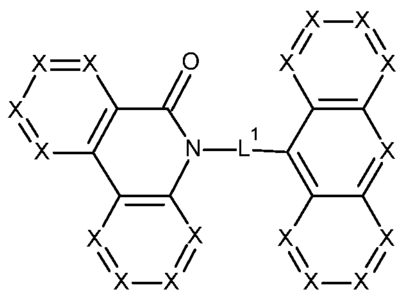

formule (II-1)

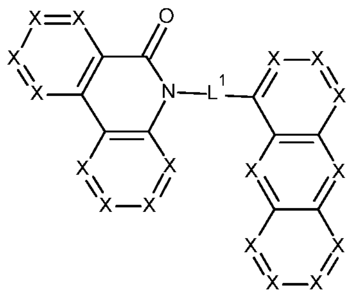

formule (II-2)

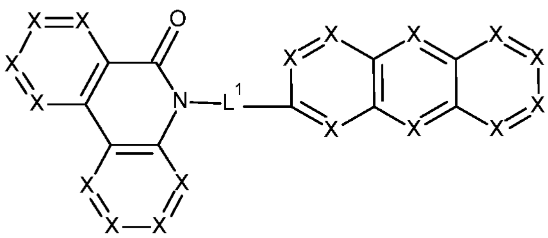

formule (II-3)

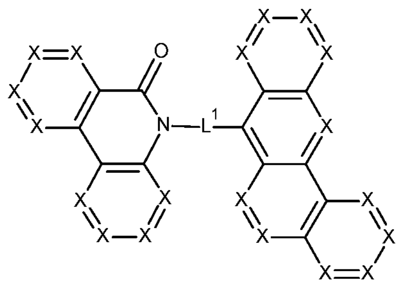

formule (II-4)

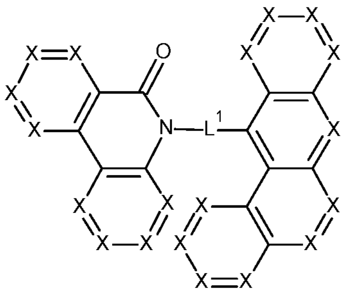

formule (II-5)

dans laquelle les symboles X, $L^1$ et $R^1$ revêtent la signification donnée selon la revendication 1.

3. Composé selon la revendication 1, **caractérisé en ce que** le composé possède une structure de formule (IV-1), (IV-2) ou (IV-3) :

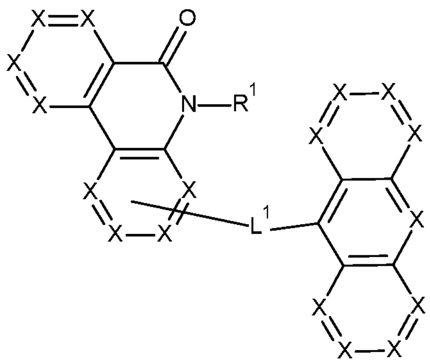

formule (IV-1)

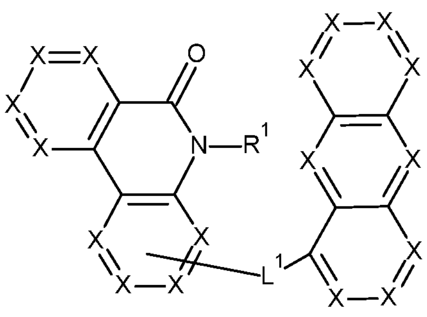

formule (IV-2)

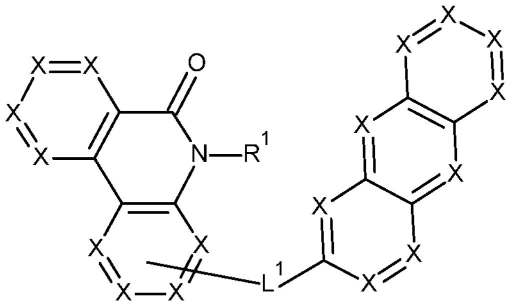

formule (IV-3)

dans laquelle les symboles X, $L^1$ et $R^1$ revêtent la signification donnée selon la revendication 1.

4. Composé selon la revendication 1, **caractérisé en ce que** le composé possède une structure de formule (VI-1), (VI-2) ou (VI-3) :

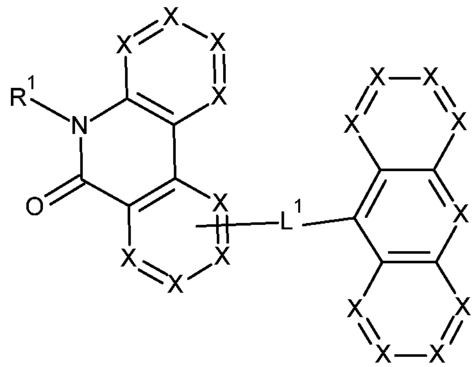

formule (VI-1)

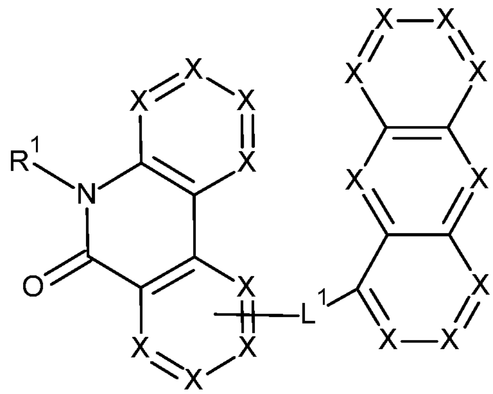

formule (VI-2)

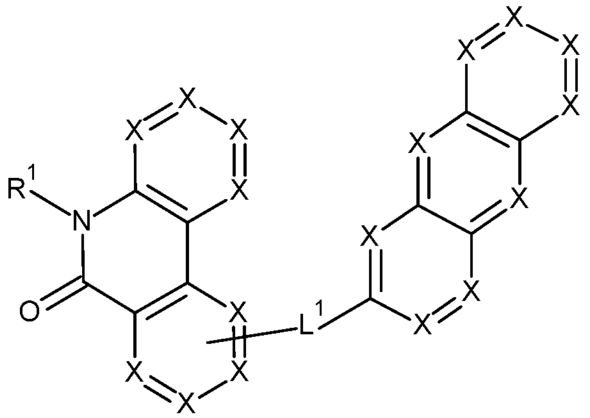

formule (VI-3)

dans laquelle les symboles X, $L^1$ et $R^1$ revêtent la signification donnée selon la revendication 1.

**5.** Composé selon la revendication 1, **caractérisé en ce que** le composé possède une structure de formule (IV-6), (IV-7), (IV-8) ou (IV-9) :

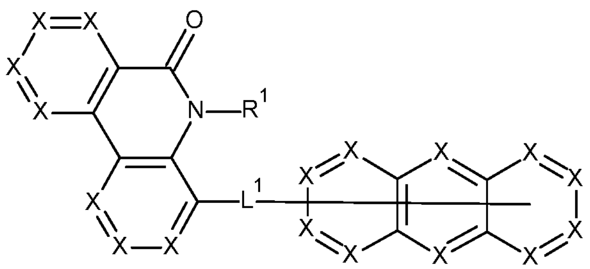

formule (IV-6)

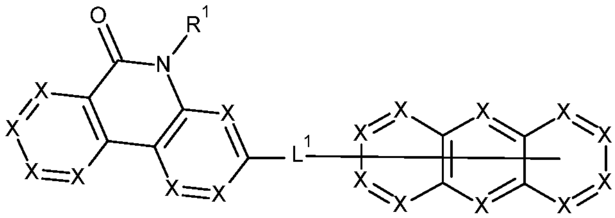

formule (IV-7)

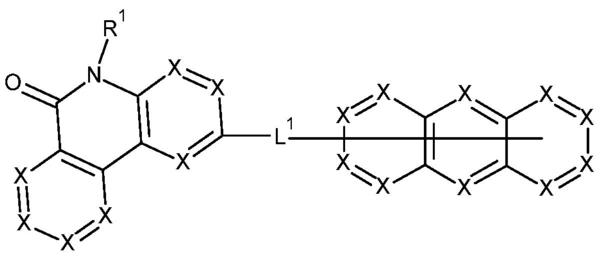

formule (IV-8)

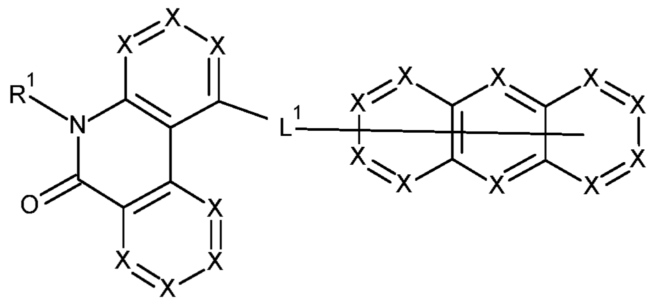

formule (IV-9)

dans laquelle les symboles X, $L^1$ et $R^1$ revêtent la signification donnée selon la revendication 1.

**6.** Composé selon la revendication 1, **caractérisé en ce que** le composé possède une structure de formule (VI-6), (VI-7), (VI-8) ou (VI-9) :

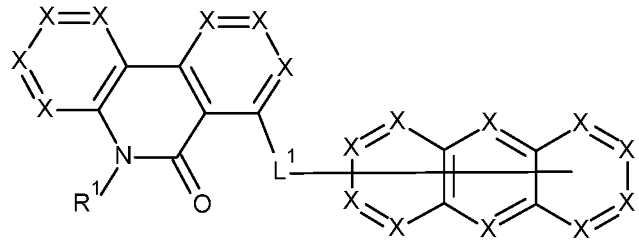

formule (VI-6)

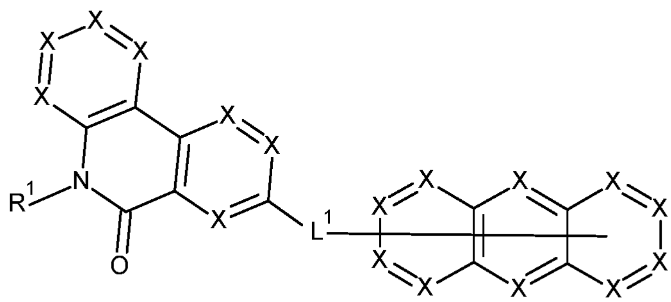

formule (VI-7)

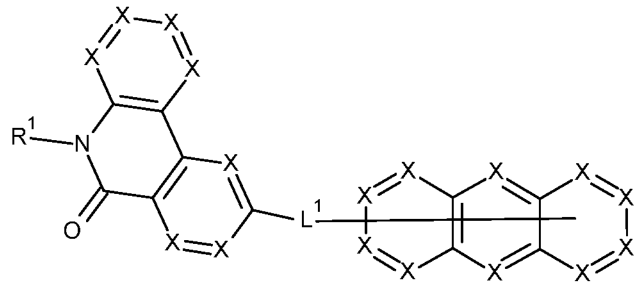

formule (VI-8)

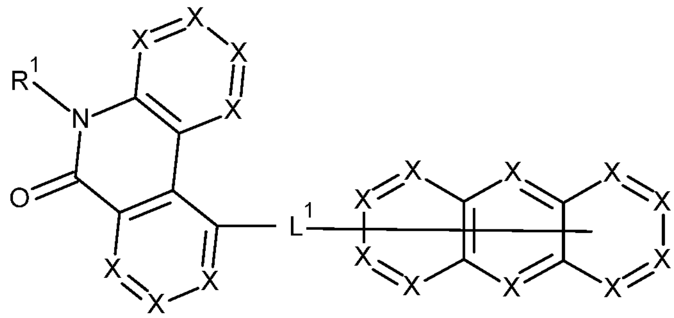

formule (VI-9)

dans laquelle les symboles X, $L^1$ et $R^1$ revêtent la signification donnée selon la revendication 1.

**7.** Composé selon la revendication 1, **caractérisé en ce que** le composé possède une structure de formule (IIa), (IVa) ou (VIa) :

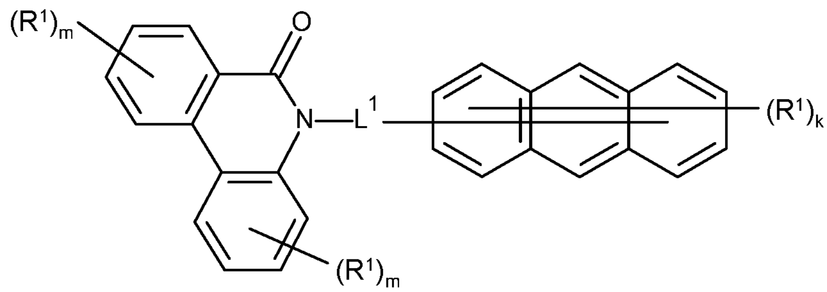

formule (IIa)

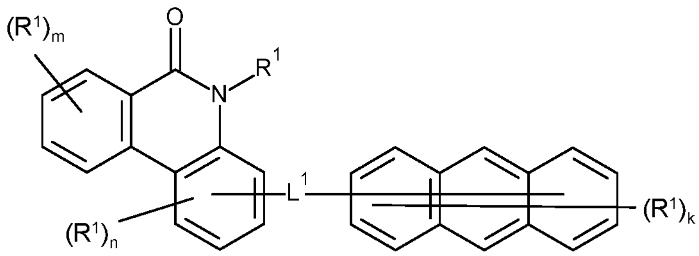

formule (IVa)

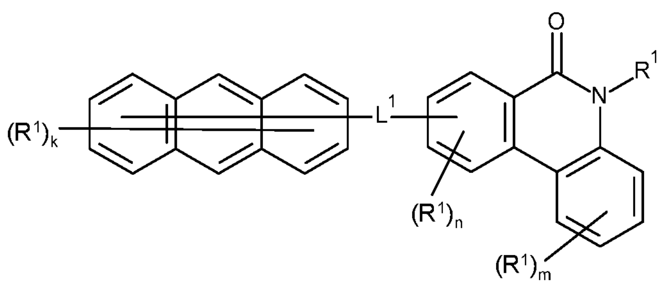

formule (VIa)

dans laquelle les symboles $R^1$ et $L^1$ revêtent la signification décrite selon la revendication 1 et l'indice k vaut 0, 1, 2, 3, 4, 5, 6, 7, 8 ou 9, préférablement 0, 1, 2 ou 3 ; l'indice m vaut 0, 1, 2, 3 ou 4, préférablement 0, 1 ou 2 ; et

l'indice n vaut 0, 1, 2 ou 3, préférablement 0 ou 1.

8. Composé selon la revendication 1 ou 7, **caractérisé en ce que** le composé possède une structure de formule (IIa-1), (IIa-2) ou (IIa-3) :

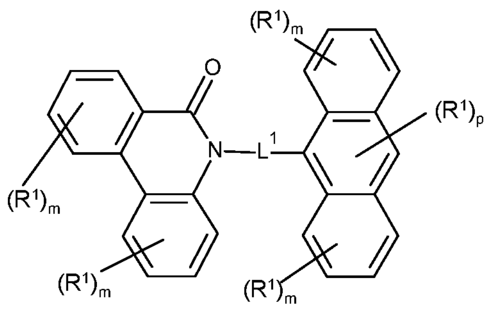

formule (IIa-1)

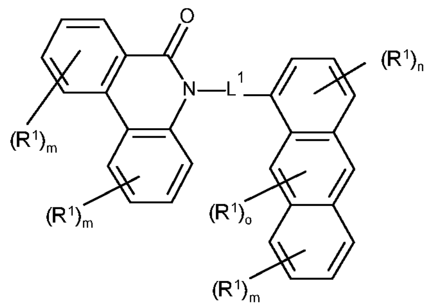

formule (IIa-2)

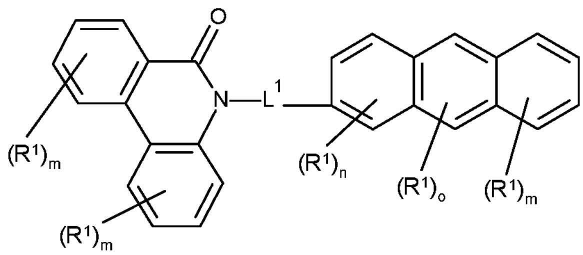

formule (IIa-3)

dans laquelle les symboles $L^1$ et $R^1$ et les indices m et n revêtent la signification donnée selon la revendication 7, l'indice o vaut 0, 1 ou 2, préférablement 0 ou 1, et l'indice p vaut 0 ou 1, préférablement 1.

9. Composition comprenant au moins un composé selon l'une ou plusieurs parmi les revendications 1 à 8 et au moins un autre composé choisi dans le groupe constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous.

10. Formulation comprenant au moins un composé selon l'une ou plusieurs parmi les revendications 1 à 8 et/ou au moins une composition selon la revendication 9, et au moins un solvant.

11. Procédé de préparation d'un composé selon l'une ou plusieurs parmi les revendications 1 à 8, **caractérisé en ce qu'**un composé possédant trois cycles aromatiques condensés est réagi dans une réaction de couplage avec un composé comprenant au moins un élément structurel contenant un valérolactame aromatique.

**12.** Utilisation d'un composé selon l'une ou plusieurs parmi les revendications 1 à 8, ou d'une composition selon la revendication 9, dans un dispositif électronique comme matériau de transport d'électrons, matériau d'injection d'électrons et/ou matériau de blocage de trous.

**13.** Dispositif électronique contenant au moins un composé selon l'une ou plusieurs parmi les revendications 1 à 8 ou une composition selon la revendication 9, le dispositif électronique étant préférablement choisi dans le groupe constitué par les dispositifs électroluminescents organiques, les circuits intégrés organiques, les transistors organiques à effet de champ, les transistors organiques en couche mince, les transistors organiques émetteurs de lumière, les cellules solaires organiques, les détecteurs optiques organiques, les photorécepteurs organiques, les dispositifs organiques à extinction de champ, les cellules électrochimiques émettrices de lumière ou les diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- WO 2011137951 A
- WO 2013064206 A
- WO 2009140467 A1
- US 2013072485 A1
- US 2010249168 A1
- US 20150270495 A1
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016015815 A
- WO 2016124304 A
- EP 15182264
- EP 16179378
- EP 16186313
- WO 2005011013 A
- WO 2004013080 A
- WO 2004093207 A
- WO 2006005627 A
- WO 2010006680 A
- WO 2014015935 A
- WO 2005039246 A
- US 20050069729 A
- JP 2004288381 A
- EP 1205527 A
- WO 2008086851 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 005111172 A
- WO 2006117052 A
- WO 2010015306 A
- EP 652273 A
- WO 2009062578 A
- WO 2010054729 A
- WO 2010054730 A
- US 20090136779 A
- WO 2010050778 A
- WO 2011042107 A
- WO 2011088877 A
- WO 2012143080 A
- WO 2012048781 A
- WO 2011116865 A
- WO 2014094963 A
- WO 2015192939 A
- WO 2015169412 A
- WO 2016015810 A

- WO 2016023608 A
- EP 16158460
- EP 16159829
- WO 2010108579 A
- WO 2005053051 A
- WO 2009030981 A


**In der Beschreibung aufgeführte Nicht-Patentliteratur**


- **K. SHICHIRI et al.** *Chem. Pharm. Bull.,* 1980, vol. 28, 493-499
- **W. CAMBARAU et al.** *Chem. Commun.,* 2015, vol. 51, 1128-1130
- **Y. HASHIKAWA et al.** *Org. Lett.,* 2014, vol. 16, 2970-2973
- **C. Y. ALMOND et al.** *J. Chem. Soc.,* 1951, 1906-1909
- **Y. HAMADA et al.** *Chem. Pharm. Bull.,* 1976, vol. 24, 2769-2774
- **M. MINTAS et al.** *J. Chem. Soc. Perkin Trans.,* 1990, vol. 2, 619-624
- **K. NAKAMURA et al.** *J. Am. Chem. Soc.,* 2014, vol. 136, 555-558
- **L. PERRIN et al.** *Eur. J. Org. Chem.,* 2011, 5427-5440
- **C. W. TANG.** *Appl. Phys. Lett.,* 1986, vol. 48, 183-185
- **J. NAKAMURA et al.** *Bull. Chem. Soc. Jpn.,* 2004, vol. 77, 2185-2188
- *CHEMICAL ABSTRACTS,* 25387-93-3
- **D. M. KOLLER et al.** *Nature Photonics,* 2008, 1-4
- **B. M. S. ARNOLD et al.** *Appl. Phys. Lett.,* 2008, vol. 92, 053301